# EUROPEAN PATENT APPLICATION

(11) **EP 1 561 465 A2**
(43) Date of publication of application: **10.08.2005**
(21) Application number: 05075938.0
(22) Date of filing: 22.09.2000
(51) Int. Cl.: A61K 31/4709, A61P 31/04

(54) **Methods of use of fluoroquinolone compounds against bacteria**

(30) Priority: 22.09.1999 GR 99100318; 23.09.1999 GR 99100321; 23.09.1999 ES 9902113; 23.09.1999 ES 9902112; 23.09.1999 ES 9902111; 23.09.1999 ES 9902114; 24.09.1999 AR 9904837; 24.09.1999 IT MI991997; 24.09.1999 JP 30975499; 24.09.1999 JP 30975699; 24.09.1999 JP 30975599; 24.09.1999 DE 19945885
(62) Divisional of application: 00966806.2
(71) Applicant: LG Life Sciences, Ltd., Seoul 150-721 (KR)
(72) Inventor: Alcala, Luis, 28007 Madrid (ES); Antonnaidou, Anastasia, 151 26 Maroussi Attikis (GR); Athanasiou, Kalomina, 151 26 Maroussi Attikis (GR); Bourousi, Maria, 151 26 Maroussi Attikis (GR); Bouza, Emilio, 28007 Madrid (ES); Bygate, Elizabeth, Harlow Essex CM19 5AW (GB); Cabria, Froilan, 28040 Madrid (ES); Cercenado, Emilia, 28007 Madrid (ES); Debbia, Eugenio, 16132 Genova (IT); Del Sol Diaz, Maria, 28007 Madrid (ES); Dolcino, Marzia, 16132 Genova (IT); Estaban, Jaime, 28040 Madrid (ES); Fairless, Amanda J., Harlow Essex CM19 5AW (GB); Fernandez-Roblas, Ricardo, 28040 Madrid (ES); Finlay, Jane, Collegeville, PA 19426 (US); Furuya, Nobihoko, Tokyo 143 (JP); Garrote, Fernando Garcia, 28007 Madrid (ES); Giamarellou, Helen, 151 26 Maroussi Attikis (GR); Giannakou, Paraskevi, 151 26 Maroussi Attikis (GR); Gonzales-Abad, Maria Jose, 28007 Madrid (ES); Greka, Panayota, 115 27 Athens (GR); Hammer, Heiko, 94315 Straubing (DE); Inoue, Matsushita, Kanagawa 228-855 (JP); Kato, Kaori, Gifu 500-8705 (JP); Katsala, Demetra, 151 26 Maroussi Attikis (GR); Kinzig-Schippers, Martina, 90562 Heroldsberg (DE); Lopez, Horacio, 3068 Buenos Aires (1425) (AR); Lopez, Juan Carlos, 28040 Madrid (ES); Makino, Michiko, Tokyo 143 (JP); Mandaraka, Aikaterini, 115 27 Athens (GR); Marchese, Anna, 16132 Genova (IT); Marin, Mercedes, 28007 Madrid (ES); Martinez-Sanchez, Lucia, 28007 Madrid (ES); Martin-Pedroviejo, Jesus, 28007 Madrid (ES); Matsumoto, Tetsuya, Tokyo 143 (JP); Mikaelion, Gabriela, Buenos Aires 1425 (AR); Miyazaka, Schuichi, Tokyo 143 (JP); Naber, Christoph K., 45122 Essen (DE); Naber, Kurt G., 94315 Straubing (DE); Ogaswara, Akira, Tokyo 143 (JP); Ohno, Akira, Tokyo 143 (JP); Okamoto, Ryoichi, Kanagawa 228-855 (JP); Ruiz-Serrano, Maria Jesus, 28007 Madrid (ES); Sarachian, Beatriz, Buenos Aires 1425 (AR); Scarano, Silvia, Buenos Aires 1425 (AR); Schito, Gian Carlo, 16132 Genova (IT); Sorgel, Fritz, 90562 Heroldsberg (DE); Soriano, Fransico, 28040 Madrid (ES); Souli, Maria, 151 26 Maroussi Attakis (GR)
(74) Representative: Gillard, Richard Edward

(57) **Abstract**

This invention relates, in part, to newly identified methods of using quinoline antibiotics, particularly a gemifloxacin compound against certain bacteria, particularly pathogenic bacteria.

## Description

This invention relates, in part, to newly identified methods of using quinolone antibiotics, particularly a gemifloxacin compound against bacteria, particularly bacterial pathogens.

### BACKGROUND OF THE INVENTION

Quinolones have been shown to be effective to varying degrees against a range of bacterial pathogens. However, as diseases caused by these pathogens are on the rise, there exists a need for antimicrobial compounds that are more potent than the present group of quinolones.

Gemifloxacin mesylate (SB-265805) is a novel fluoroquinolone useful as a potent antibacterial agent. Gemifloxacin compounds are described in detail in patent application PCT/KR98/00051 published as WO 98/42705. Patent application EP 688772 discloses novel quinoline(naphthyridine)carboxylic acid derivatives, including anhydrous (R,S)-7-(3-aminomethyl-4-methoxyiminopyrrolidin-1-yl)-1-cyclopropyl-6-fluoro-4-oxo-1,4-dihydro-1,8-naphthyridine-3-carboxylic acid of formula I.

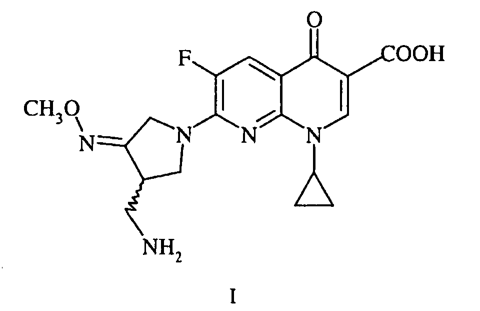

PCT/KR98/00051 discloses (R,S)-7-(3-aminomethyl-4-syn-methoxyimino-pyrrolidin-1-yl)-1-cyclopropyl-6-fluoro-4-oxo-1,4-dihydro-1,8-naphthyridine-3-carboxylic acid methanesulfonate and hydrates thereof including the sesquihydrate.

Provided herein is a significant discovery made using a gemifloxacin compound against certain bacteria, such as, but not limited to MTB, enteropathogenic bacteria, certain Gram positive bacteria, respiratory tract pathogens, genital tract pathogenic bacteria, anaerobic pathogenic bacteria, uropathogenic bacteria, nosocomial Gram negative bacteria, and enteropathogenic bacteria. Disclosed herein are demonstrations that activity of a gemifloxacin compound of the invention was superior to a number of quinolones as described in more detail herein. Gemifloxacin compounds are, therefore, valuable compounds for the treatment of clinical indications caused by a range of bacteria, including those resistant to usual oral therapy, thereby filling an unmet medical need.

### SUMMARY OF THE INVENTION

An object of the invention is a method for modulating metabolism of MTB pathogenic bacteria comprising the step of contacting MTB pathogenic bacteria with an antibacterially effective amount of a composition comprising a quinolone, particularly a gemifloxacin compound, or an antibacterially effective derivative thereof.

A further object of the invention is a method wherein said MTB pathogenic bacteria is selected from the group consisting of: MTB strain H37Rv, RMTB strains and SMTB strains.

Also provided by the invention is a method of treating or preventing a bacterial infection by MTB pathogenic bacteria comprising the step of administering an antibacterially effective amount of a composition comprising a quinolone, particularly a gemifloxacin compound to a mammal suspected of having or being at risk of having an infection with MTB pathogenic bacteria.

Further preferred methods are provided by the invention wherein said bacteria is selected from the group consisting of: MTB strain H37Rv, RMTB strains and SMTB strains.

An object of the invention is a method for modulating metabolism of enteropathogenic pathogenic bacteria comprising the step of contacting enteropathogenic pathogenic bacteria with an antibacterially effective amount of a composition comprising a quinolone, particularly a gemifloxacin compound, or an antibacterially effective derivative thereof.

A further object of the invention is a method wherein said enteropathogenic pathogenic bacteria is selected from the group consisting of: *Salmonella* spp. (e.g., *S. hadar, S. virchow, S. tshiongwe,* and *S. newport*), *Hafnia alvei, Yersinia enterocolitica, Shigella* spp., *Aeromonas* spp. and *Campylobacter jejuni*.

Also provided by the invention is a method of treating or preventing a bacterial infection by enteropathogenic pathogenic bacteria comprising the step of administering an antibacterially effective amount of a composition comprising a quinolone, particularly a gemifloxacin compound to a mammal suspected of having or being at risk of having an infection with enteropathogenic pathogenic bacteria.

Further preferred methods are provided by the invention wherein said bacteria is selected from the group consisting of: *Salmonella* spp. (e.g., *S. hadar, S. virchow*, *S. tshiongwe,* and *S. newport*), *Hafnia alvei, Yersinia enterocolitica, Shigella* spp., *Aeromonas* spp. and *Campylobacter jejuni*.

An object of the invention is a method for modulating metabolism of Gram positive pathogenic bacteria comprising the step of contacting Gram positive pathogenic bacteria with an antibacterially effective amount of a composition comprising a quinolone, particularly a gemifloxacin compound, or an antibacterially effective derivative thereof.

A further object of the invention is a method wherein said Gram positive pathogenic bacteria is selected from the group consisting of: *Streptococcus pneumoniae, Haemophilus influenzae, Moraxella catarrhalis, Streptococcus pyogenes, Staphylococcus aureus* and *Enterococcus faecalis*.

Also provided by the invention is a method of treating or preventing a bacterial infection by Gram positive pathogenic bacteria comprising the step of administering an antibacterially effective amount of a composition comprising a quinolone, particularly a gemifloxacin compound to a mammal suspected of having or being at risk of having an infection with Gram positive pathogenic bacteria.

Further preferred methods are provided by the invention wherein said bacteria is selected from the group consisting of: *Streptococcus pneumoniae, Haemophilus influenzae, Moraxella catarrhalis, Streptococcus pyogenes, Staphylococcus aureus* and *Enterococcus faecalis.*

An object of the invention is a method for modulating metabolism of respiratory tract pathogenic bacteria comprising the step of contacting respiratory tract pathogenic bacteria with an antibacterially effective amount of a composition comprising a quinolone, particularly a gemifloxacin compound, or an antibacterially effective derivative thereof.

A further object of the invention is a method wherein said respiratory tract pathogenic bacteria is selected from the group consisting of: *Streptococcus pneumoniae, Streptococcus pyogenes, Haemophilus influenzae, Moraxella catarrhalis, Staphylococcus aureus* and *Klebsiella pneumoniae*.

Also provided by the invention is a method of treating or preventing a bacterial infection by respiratory tract pathogenic bacteria comprising the step of administering an antibacterially effective amount of a composition comprising a quinolone, particularly a gemifloxacin compound to a mammal suspected of having or being at risk of having an infection with respiratory tract pathogenic bacteria.

Further preferred methods are provided by the invention wherein said bacteria is selected from the group consisting of: *Streptococcus pneumoniae, Streptococcus pyogenes, Haemophilus influenzae, Moraxella catarrhalis, Staphylococcus aureus* and *Klebsiella pneumoniae*.

An object of the invention is a method for modulating metabolism of respiratory tract pathogenic bacteria comprising the step of contacting respiratory tract pathogenic bacteria with an antibacterially effective amount of a composition comprising a quinolone, particularly a gemifloxacin compound, or an antibacterially effective derivative thereof.

A further object of the invention is a method wherein said respiratory tract pathogenic bacteria is selected from the group consisting of: *Streptococcus pneumoniae* and *Haemophilus influenzae*.

Also provided by the invention is a method of treating or preventing a bacterial infection by respiratory tract pathogenic bacteria comprising the step of administering an antibacterially effective amount of a composition comprising a quinolone, particularly a gemifloxacin compound to a mammal suspected of having or being at risk of having an infection with respiratory tract pathogenic bacteria.

Further preferred methods are provided by the invention wherein said bacteria is selected from the group consisting of: *Streptococcus pneumoniae* and *Haemophilus influenzae.*

An object of the invention is a method for modulating metabolism of respiratory tract pathogenic bacteria comprising the step of contacting respiratory tract pathogenic bacteria with an antibacterially effective amount of a composition comprising a quinolone, particularly a gemifloxacin compound, or an antibacterially effective derivative thereof.

A further object of the invention is a method wherein said respiratory tract pathogenic bacteria is selected from the group consisting of: *Haemophilus influenzae, Staphylococcus aureus, Streptococcus pneumoniae, E. coli, P. aeruginosa* and *Moraxella catarrhalis*.

Also provided by the invention is a method of treating or preventing a bacterial infection by respiratory tract pathogenic bacteria comprising the step of administering an antibacterially effective amount of a composition comprising a quinolone, particularly a gemifloxacin compound to a mammal suspected of having or being at risk of having an infection with respiratory tract pathogenic bacteria.

Further preferred methods are provided by the invention wherein said bacteria is selected from the group consisting of: *Haemophilus influenzae, Staphylococcus aureus, Streptococcus pneumoniae, E. coli, P. aeruginosa* and *Moraxella catarrhalis.*

An object of the invention is a method for modulating metabolism of respiratory or urinary tract pathogenic bacteria comprising the step of contacting respiratory or urinary tract pathogenic bacteria with an antibacterially effective amount of a composition comprising a quinolone, particularly a gemifloxacin compound, or an antibacterially effective derivative thereof.

A further object of the invention is a method wherein said respiratory or urinary tract pathogenic bacteria is selected from the group consisting of: *Staphylococcus aureus* (including oxacillin-susceptible, oxacillin-resistant, and quinolone-resistant strains), *Staphylococcus epidermidis* (including oxacillin-susceptible, oxacillin-resistant strains), *Streptococcus pneumoniae* (including qauinolone-resistant, amoxicillin-susceptible, amoxicillin-resistant, erythromycin-susceptible and erythromycin-resistant strains), *Streptococcus pyrogenes, Enterococcus faecalis* (including ciprofloxacin-susceptible and ciprofloxacin-resistant strains), *Enterococcus faecium* (including vanA and vanB vancomycin-resistant strains), *Enterococcus gallinarum* (including vanC1), *Escherichia coli* (including ciprofloxacin-susceptible and ciprofloxacin-resistant strains), *Citrobacter freundii, Klebsiella pneumoniae, Klebsiella oxytoca, Enterobacter aerogenes, Enterobacter cloacae, Salmonella spp., Shigella spp., Proteus mirabilis, Proteus vulgaris, Morganella morganii, Providencia rettgeri, Serratia marcescens, Pseudomonas aeruginosa, Bukolderia cepacia, Stenotrophomonas maltophilia, Acintobacter calcoaceticus, Haemophilus influenzae, Moraxella catarrhalis,* and *Neisseria gonorrhoeae.*

Also provided by the invention is a method of treating or preventing a bacterial infection by respiratory or urinary tract pathogenic bacteria comprising the step of administering an antibacterially effective amount of a composition comprising a quinolone, particularly a gemifloxacin compound to a mammal suspected of having or being at risk of having an infection with respiratory or urinary tract pathogenic bacteria.

Further preferred methods are provided by the invention wherein said bacteria is selected from the group consisting of: *Staphylococcus aureus* (including oxacillin-susceptible, oxacillin-resistant, and quinolone-resistant strains), *Staphylococcus epidermidis* (including oxacillin-susceptible, oxacillin-resistant strains), *Streptococcus pneumoniae* (including qauinolone-resistant, amoxicillin-susceptible, amoxicillin-resistant, erythromycin-susceptible and erythromycin-resistant strains), *Streptococcus pyrogenes, Enterococcus faecalis* (including ciprofloxacin-susceptible and ciprofloxacin-resistant strains), *Enterococcus faecium* (including vanA and vanB vancomycin-resistant strains), *Enterococcus gallinarum* (including vanC1), *Escherichia coli* (including ciprofloxacin-susceptible and ciprofloxacin-resistant strains), *Citrobacter freundii, Klebsiella pneumoniae, Klebsiella oxytoca, Enterobacter aerogenes, Enterobacter cloacae, Salmonella spp., Shigella spp., Proteus mirabilis, Proteus vulgaris, Morganella morganii, Providencia rettgeri, Serratia marcescens, Pseudomonas aeruginosa, Bukolderia cepacia, Stenotrophomonas maltophilia, Acintobacter calcoaceticus, Haemophilus influenzae, Moraxella catarrhalis,* and *Neisseria gonorrhoeae.*

An object of the invention is a method for modulating metabolism of anaerobic pathogenic bacteria comprising the step of contacting anaerobic pathogenic bacteria with an antibacterially effective amount of a composition comprising a quinolone, particularly a gemifloxacin compound, or an antibacterially effective derivative thereof.

A further object of the invention is a method wherein said anaerobic pathogenic bacteria is selected from the group consisting of: Gram positive and Gram negative anaerobic bacteria, including *Peptostreptococcus anaerobius, Peptostreptococcus asaccharolyticus,Peptostreptococcus indolicus, Peptostreptococcus magnus, Peptostreptococcus micros, Peptostreptococcus prevotii, Staphylococcus saccharolyticus, Atopobium parvulus, Streptococcus constellatus, Streptococcus intermedius, Gemella morbillorum, Clostridium clostridioforme, Clostridium difficile, Clostridium perfringens, Clostridium septicum, Clostridium sordellii, Clostridium ramosum, Propionibacterium acnes, Propionibacterium granulosum, Eubacterium lentum, Actinomyces odontolyticus, Bifdobacterium adolescentis, Bifidobacterium bifidum, Bifidobacterium breve, Bifidobacterium longum, Bifidobacterium pseudolongum, Lactobacillus, Lactobacillus brevis subsp. Brevis, Lactobacillus casei subsp. casei, Lactobacillus fermentum, Lactobacillus plantarum, Lactobacillus reuteri, Lactobacillus salivarius subsp. salivarius, bacteroides fragilis, Bacteroides vulgatus, Bacteroides distasonis, Bacteroides ovatus, Bacteroides thetaiotaomicron, Bacteroides uniformis, Bacteroides eggerthii, Bacteroides ureolyticus, Campylobacter gracilis, Sutterella wadsworthensis, Prevotella bivia, Prevotella buccae, Prevotella corporis, Prevotella heparinolytica, Prevotella intermedia, Prevotella melaninogenica, Prevotella oralis, Prevotella oris, Porphyromonas asaccharolytica, Porphylomonas gingivalis, Fusobacterium nucleatum, Fusobacterium varium, Fusobacterium necrophorum, Bilophilla wadsworthia, Desulfomonas pigra, Capnocytophaga ochracea, Veillonella parvula, Veillonella dispar, Peptostreptococcus anaerobius, Peptostreptococcus asccharolyticus, Peptostreptococcus magnus, Peptostreptococcus micros, Propionibacterium acnes, Actinomyces spp., Clostridium difficile, Clostridium perfringens, Bacteroides distasonis, Bacteroides fragilis, Bacteroides thetaiotaomicron, Bacteroides uniformis, B. fragilis group organisms (B. caccae; B. eggerthii, B. ovatus), lmipenem-resistant B. fragilis group organisms (B. distasonis, B. fragilis), Prevotella bivia, Prevotella intermedia, Other Prevotella spp., Porphyromonas spp., Fusobacterium nucleatum,* and *Veillonella spp..*

Also provided by the invention is a method of treating or preventing a bacterial infection by anaerobic pathogenic bacteria comprising the step of administering an antibacterially effective amount of a composition comprising a quinolone, particularly a gemifloxacin compound to a mammal suspected of having or being at risk of having an infection with anaerobic pathogenic bacteria.

Further preferred methods are provided by the invention wherein said bacteria is selected from the group consisting of: Gram positive and Gram negative anaerobic bacteria, including *Peptostreptococcus anaerobius, Peptostreptococcus asaccharolyticus,Peptostreptococcus indolicus, Peptostreptococcus magnus, Peptostreptococcus micros, Peptostreptococcus prevotii, Staphylococcus saccharolyticus, Atopobium parvulus, Streptococcus constellatus, Streptococcus intermedius, Gemella morbillorum, Clostridium clostridioforme, Clostridium difficile, Clostridium perfringens, Clostridium septicum, Clostridium sordellii, Clostridium ramosum, Propionibacterium acnes, Propionibacterium granulosum, Eubacterium lentum, Actinomyces odontolyticus, Bifidobacterium adolescentis, Bifidobacterium bifidum, Bifidobacterium breve, Bifidobacterium longum, Bifidobacterium pseudolongum, Lactobacillus, Lactobacillus brevis subsp. Brevis, Lactobacillus casei subsp. casei, Lactobacillus fermentum, Lactobacillus plantarum, Lactobacillus reuteri, Lactobacillus salivarius subsp. salivarius, bacteroides fragilis, Bacteroides vulgatus, Bacteroides distasonis, Bacteroides ovatus, Bacteroides thetaiotaomicron, Bacteroides uniformis, Bacteroides eggerthii, Bacteroides ureolyticus, Campylobacter gracilis, Sutterella wadsworthensis, Prevotella bivia, Prevotella buccae, Prevotella corporis, Prevotella heparinolytica, Prevotella intermedia, Prevotella melaninogenica, Prevotella oralis, Prevotella oris, Porphyromonas asaccharolytica, Porphylomonas gingivalis, Fusobacterium nucleatum, Fusobacterium varium, Fusobacterium necrophorum, Bilophilla wadsworthia, Desulfomonas pigra, Capnocytophaga ochracea, Veillonella parvula, Veillonella dispar, Peptostreptococcus anaerobius, Peptostreptococcus asccharolyticus, Peptostreptococcus magnus, Peptostreptococcus micros, Propionibacterium acnes, Actinomyces spp., Clostridium difficile, Clostridium perfringens, Bacteroides distasonis, Bacteroides fragilis, Bacteroides thetaiotaomicron, Bacteroides uniformis, B. fragilis group organisms (B. caccae; B. eggerthii, B. ovatus), Imipenem-resistant B. fragilis group organisms (B. distasonis, B. fragilis), Prevotella bivia, Prevotella intermedia, Other Prevotella spp., Porphyromonas spp., Fusobacterium nucleatum,* and *Veillonella spp.*

An object of the invention is a method for modulating metabolism of uropathogenic pathogenic bacteria comprising the step of contacting uropathogenic pathogenic bacteria with an antibacterially effective amount of a composition comprising a quinolone, particularly a gemifloxacin compound, or an antibacterially effective derivative thereof.

A further object of the invention is a method wherein said uropathogenic pathogenic bacteria is selected from the group consisting of: *K. pneumoniae, P. mirabilis, E. coli, P. aeruginosa, S. aureus,* and *E. faecalis*.

Also provided by the invention is a method of treating or preventing a bacterial infection by uropathogenic bacteria comprising the step of administering an antibacterially effective amount of a composition comprising a quinolone, particularly a gemifloxacin compound to a mammal suspected of having or being at risk of having an infection with uropathogenic bacteria.

Further preferred methods are provided by the invention wherein said bacteria is selected from the group consisting of: *K. pneumoniae, P. mirabilis, E. coli, P. aeruginosa, S. aureus*, and *E. faecalis.*

An object of the invention is a method for modulating metabolism of nosocomial Gram negative pathogenic bacteria comprising the step of contacting nosocomial Gram negative pathogenic bacteria with an antibacterially effective amount of a composition comprising a quinolone, particularly a gemifloxacin compound, or an antibacterially effective derivative thereof.

A further object of the invention is a method wherein said nosocomial Gram negative pathogenic bacteria is selected from the group consisting of: *Acinetobacter* spp., *Enterobacter* spp., *Proteus mirabilis, Klebsiella pneumoniae, Escherichia coli,* and *Pseudomonas aeruginosa.*

Also provided by the invention is a method of treating or preventing a bacterial infection by nosocomial Gram negative pathogenic bacteria comprising the step of administering an antibacterially effective amount of a composition comprising a quinolone, particularly a gemifloxacin compound to a mammal suspected of having or being at risk of having an infection with nosocomial Gram negative pathogenic bacteria.

Further preferred methods are provided by the invention wherein said bacteria is selected from the group consisting of: *Acinetobacter* spp., *Enterobacter* spp., *Proteus mirabilis, Klebsiella pneumoniae, Escherichia coli,* and *Pseudomonas aeruginosa.*

An object of the invention is a method for modulating metabolism of Gram positive pathogenic bacteria comprising the step of contacting Gram positive pathogenic bacteria with an antibacterially effective amount of a composition comprising a quinolone, particularly a gemifloxacin compound, or an antibacterially effective derivative thereof.

A further object of the invention is a method wherein said Gram positive pathogenic bacteria is selected from the group consisting of: *Streptococcus pneumoniae, Enterococcus faecalis, Enterococcus faecium, Staphylococcus aureus* (including Methicillin-susceptible and Methicillin-resistant strains), and *Staphylococcus epidermidis* (including Methicillin-susceptible and Methicillin-resistant strains).

Also provided by the invention is a method of treating or preventing a bacterial infection by Gram positive pathogenic bacteria comprising the step of administering an antibacterially effective amount of a composition comprising a quinolone, particularly a gemifloxacin compound to a mammal suspected of having or being at risk of having an infection with Gram positive pathogenic bacteria.

Further preferred methods are provided by the invention wherein said bacteria is selected from the group consisting of: *Streptococcus pneumoniae, Enterococcus faecalis, Enterococcus faecium, Staphylococcus aureus* (including Methicillin-susceptible and Methicillin-resistant strains), and *Staphylococcus epidermidis* (including Methicillin-susceptible and Methicillin-resistant strains).

An object of the invention is a method for modulating metabolism of enteropathogenic bacteria comprising the step of contacting enteropathogenic bacteria with an antibacterially effective amount of a composition comprising a quinolone, particularly a gemifloxacin compound, or an antibacterially effective derivative thereof.

A further object of the invention is a method wherein said enteropathogenic bacteria is selected from the group consisting of: *Salmonella* spp. (including *S. enteritidis, S. typhimurium,* S. *virchow, S. tshiongwe, S. newport, S. ohio, S. hadar,* and *S. georgia*), *Hafnia alvei, Yersinia enterocolitica, Shigella* spp. (including *S. sonnei, S. flexneri,* and *S. boydii*), *Aeromonas* spp. (including *A. hydrophila* and A. *sobria*), and *Campylobacter jejuni*.

Also provided by the invention is a method of treating or preventing a bacterial infection by enteropathogenic bacteria comprising the step of administering an antibacterially effective amount of a composition comprising a quinolone, particularly a gemifloxacin compound to a mammal suspected of having or being at risk of having an infection with enteropathogenic bacteria.

Further preferred methods are provided by the invention wherein said bacteria is selected from the group consisting of: *Salmonella* spp. (including *S. enteritidis, S. typhimurium, S. virchow, S. tshiongwe, S. newport, S. ohio, S. hadar,* and *S. georgia*), *Hafnia alvei, Yersinia enterocolitica, Shigella* spp. (including *S. sonnei, S. flexneri,* and *S. boydii*), *Aeromonas* spp. (including *A. hydrophila* and *A. sobria*), and *Campylobacter jejuni*.

A preferred method is provided wherein said modulating metabolism is inhibiting growth of said bacteria or killing said bacteria.

A further preferred method is provided wherein said contacting said bacteria comprises the further step of introducing said composition into a mammal, particularly a human.

Still further preferred methods comprise a gemifloxacin compound selected from the group consisting of gemifloxacin mesylate, gemifloxacin mesylate hydrate, gemifloxacin mesylate hemihydrate and gemifloxacin mesylate sesquihydrate.

Various changes and modifications within the spirit and scope of the disclosed invention will become readily apparent to those skilled in the art from reading the following descriptions and from reading the other parts of the present disclosure.

### DESCRIPTION OF THE INVENTION

The present invention provides, among other things, methods for using a composition comprising a quinolone, particularly a gemifloxacin compound against MTB.

As used herein "gemifloxacin compound(s)" means a compound having antibacterial activity described in patent application PCT/KR98/00051 published as WO 98/42705 on 1 October 1998, or patent application EP 688772, and these applications are incorporated herein by reference.

This invention was based, in part, on analyses evaluating the comparative activity of gemifloxacin against various MTB pathogens. An objective of these analyses was to determine the MIC range, MIC₅₀ and MIC₉₀ of six fluoroquinolones: ciprofloxacin, ofloxacin, levofloxacin, grepafloxacin, trovafloxacin and gemifloxacin.

Tuberculosis caused by multi-drug-resistant strains of *Mycobacterium tuberculosis* (MTB) poses a therapeutic challenge in terms of the selection of appropriate antimicrobial agents. Although quinolones may have a useful role in treating these infections, comparative *in vitro* data with classic and new agents of this class are scarce. An objective of this study was to evaluate the *in vitro* activity of six fluoroquinolones against clinical isolates of MTB with different levels of susceptibility to first-line antituberculous drugs.

Tuberculosis caused by multi-drug-resistant strains of *Mycobacterium tuberculosis* (MTB) presents a therapeutic challenge to physicians selecting antimicrobial agents. Quinolones can have a useful role in treating these infections, but comparative *in vitro* data with classic and new agents of this class are scarce. A total of 82 MTB strains were tested by the proportions method using NCCLS methodology (M24-T, 1995) against the quinolones ciprofloxacin, ofloxacin, levofloxacin, grepafloxacin, trovafloxacin and the novel compound gemifloxacin (SB-265805). The concentration range of antimicrobials assayed was 0.5-4 µg/ml and the MTB strain H37Rv was used for quality control. Thirty-seven strains were resistant (RMTB) to at least one first-line antituberculous drug, while the rest were fully susceptible (SMTB). The MIC range, MIC₅₀ and MIC₉₀ (µg/ml) were obtained for the agents tested.

MICs of 82 MTB strains were evaluated using the proportions method according to NCCLS criteria (M24-T, 1995). The fluoroquinolones investigated were ciprofloxacin, ofloxacin, levofloxacin, grepafloxacin, trovafloxacin and gemifloxacin (SB-265805). The antimicrobial concentrations assayed were 0.5-4 µg/ml. MTB strain H37Rv was used for quality control. All MTB strains had been previously tested with first-line antituberculous drugs (except pyrazinamide) by the agar proportions method. Thirty-seven strains were resistant (RMTB) to at least one first-line antituberculous drug, while the rest were fully susceptible (SMTB).

Levofloxacin (MIC₉₀ 1 µg/ml) demonstrates the greatest activity against the strains of MTB tested, although ciprofloxacin, ofloxacin and grepafloxacin also demonstrate good activity with MIC₉₀s of 2 µg/ml. Trovafloxacin and gemifloxacin show lower *in vitro* activity against MTB than the other quinolones. In general, quinolone activity is higher in SMTB strains than in RMTB strains, with a twofold difference in MIC₉₀.

The invention provides a method for modulating metabolism of MTB pathogenic bacteria. Skilled artisans can readily choose MTB pathogenic bacteria or patients infected with or suspected to be infected with these organisms to practice the methods of the invention. Alternatively, the bacteria useful in the methods of the invention may be those described herein.

Also provided by the invention is a method of treating or preventing a bacterial infection by MTB pathogenic bacteria comprising the step of administering an antibacterially effective amount of a composition comprising a quinolone, particularly a gemifloxacin compound to a mammal, preferably a human, suspected of having or being at risk of having an infection with MTB pathogenic bacteria.

While a preferred object of the invention provides a method wherein said MTB pathogenic bacteria is selected from the group consisting of: MTB strain H37Rv, RMTB strains and SMTB strains. Other MTB pathogenic bacteria may also be included in the methods. The skilled artisan may identify these organisms as provided herein as well as using other methods known in the art, *e.g.* MIC tests.

The present invention provides, among other things, methods for using a composition comprising a quinolone, particularly a gemifloxacin compound against enteropathogenic bacteria.

This invention was based, in part, on analyses evaluating the comparative activity of gemifloxacin against 288 clinical isolates obtained from 1996 to 1999. An objective of these analyses was to determine the MICs of gemifloxacin, trovafloxacin, grepafloxacin, ciprofloxacin, ofloxacin, norfloxacin, levofloxacin and nalidixic acid by the agar dilution method (NCCLS, *4th ed.,* M7-A4, Villanova, PA (1997)) on Mueller-Hinton agar supplemented with 5% sheep blood for *Campylobacter* isolates.

Gemifloxacin was compared to trovafloxacin, grepafloxacin, ciprofloxacin, ofloxacin, norfloxacin, levofloxacin and nalidixic acid. Isolates were incubated aerobically at 35°C for 24 h, except for *Campylobacter* strains, which were incubated microaerophilically for 48 h. MIC₅₀s are equal to MIC₉₀s except in the case of *Salmonella* spp. and *C. jejuni,* where the MICs differ by 1-2 dilutions. Non-susceptibility rates for nalidixic acid range from 0% (*H. alvei, Shigella* spp.) to 4.50% (*Y. enterocolitica*), 6.25% (*Aeromonas* spp.), 27.00% (*Salmonella* spp.) and 69.24% (*C*. *jejuni*). One per cent of *Salmonella* spp. were non-susceptible to grepafloxacin. Gemifloxacin and ciprofloxacin are the most active compounds tested. A high rate of quinolone-resistant *C. jejuni* was detected.

Gemifloxacin possesses a broad spectrum of antibacterial activity that includes both Gram negative and Gram positive pathogens. Classic oral antimicrobials used against enteropathogenic bacterial isolates have recently demonstrated poor *in vitro* activity, emphasizing the need to test new quinolones against these organisms.

A total of 288 enteropathogenic bacterial isolates from patients with acute gastroenteritis were studied, including 106 *Salmonella* spp., 32 *Hafnia alvei, 22 Yersinia enterocolitica,* 21 *Shigella* spp., 16 *Aeromonas* spp. and 91 *Campylobacter jejuni*. The microrganisms were isolated during the period 1996-99 and strains were stored in skimmed milk at -80°C until studied. The eight quinolones tested were gemifloxacin, trovafloxacin, grepafloxacin, ciprofloxacin, ofloxacin, norfloxacin, levofloxacin and nalidixic acid. MICs were determined by an agar dilution method (NCCLS, *4th ed.,* M7-A4, Villanova, PA (1997)) on Mueller-Hinton agar supplemented with 5% sheep blood for *C. jejuni* isolates. The plates were incubated aerobically at 35°C for 24 h, except for *C. jejuni,* where a microaerophilic atmosphere was obtained using Campy Pack and incubation was for 48 h. All organisms were tested with an inoculum of approximately 10⁴ CFU/spot. MICs were defined as the lowest antimicrobial concentration at which there was no visible bacterial growth. *Escherichia coli* ATCC 25922, *Staphylococcus aureus* ATCC 29213 and *Pseudomonas aeruginosa* ATCC 27853 were used as controls. The antimicrobial susceptibility breakpoints used to define the percentage of susceptible isolates are as follows: gemifloxacin, trovafloxacin, grepafloxacin and ciprofloxacin 1 µg/ml; ofloxacin and levofloxacin 2 µg/ml; norfloxacin 4 µg/ml; and nalidixic acid 16 µg/ml.

Table 1 shows the activity of gemifloxacin and seven other quinolones against 288 enteropathogenic bacterial strains. Table 2 shows the MICs of all quinolones tested against six *Salmonella* spp. (*S. hadar,* n = 2; *S. virchow,* n = 2; *S. tshiongwe,* n = 1; and *S. newport, n* = 1) in which MICs for ciprofloxacin were ≥0.25 µg/ml, which is considered by some authors to be clinically significant resistance. The activity of gemifloxacin against the six groups of enteropathogenic bacterial strains herein was measured.

All *H. alvei* and *Shigella* spp. isolates are susceptible to all quinolones studied at the breakpoint stated. Of 106 *Salmonella spp.* studied, 27% of isolates are not susceptible to nalidixic acid and 1% are not susceptible to grepafloxacin. All strains are susceptible to the other six quinolones. Among the isolates of *Y. enterocolitica* and *Aeromonas* spp. studied, 100% are susceptible to the quinolones tested, except for nalidixic acid, where the proportions of non-susceptible strains are 4.5% and 6.3%, respectively. A high incidence of quinolone-resistant *C*. *jejuni* is detected, with only 31.9% of strains being susceptible to all the antimicrobials tested. MIC₅₀s are equal to MIC₉₀s, except for *Salmonella* spp. and *C. jejuni* where the MICs differ by 1-2 dilutions. Against six *Salmonella* spp. with ciprofloxacin MICs ≥0.25 µg/ml (considered by some authors to be clinically significant resistance), MICs of gemifloxacin are 1-4 dilutions lower than those of the other fluoroquinolones studied. By weight, gemifloxacin and ciprofloxacin are the most active compounds tested in this study.

**Table 1.**

| The *In Vitro* activity of gemifloxacin and seven other quinolones against Enteropathogenic Bacterial Strains. | | | | |
|---|---|---|---|---|
| Isolates and antimicrobials | MIC (µg/ml) | | | |
| | Range | 50% | 90% | % Susceptible |
| *Salmonella* spp. (n = 106) | | | | |
| Gemifloxacin | ≤0.015-0.25 | 0.03 | 0.12 | 100 |
| Trovafloxacin | ≤0.015-1 | 0.06 | 0.25 | 100 |
| Grepafloxacin | 0.03-2 | 0.06 | 0.25 | 99 |
| Ciprofloxacin | ≤0.015-1 | 0.03 | 0.12 | 100 |
| Ofloxacin | 0.06-2 | 0.12 | 0.5 | 100 |
| Norfloxacin | 0.06-4 | 0.06 | 0.5 | 100 |
| Levofloxacin | 0.06-1 | 0.06 | 0.25 | 100 |
| Nalidixic acid | 2->128 | 4 | >128 | 73 |

| *Hafnia alvei* (n = 32) | | | | |
|---|---|---|---|---|
| Gemifloxacin | ≤0.015-0.06 | 0.03 | 0.03 | 100 |
| Trovafloxacin | 0.03-0.12 | 0.06 | 0.06 | 100 |
| Grepafloxacin | ≤0.015-0.06 | 0.03 | 0.06 | 100 |
| Ciprofloxacin | ≤0.015 | ≤0.015 | ≤0.015 | 100 |
| Ofloxacin | 0.03 | 0.03 | 0.03 | 100 |
| Norfloxacin | ≤0.015-0.03 | 0.03 | 0.03 | 100 |
| Levofloxacin | ≤0.015-0.03 | 0.03 | 0.03 | 100 |
| Nalidixic acid | 1-2 | 2 | 2 | 100 |

| *Yersinia enterocolitica* (n = 22) | | | | |
|---|---|---|---|---|
| Gemifloxacin | ≤0.015-0.12 | 0.03 | 0.03 | 100 |
| Trovafloxacin | ≤0.015-0.25 | 0.06 | 0.06 | 100 |
| Grepafloxacin | ≤0.015-0.25 | 0.03 | 0.03 | 100 |
| | | | | |
| Ciprofloxacin | ≤0.015-0.25 | 0.03 | 0.03 | 100 |
| Ofloxacin | 0.03-0.5 | 0.12 | 0.12 | 100 |
| Norfloxacin | 0.06-0.5 | 0.06 | 0.06 | 100 |
| Levofloxacin | 0.03-0.5 | 0.06 | 0.06 | 100 |
| Nalidixic acid | 0.5->128 | 2 | 2 | 95.5 |

| *Shigella* spp. (n = 21) | | | | |
|---|---|---|---|---|
| Gemifloxacin | ≤0.015 | ≤0.015 | ≤0.015 | 100 |
| Trovafloxacin | ≤0.015 | ≤0.015 | ≤0.015 | 100 |
| Grepafloxacin | ≤0.015-0.03 | ≤0.015 | 0.03 | 100 |
| Ciprofloxacin | ≤0.015 | ≤0.015 | ≤0.015 | 100 |
| Ofloxacin | ≤0.015-0.06 | 0.03 | 0.06 | 100 |
| Norfloxacin | 0.03-0.06 | 0.06 | 0.06 | 100 |
| Levofloxacin | ≤0.015-0.03 | 0.03 | 0.03 | 100 |
| Nalidixic acid | 1-2 | 1 | 2 | 100 |

| *Aeromonas* spp. (n = 16) | | | | |
|---|---|---|---|---|
| Gemifloxacin | ≤0.015-0.12 | ≤0.015 | 0.03 | 100 |
| Trovafloxacin | ≤0.015-0.25 | ≤0.015 | 0.03 | 100 |
| Grepafloxacin | ≤0.015-0.12 | 0.03 | 0.06 | 100 |
| Ciprofloxacin | ≤0.015-0.06 | ≤0.015 | ≤0.015 | 100 |
| Ofloxacin | ≤0.015-0.12 | ≤0.015 | 0.03 | 100 |
| Norfloxacin | ≤0.015-0.12 | ≤0.015 | 0.03 | 100 |
| Levofloxacin | ≤0.015-0.06 | ≤0.015 | ≤0.015 | 100 |
| Nalidixic acid | 0.06->128 | 0.12 | 0.25 | 93.75 |

| *Campylobacter jejuni* | | | | |
|---|---|---|---|---|
| (n=91) | | | | |
| Gemifloxacin | 0.03-128 | 32 | 128 | 31.86 |
| Trovafloxacin | ≤0.015-32 | 8 | 8 | 31.86 |
| Grepafloxacin | 0.03-128 | 32 | 64 | 31.86 |
| Ciprofloxacin | 0.06-128 | 16 | 64 | 31.86 |
| | | | | |
| Ofloxacin | 0.06->128 | 16 | 32 | 31.86 |
| Norfloxacin | 0.12->128 | 128 | >128 | 31.86 |
| Levofloxacin | 0.06-128 | 8 | 32 | 31.86 |
| Nalidixic acid | 2->128 | >128 | >128 | 30.76 |

**Table 2.**

| **MICs of Quinolones Tested Against Six** ***Salmonella*** **spp. in Which MICs for Ciprofloxacin were ≥0.25 µg/ml** | | | | |
|---|---|---|---|---|
| Antimicrobial | MIC (µg/ml) | | | |
| | *S. virchow* (n = 2) | *S. hadar* (n = 2) | *S. tshiongwe* (n = 1) | *S. newport* (n = 1) |
| Gemifloxacin | 0.12 | 0.25 | 0.25 | 0.25 |
| Trovafloxacin | 0.25 | 0.5 | 1 | 0.5 |
| Grepafloxacin | 0.25 | 1 | 2 | 1 |
| Ciprofloxacin | 0.25 | 0.5 | 1 | 0.5 |
| Ofloxacin | 0.5 | 2 | 2 | 2 |
| Norfloxacin | 0.5 | 4 | 4 | 2 |
| Levofloxacin | 0.25 | 1 | 1 | 1 |
| Nalidixic acid | >128 | >128 | >128 | >128 |

The invention provides a method for modulating metabolism of enteropathogenic bacteria. Skilled artisans can readily choose enteropathogenic bacteria or patients infected with or suspected to be infected with these organisms to practice the methods of the invention. Alternatively, the bacteria useful in the methods of the invention may be those described herein.

Also provided by the invention is a method of treating or preventing a bacterial infection by enteropathogenic bacteria comprising the step of administering an antibacterially effective amount of a composition comprising a quinolone, particularly a gemifloxacin compound to a mammal, preferably a human, suspected of having or being at risk of having an infection with enteropathogenic bacteria.

While a preferred object of the invention provides a method wherein said enteropathogenic bacteria is selected from the group consisting of: *Salmonella* spp. (e.g., *S*. *hadar, S. virchow, S. tshiongwe,* and *S. newport*), *Hafnia alvei, Yersinia enterocolitica, Shigella* spp., *Aeromonas* spp. and *Campylobacter jejuni*. Other enteropathogenic bacteria may also be included in the methods. The skilled artisan may identify these organisms as provided herein as well as using other methods known in the art, *e.g.* MIC tests.

The present invention provides, among other things, methods for using a composition comprising a quinolone, particularly a gemifloxacin compound against Gram positive bacteria.

This invention was based, in part, on analyses evaluating the comparative activity of gemifloxacin against various Gram positive *cocci* pathogens. An objective of these analyses was to determine the MICs of gemifloxacin and comparator quinolones.

Gemifloxacin was compared to other quinolones ciprofloxacin, ofloxacin, levofloxacin and trovafloxacin (CIP = ciprofloxacin, OFL = ofloxacin, LEV = levofloxacin, TRO = trovafloxacin, GEM = gemifloxacin) and with that of other commonly used antimicrobials (penicillin, amoxycillin/clavulanate, cefuroxime, ceftriaxone, co-trimoxazole and azithromycin). The organisms studied were strains of *Streptococcus pneumoniae, Haemophilus influenzae*, *Moraxella catarrhalis, Streptococcus pyogenes, Staphylococcus aureus* and *Enterococcus faecalis* isolated during 1998-99 in three Medical Centers in Buenos Aires, Argentina. The macrodilution agar method was used following the NCCLS guidelines. From 90 S. pneumoniae isolates studied, 76.7% were penicillin susceptible, 18.9% were penicillin intermediate and 4.4% were penicillin resistant. A total of 18.5% of H. influenzae isolates were β-lactamase positive and 17% of S. aureus isolates were methicillin resistant. Certain results of this study indicate that gemifloxacin has enhanced activity against Gram positive bacteria compared with the other quinolones tested.

Gemifloxacin has enhanced activity against Gram positive organisms, particularly *S*. *pneumoniae,* including strains of pneumococci resistant to other quinolones. This study compares the *in vitro* activity of gemifloxacin with that of other quinolones and commonly used antimicrobials.

A total of 411 fresh clinical bacterial isolates obtained during 1998-99 and considered to have clinical significance were investigated. The following antimicrobials were tested: gemifloxacin, ofloxacin, levofloxacin, trovafloxacin, ciprofloxacin, penicillin, co-trimoxazole, azithromycin, amoxycillin/clavulanate, cefuroxime and ceftriaxone.

MICs were determined using the agar dilution method following NCCLS recommended procedures. The nitrocefin method was used to determine β-lactamase production in *H*. *influenzae* and *M. catarrhalis.* The methicillin resistance of *S. aureus* was evaluated using the Kirby-Bauer disk diffusion method with oxacillin disks.

The distribution of the organisms studied is given in Table 3. The antimicrobial activity of gemifloxacin and comparator quinolones is shown in Table 4. The activity of quinolones against *S. pneumoniae* is given in Tables 5 and 6 and that against *S. aureus* in Table 7.

Gemifloxacin has excellent *in vitro* activity against Gram positive *cocci.* Gemifloxacin has enhanced activity against *S. pneumoniae,* with MIC₉₀s of 0.03 µg/ml for penicillin-susceptible and penicillin-resistant strains. Gemifloxacin maintains excellent activity against ciprofloxacin-resistant strains (MIC ≥4 µg/ml) of *S. pneumoniae*. Gemifloxacin demonstrated excellent activity against *S. aureus,* with MIC₉₀s of 0.03 µg/ml for methicillin-susceptible strains. Gemifloxacin shows excellent *in vitro* activity against all isolates of *H. influenzae* (MIC₉₀ 0.008 µg/ml) and *M*. *catarrhalis* (MIC₉₀ 0.008 µg/ml).

**Table 3.**

| Distribution of the Organisms Studied | | |
|---|---|---|
| Microrganism | n | % |
| *Streptococcus pneumoniae* | 90 | 100 |
| Penicillin susceptible | 69 | 76.7 |
| Penicillin intermediate | 17 | 18.9 |
| Microrganism | n | % |
| Penicillin resistant | 4 | 4.4 |
| *Haemophilus influenzae* | 65 | 100 |
| β-Lactamase positive | 12 | 18.5 |
| Ciprofloxacin resistant | 1 | 1.5 |
| *Moraxella catarrhalis* | 59 | 100 |
| β-Lactamase positive | 57 | 96.6 |
| *Streptococcus pyogenes* | 69 | 100 |
| Macrolide resistant | 4 | 5.8 |
| *Staphylococcus aureus* | 80 | 100 |
| Methicillin resistant | 14 | 17.5 |
| *Enterococcus faecalis* | 48 | 100 |

**Table 4.**

| *In Vitro* Activity of Gemifloxacin and Comparator Quinolones | | | |
|---|---|---|---|
| Microorganism | Antimicrobial | MIC (µg/ml) | |
| | | Range | MIC₉₀ |
| *Streptococcus pneumoniae (n = 90)* | Gemifloxacin | ≤0.004-0.125 | 0.03 |
| | Ciprofloxacin | 0.5-16 | 2 |
| | Ofloxacin | 1-16 | 2 |
| | Levofloxacin | 0.25-4 | 1 |
| | Trovafloxacin | 0.016-1 | 0.25 |
| *Haemophilus influenzae (n = 65)* | Gemifloxacin | ≤0.004-0.125 | 0.008 |
| | Ciprofloxacin | ≤0.004-2 | 0.03 |
| | Ofloxacin | 0.008-0.25 | 0.06 |
| | Levofloxacin | ≤0.004-0.25 | 0.016 |
| | Trovafloxacin | ≤0.004-0.5 | 0.03 |
| *Moraxella catarrhalis (n = 59)* | Gemifloxacin | ≤0.004-0.016 | 0.008 |
| | Ciprofloxacin | 0.008-0.03 | 0.03 |
| | Ofloxacin | 0.06-0.125 | 0.125 |
| | Levofloxacin | ≤0.004-0.03 | 0.03 |
| | Trovafloxacin | ≤0.004-0.03 | 0.016 |
| *Streptococcus pyogenes (n = 69)* | Gemifloxacin | ≤0.004-0.125 | 0.03 |
| | Ciprofloxacin | 0.125-4 | 1 |
| | Ofloxacin | 0.5-4 | 2 |
| | Levofloxacin | 0.25-2 | 0.5 |
| | Trovafloxacin | 0.06-1 | 0.125 |
| *Staphylococcus aureus (n = 80)* | Gemifloxacin | ≤0.004-2 | 1 |
| | Ciprofloxacin | 0.125->32 | 16 |
| | Ofloxacin | 0.125->32 | 8 |
| | Levofloxacin | 0.06-8 | 4 |
| | Trovafloxacin | ≤0.004-8 | 2 |
| *Enterococcus faecalis (n = 48)* | Gcmifloxacin | 0.008-8 | 2 |
| | Ciprofloxacin | 0.5->32 | >32 |
| | Ofloxacin | 0.125->32 | >32 |
| | Levofloxacin | 0.125->32 | >32 |
| | Trovafloxacin | ≤0.004-16 | 8 |
| Gemifloxacin | 0.03 | 0.03 | 0.03 |

**Table 5.**

| Comparative *In Vitro* Activity of Antimicrobials Against *S. pneumoniae* Strains | | | |
|---|---|---|---|
| Antimicrobial | MIC₉₀ (µg/ml) | | |
| | Penicillin susceptible (n = 69) | Penicillin intermediate (n = 17) | Penicillin resistant (n = 4) |
| Amoxycillin/clavulanate | 0.016 | 1 | 1 |
| Cefuroxime | 0.03 | 4 | 4 |
| Ceftriaxone | 0.016 | 1 | 1 |
| Co-trimoxazole | 8 | 8 | >16 |
| Azithromycin | 0.06 | 1 | 0.06 |
| Ciprofloxacin | 2 | 2 | 2 |
| Ofloxacin | 2 | 2 | 1 |
| Levofloxacin | 1 | 1 | 1 |
| Trovafloxacin | 0.25 | 0.25 | 0.125 |

**Table 6.**

| Comparative *In Vitro* Activity of Quinolones Against Ciprofloxacin-resistant *S. pneumoniae* Strains (in the Absence of NCCLS Breakpoint Criteria MICs ≥4 µg/ml Were Considered to be Resistant) | | | | |
|---|---|---|---|---|
| | | MIC (µg/ml) | | |
| | Strain 1 | Strain 2 | Strain 3 | Strain 4 |
| Ciprofloxacin | 4 | 4 | 16 | 16 |
| Ofloxacin | 2 | 2 | 16 | 8 |
| Levofloxacin | 1 | 1 | 4 | 2 |
| Trovafloxacin | 0.25 | 0.025 | 1 | 1 |
| Gemifloxacin | 0.03 | 0.06 | 0.125 | 0.06 |

**Table 7.**

| Comparative *In Vitro* Activity of Fluoroquinolones in *S. aureus* Strains | | | | |
|---|---|---|---|---|
| | MIC₉₀ (µg/ml) | | Resistance (%) | |
| | Methicillin susceptible (n = 66) | Methicillin resistant (n = 14) | Methicillin susceptible (n = 66) | Methicillin resistant (n = 14) |
| Ciprofloxacin | 0.5 | >32 | 0 | 100 |
| Ofloxacin | 0.5 | 16 | 0 | 100 |
| Levofloxacin | 0.25 | 8 | 0 | 100 |
| Trovafloxacin | 0.06 | 4 | 0 | 7 |
| Gemifloxacin | 0.03 | 2 | 0 | 0 |

The invention provides a method for modulating metabolism of Gram positive pathogenic bacteria. Skilled artisans can readily choose Gram positive pathogenic bacteria or patients infected with or suspected to be infected with these organisms to practice the methods of the invention. Alternatively, the bacteria useful in the methods of the invention may be those described herein.

Also provided by the invention is a method of treating or preventing a bacterial infection by Gram positive pathogenic bacteria comprising the step of administering an antibacterially effective amount of a composition comprising a quinolone, particularly a gemifloxacin compound to a mammal, preferably a human, suspected of having or being at risk of having an infection with Gram positive pathogenic bacteria.

While a preferred object of the invention provides a method wherein said Gram positive pathogenic bacteria is selected from the group consisting of: *Streptococcus pneumoniae, Haemophilus influenzae, Moraxella catarrhalis, Streptococcus pyogenes, Staphylococcus aureus* and *Enterococcus faecalis*. Other Gram positive pathogenic bacteria may also be included in the methods. The skilled artisan may identify these organisms as provided herein as well as using other methods known in the art, *e.g.* MIC tests.

The present invention provides, among other things, methods for using a composition comprising a quinolone, particularly a gemifloxacin compound against respiratory tract pathogenic bacteria.

This invention was based, in part, on analyses evaluating the comparative activity of gemifloxacin against various respiratory tract pathogens. An objective of these analyses was to determine the MIC₉₀s of gemifloxacin and comparator drugs against strains of *Streptococcus pneumoniae, Streptococcus pyogenes, Haemophilus influenzae, Moraxella catarrhalis, Staphylococcus aureus* and *Klebsiella pneumoniae.*

Gemifloxacin was compared to ciprofloxacin, clinafloxacin, levofloxacin, moxifloxacin, sparfloxacin, trovafloxacin and cefuroxime against 450 respiratory tract pathogens (GEM = gemifloxacin, CIP = ciprofloxacin, CLI = clinafloxacin, LEV = levofloxacin, MOX = moxifloxacin, SPA = sparfloxacin, TRO = trovafloxacin, CEF = cefuroxime).

Using NCCLS reference broth microdilution methodology, 100 strains of *Streptococcus pneumoniae* (18 penicilin resistant, 16 penicillin intermediate, 20 penicillin susceptible, 30 erythromycin resistant, 16 multi-drug resistant), 100 *Streptococcus pyogenes* (60 erythromycin resistant, 20 multi-drug resistant, 20 fully susceptible), 100 *Haemophilus influenzae* (20 β-lactamase positive), 50 *Moraxella catarrhalis* (37 β-lactamase positive), 50 *Staphylococcus aureus* (20 β-lactamase positive, 10 erythromycin resistant) and 50 *Klebsiella pneumoniae (20* extended spectrum β-lactamase positive) were tested. The MIC₉₀s of gemifloxacin and comparator drugs are listed in Tables 8-13.

Gemifloxacin is 4-64 times more potent than that of all comparator agents against *S*. *pneumoniae.* Gemifloxacin activity against *S. pyogenes* is 2-32 fold higher than that of the other fluoroquinolones tested. Gemifloxacin and clinafloxacin has superior activity than other test drugs against *H. influenzae* and *M. catarrhalis.* Against *S*. *aureus* and *K. pneumoniae*, the new fluoroquinolone display the lowest MIC₉₀. These results indicate a role for gemifloxacin in the treatment of community-acquired respiratory tract infections.

Bacterial resistance to antimicrobials, if widespread, can translate into failure of treatment for any type of infection. The appearance of new drug is therefore met with great expectations, particularly if the novel molecule displays both a relevant spectrum of antibacterial activity and the ability to overcome resistance. The worldwide increase in the number of respiratory tract pathogens resistant to antimicrobials documented in the past few years has made the development of more potent drugs to treat these microrganisms crucial.

Gemifloxacin possesses a spectrum bactericidal activity against Gram positive and Gram negative, aerobic and anaerobic species, and is being investigated in phase III clinical trials (Paek, *et al.,* 38th ICAAC, Abstract F-092 (1998); Kelly, *et al.,* 38th ICAAC, Abstract F-087 (1998)). As the incidence of bacterial resistance is dictated by patterns of antimicrobial usage and this is known to vary widely in different geographic settings, it is important to explore the efficacy of any newly developed compound to establish its usefulness in various environmental niches. To this end, gemifloxacin has been tested against 450 recently isolated respiratory tract pathogens.

The following microrganisms were studied: 100 *Streptococcus pneumoniae* (18 penicillin-resistant, 16 penicillin-intermediate, 20 penicillin-susceptible, 30 erythromycin-resistant and 16 multi-drug-resistant strains), 100 *Streptococcus pyogenes* (60 erythromycin-resistant, 20 multi-drug-resistant and 20 fully susceptible strains), 100 *Haemophilus influenzae* (20 β-lactamase-producing strains), 50 *Moraxella catarrhalis* (37 β-lactamase-producing strains), 50 *Staphylococcus aureus* (20 β-lactamase producers and 10 erythromycin-resistant strains) and 50 *Klebsiella pneumoniae* (20 extended-spectrum, β-lactamase-producing strains).

The activity of gemifloxacin was compared with that of ciprofloxacin, clinafloxacin, levofloxacin, moxifloxacin, sparfloxacin, trovafloxacin and cefuroxime using NCCLS reference broth microdilution methodology (NCCLS, *4th ed*., pages M7-A3 and M100-S8, Wayne, PA (1998)).

The *in vitro* susceptibilities of gemifloxacin and comparator drugs against 100 *S*. *pneumoniae* strains are reported in Table 8. Gemifloxacin emerged as the most potent antimicrobial among those studied (MIC₉₀ 0.03 µg/ml). Clinafloxacin and trovafloxacin had the same MIC₉₀ (0.12 µg/ml), while moxifloxacin, sparfloxacin and levofloxacin had MIC₉₀s of 0.25, 0.5 and 1 µg/ml, respectively. Ciprofloxacin and cefuroxime displayed the highest MIC₉₀ (2 µg/ml). When strains of *S. pneumoniae* are considered according to their level of penicillin susceptibility, gemifloxacin is again the most potent drug tested against penicillin-intermediate and penicillin-resistant microrganisms.

Gemifloxacin and cefuroxime have comparable activity against penicillin-susceptible *S. pneumoniae* (MIC₉₀s 0.03 and 0.015 µg/ml, respectively). The same was true when macrolide-resistant *pneumococci* were considered: gemifloxacin has the lowest MIC₉₀.

MICs of gemifloxacin and the other drugs against 100 *S. pyogenes* strains are listed in Table 9. Gemifloxacin (MIC₉₀ 0.03 µg/ml) and cefuroxime (MIC₉₀ 0.015 µg/ml) had the lowest MIC₉₀s, followed by clinafloxacin (0.06 µg/ml), moxifloxacin and trovafloxacin (0.25 µg/ml), levofloxacin and ciprofloxacin (0.5 µg/ml) and sparfloxacin (1 µg/ml). Against *S. pyogenes,* gemifloxacin and cefuroxime emerged as the most effective drugs, irrespective of the mechanism of macrolide resistance the bacteria possessed.

Table 10 reports the activities of the drugs against 100 *H. influenzae* strains. Gemifloxacin and clinafloxacin were the most potent drugs tested (MIC₉₀≤0.00075 µg/ml) Ciprofloxacin, sparfloxacin and levofloxacin possessed good activity (MIC₉₀ 0.03 µg/ml). Trovafloxacin and moxifloxacin had a MIC₉₀ of 0.06 µg/ml. Cefuroxime was the least potent drug (MIC₉₀ 1 µg/ml).

**Table 8.**

| MICs of Gemifloxacin and Comparative Drugs Against 100 Strains of *S. pneumoniae* | | | |
|---|---|---|---|
| Microrganism and antimicrobial | MIC (µg/ml) | | |
| | Range | MIC₅₀ | MIC₉₀ |
| *Streptococcus pneumoniae* (n = 100) | | | |
| Gemifloxacin | ≤0.0075-0.03 | 0.015 | 0.03 |
| Ciprofloxacin | 0.06-4 | 1 | 2 |
| Trovafloxacin | 0.03-0.25 | 0.12 | 0.12 |
| Sparfloxacin | 0.12-1 | 0.5 | 0.5 |
| Clinafloxacin | 0.03-0.25 | 0.06 | 0.12 |
| Moxifloxacin | 0.03-0.25 | 0.12 | 0.25 |
| Levofloxacin | 0.25-2 | 1 | 1 |
| Cefuroxime | ≤0.0075-8 | 0.03 | 2 |

| *Streptococcus pneumoniae* (penicillin susceptible) (n = 20) | | | |
|---|---|---|---|
| Gemifloxacin | ≤0.0075-0.03 | 0.015 | 0.03 |
| Ciprofloxacin | 0.06-2 | 1 | 2 |
| Trovafloxacin | 0.06-0.12 | 0.12 | 0.12 |
| Sparfloxacin | 0.12-0.5 | 0.25 | 0.25 |
| Clinafloxacin | 0.015-0.12 | 0.06 | 0.12 |
| Moxifloxacin | 0.06-0.25 | 0.25 | 0.25 |
| Levofloxacin | 0.25-1 | 1 | 1 |
| Cefuroxime | ≤0.0075-0.015 | 0.015 | 0.015 |

| *Streptococcus pneumoniae* (penicillin resistant) (n = 18) | | | |
|---|---|---|---|
| Gemifloxacin | ≤0.0075-0.06 | ≤0.0075 | 0.06 |
| Ciprofloxacin | 0.5-8 | 0.5 | 8 |
| Trovafloxacin | 0.06-0.25 | 0.12 | 0.12 |
| Sparfloxacin | 0.25-1 | 0.5 | 1 |
| Clinafloxacin | 0.06-0.25 | 0.12 | 0.25 |
| | | | |
| Moxifloxacin | 0.03-0.12 | 0.06 | 0.12 |
| Levofloxacin | 0.5-2 | 0.5 | 1 |
| Cefuroxime | 2-8 | 2 | 8 |

| *Streptococcus pneumoniae* (penicillin intermediate) (n = 16) | | | |
|---|---|---|---|
| Gemifloxacin | ≤0.0075-0.03 | 0.015 | 0.015 |
| Ciprofloxacin | 0.5-2 | 1 | 1 |
| Trovafloxacin | 0.06-0.12 | 0.12 | 0.12 |
| Sparfloxacin | 0.5-1 | 0.5 | 0.5 |
| Clinafloxacin | 0.06-0.12 | 0.12 | 0.12 |
| Moxifloxacin | 0.06 | 0.06 | 0.06 |
| Levofloxacin | 0.5 - 1 | 1 | 1 |
| Cefuroxime | 0.12-8 | 2 | 2 |

| *Streptococcus pneumoniae* (multi-drug resistant)* (n = 16) | | | |
|---|---|---|---|
| Gemifloxacin | ≤0.0075-0.03 | 0.015 | 0.03 |
| Ciprofloxacin | 0.5-4 | 1 | 2 |
| Trovafloxacin | 0.06-0.12 | 0.06 | 0.12 |
| Sparfloxacin | 0.12-0.5 | 0.5 | 0.5 |
| Clinafloxacin | 0.06-0.12 | 0.12 | 0.12 |
| Moxifloxacin | 0.06-0.25 | 0.06 | 0.12 |
| Levofloxacin | 0.5-1 | 1 | 1 |
| Cefuroxime | 0.015-0.25 | 0.03 | 0.03 |

| *Streptococcus pneumoniae* (erythromycin resistant MLS_{B} phenotype) (n = 16) | | | |
|---|---|---|---|
| Gemifloxacin | ≤0.0075-0.03 | 0.03 | 0.015 |
| Ciprofloxacin | 1-2 | 1 | 2 |
| Trovafloxacin | 0.06-0.12 | 0.06 | 0.12 |
| Sparfloxacin | 0.12-0.5 | 0.25 | 0.25 |
| Clinafloxacin | 0.03-0.12 | 0.06 | 0.12 |
| Moxifloxacin | 0.12-0.25 | 0.25 | 0.25 |
| Levofloxacin | 0.5-2 | 1 | 1 |
| Cefuroxime | 0.015-0.5 | 0.015 | 0.03 |

| *Streptococcus pneumoniae* (erythromycin resistant M phenotype) (n = 16) | | | |
|---|---|---|---|
| Gemifloxacin | 0.015-0.05 | 0.015 | 0.03 |
| Ciprofloxacin | 1-2 | 1 | 2 |
| | | | |
| Trovafloxacin | 0.06-0.12 | 0.06 | 0.12 |
| Sparfloxacin | 0.25 | 0.25 | 0.25 |
| Clinafloxacin | 0.03-0.12 | 0.06 | 0.12 |
| Moxifloxacin | 0.12-0.25 | 0.25 | 0.25 |
| Levofloxacin | 1-2 | 1 | 1 |
| Cefuroxime | ≤0.0075-0.12 | 0.015 | 0.12 |
| *Multi-drug resistant = simultaneously resistant to erythromycin, co-trimoxazole, tetracycline and chloramphenicol | | | |

**Table 9.**

| MICs of Gemifloxacin and Comparative Drugs Against 100 Strains of *S. pyogenes* | | | |
|---|---|---|---|
| Microrganism and antimicrobial | MIC (µg/ml) | | |
| | Range | MIC₅₀ | MIC₉₀ |
| *Streptococcus pyogenes* (n = 100) | | | |
| Gemifloxacin | ≤0.00375-0.06 | 0.015 | 0.03 |
| Ciprofloxacin | 0.12-2 | 0.25 | 0.5 |
| Trovafloxacin | 0.03-1 | 0.12 | 0.25 |
| Sparfloxacin | 0.06-1 | 0.5 | 1 |
| Clinafloxacin | 0.015-0.12 | 0.06 | 0.06 |
| Moxifloxacin | 0.06-0.5 | 0.12 | 0.25 |
| Levofloxacin | 0.06-2 | 0.5 | 0.5 |
| Cefuroxime | ≤0.00375-0.015 | 0.0075 | 0.015 |

| *Streptococcus pyogenes* (erythromycin resistant C phenotype [MLS_{B}]) (n = 20) | | | |
|---|---|---|---|
| Gemifloxacin | ≤0.00375-0.03 | 0.015 | 0.015 |
| Ciprofloxacin | 0.25-0.5 | 0.25 | 0.25 |
| Trovafloxacin | 0.03-0.12 | 0.06 | 0.12 |
| Sparfloxacin | 0.25-1 | 0.25 | 0.25 |
| Clinafloxacin | 0.03-0.12 | 0.06 | 0.12 |
| Moxifloxacin | 0.12-0.25 | 0.25 | 0.25 |
| Levofloxacin | 0.25-1 | 0.25 | 0.25 |
| Cefuroxime | ≤0.00375 - 0.015 | ≤0.00375 | 0.015 |

| *Streptococcus pyogenes* (erythromycin resistant M phenotype) (n = 20) | | | |
|---|---|---|---|
| Gemifloxacin | ≤0.00375-0.03 | 0.015 | 0.015 |
| Ciprofloxacin | 0.12-0.5 | 0.25 | 0.5 |
| Trovafloxacin | 0.06-0.5 | 0.12 | 0.12 |
| | | | |
| Sparfloxacin | 0.25-1 | 0.5 | 0.5 |
| Clinafloxacin | 0.015-0.06 | 0.03 | 0.06 |
| Moxifloxacin | 0.06-0.25 | 0.12 | 0.25 |
| Levofloxacin | 0.06-1 | 0.5 | 0.5 |
| Cefuroxime | ≤0.00375-0.015 | ≤0.00375 | 0.015 |

| *Streptococcus pyogenes* (erythromycin resistant I phenotype [MLS_{B}]) (n = 20) | | | |
|---|---|---|---|
| Gemifloxacin | ≤0.00375-0.06 | 0.015 | 0.03 |
| Ciprofloxacin | 0.12-2 | 0.25 | 0.5 |
| Trovafloxacin | 0.06-0.5 | 0.12 | 0.5 |
| Sparfloxacin | 0.06-1 | 0.5 | 0.5 |
| Clinafloxacin | 0.03-0.06 | 0.06 | 0.06 |
| Moxifloxacin | 0.06-0.5 | 0.25 | 0.25 |
| Levofloxacin | 0.25-2 | 0.25 | 0.25 |
| Cefuroxime | ≤0.00375-0.01 | ≤0.00375 | 0.015 |

| *Streptococcus pyogenes* (fully susceptible) (n = 20) | | | |
|---|---|---|---|
| Gemifloxacin | 0.0075-0.03 | 0.015 | 0.03 |
| Ciprofloxacin | 0.12-2 | 0.25 | 0.5 |
| Trovafloxacin | 0.06-1 | 0.12 | 0.25 |
| Sparfloxacin | 0.25-1 | 0.5 | 1 |
| Clinafloxacin | 0.03-0.12 | 0.03 | 0.06 |
| Moxifloxacin | 0.06-0.25 | 0.12 | 0.25 |
| Levofloxacin | 0.25-2 | 0.5 | 0.5 |
| Cefuroxime | 0.0075-0.015 | 0.0075 | 0.015 |

| *Streptococcus pyogenes* (multi-drug resistant)* (n = 20) | | | |
|---|---|---|---|
| Gemifloxacin | 0.0075-0.015 | 0.015 | 0.03 |
| Ciprofloxacin | 0.25-1 | 0.5 | 0.5 |
| Trovafloxacin | 0.12-0.25 | 0.12 | 0.25 |
| Sparfloxacin | 0.25-1 | 1 | 1 |
| Clinafloxacin | 0.03-0.06 | 0.03 | 0.06 |
| Moxifloxacin | 0.06-0.12 | 0.12 | 0.12 |
| Levofloxacin | 0.25-1 | 0.5 | 0.5 |
| Cefuroxime | 0.0075-0.015 | 0-0075 | 0.015 |

| | | | |
|---|---|---|---|
| *Multi-drug resistant = simultaneously resistant to erythromycin, clindamycin and tetracycline | | | |

**Table 10.**

| MICs of Gemifloxacin and Comparative Drugs Against 100 strains of *H. influenzae* | | | |
|---|---|---|---|
| Microrganism and antimicrobial | MIC (µg/ml) | | |
| | Range | MIC₅₀ | MIC₉₀ |
| *Haemophilus influenzae* (n = 100) | | | |
| Gemifloxacin | ≤0.0075-0.06 | ≤0.0075 | ≤0.00075 |
| Ciprofloxacin | ≤0.0075-0.5 | ≤0.00075 | 0.015 |
| Trovafloxacin | ≤0.0075-0.25 | ≤0.00075 | 0.06 |
| Sparfloxacin | ≤0.0075-0.25 | 0.01 | 0.03 |
| Clinafloxacin | ≤0.0075-0.06 | ≤0.00075 | ≤0.00075 |
| Moxifloxacin | ≤0.0075-0.12 | 0.03 | 0.06 |
| Levofloxacin | ≤0.0075-0.5 | 0.015 | 0.03 |
| Cefuroxime | 0.12-4 | 0.5 | 1 |

| *Haemophilus influenzae* (β-lactamase negative) (n = 80) | | | |
|---|---|---|---|
| Gemifloxacin | ≤0.0075-0.015 | ≤0.0075 | ≤0.0075 |
| Ciprofloxacin | ≤0.0075-0.5 | ≤0.0075 | 0.015 |
| Trovafloxacin | ≤0.0075-0.25 | ≤0.0075 | 0.015 |
| Sparfloxacin | ≤0.0075-0.25 | 0.015 | 0.25 |
| Clinafloxacin | ≤0.0075-0.06 | ≤0.0075 | ≤0.0075 |
| Moxifloxacin | ≤0.0075-0.06 | 0.03 | 0.06 |
| Levofloxacin | ≤0.0075-0.5 | 0.015 | 0.015 |
| Cefuroxime | 0.25-1 | 0.5 | 1 |

| *Haemophilus influenzae* (β-lactamase positive) (n = 20) | | | |
|---|---|---|---|
| Gemifloxacin | ≤0.0075-0.06 | ≤0.0075 | 0.015 |
| Ciprofloxacin | ≤0.0075-0.015 | ≤0.0075 | 0.015 |
| Trovafloxacin | ≤0.0075-0.06 | ≤0.0075 | 0.06 |
| Sparfloxacin | ≤0.0075-0.12 | 0.015 | 0.03 |
| Clinafloxacin | ≤0.0075-0.25 | ≤0.0075 | ≤0.0075 |
| Moxifloxacin | ≤0.0075-0.06 | 0.015 | 0.015 |
| Levofloxacin | ≤0.0075-0.03 | 0.015 | 0.03 |
| Cefuroxime | 0.12-4 | 0.5 | 1 |

**Table 11.**

| MICs of Gemifloxacin and Comparative Drugs against 50 Strains of *M. catarrhalis* | | | |
|---|---|---|---|
| Microrganism and antimicrobial | MIC (µg/ml) | | |
| | Range | MIC₅₀ | MIC₉₀ |
| *Moraxella catarrhalis* (n = 50) | | | |
| Gemifloxacin | ≤0.00375-0.03 | 0.015 | 0.015 |
| Ciprofloxacin | ≤0.00375-0.06 | 0.03 | 0.03 |
| Trovafloxacin | ≤0.00375-0.06 | 0.015 | 0.06 |
| Sparfloxacin | ≤0.00375-0.06 | 0.015 | 0.03 |
| Clinafloxacin | ≤0.00375-0.015 | ≤0.00375 | 0.015 |
| Moxifloxacin | ≤0.00375-0.12 | 0.06 | 0.06 |
| Levofloxacin | ≤0.00375-0.03 | 0.015 | 0.03 |
| Cefuroxime | ≤0.00375-1 | 0.25 | 1 |

| *Moraxella catarrhalis* (β-lactamase positive) (n = 37) | | | |
|---|---|---|---|
| Gemifloxacin | ≤0.00375-0.03 | 0.015 | 0.015 |
| Ciprofloxacin | ≤0.00375-0.06 | 0.03 | 0.03 |
| Trovafloxacin | ≤0.00375-0.06 | 0.015 | 0.03 |
| Sparfloxacin | ≤0.00375-0.06 | 0.015 | 0.03 |
| Clinafloxacin | ≤0.00375-0.015 | ≤0.00375 | 0.015 |
| Moxifloxacin | ≤0.00375-0.12 | 0.03 | 0.06 |
| Levofloxacin | ≤0.00375-0.03 | 0.015 | 0.03 |
| Cefuroxime | ≤0.00375-1 | 0.25 | 1 |

| *Moraxella catarrhalis* (β-lactamase negative) (n = 13) | | | |
|---|---|---|---|
| Gemifloxacin | ≤0.00375-0.015 | 0.015 | 0.015 |
| Ciprofloxacin | ≤0.00375-0.03 | 0.015 | 0.03 |
| Trovafloxacin | ≤0.00375-0.06 | 0.0075 | 0.036 |
| Sparfloxacin | ≤0.00375-0.06 | 0.0075 | 0.06 |
| Clinafloxacin | ≤0.00375-0.015 | ≤0.00375 | 0.015 |
| Moxifloxacin | ≤0.00375-0.12 | 0.015 | 0.06 |
| Levofloxacin | ≤0.00375-0.03 | 0.03 | 0.03 |
| Cefuroxime | ≤0.00375-1 | 0.12 | 0.5 |

**Table 12.**

| MICs of Gemifloxacin and Comparative Drugs Against 50 Strains of *S. aureus* | | | |
|---|---|---|---|
| Microrganism and antimicrobial | MIC (µg/ml) | | |
| | Range | MIC₅₀ | MIC₉₀ |
| *Staphylococcus aureus* (n = 50) | | | |
| Gemifloxacin | ≤0.00375-0.12 | 0.06 | 0.12 |
| Ciprofloxacin | 0.06-4 | 2 | 4 |
| Trovafloxacin | ≤0.00375-0.5 | 0.12 | 0.25 |
| Sparfloxacin | 0.06-1 | 0.5 | 0.5 |
| Clinafloxacin | 0.06-0.25 | 0.12 | 0.25 |
| Moxifloxacin | 0.015-0.5 | 0.25 | 0.5 |
| Levofloxacin | 0.03-2 | 1 | 2 |
| Cefuroxime | ≤0.00375-0.25 | 0.25 | 0.25 |

| *Staphylococcus aureus* (β-lactamase negative) (n = 20) | | | |
|---|---|---|---|
| Gemifloxacin | 0.06-0.12 | 0.12 | 0.12 |
| Ciprofloxacin | 0.06-4 | 2 | 4 |
| Trovafloxacin | ≤0.00375-0.25 | 0.12 | 0.25 |
| Sparfloxacin | 0.06-1 | 0.5 | 0.5 |
| Clinafloxacin | 0.06-0.25 | 0.12 | 0.25 |
| Moxifloxacin | 0.06-0.5 | 0.25 | 0.5 |
| Levofloxacin | 0.03-2 | 2 | 2 |
| Cefuroxime | 0.0075-0.25 | 0.25 | 0.25 |

| *Staphylococcus aureus* (β-lactamase positive) (n = 20) | | | |
|---|---|---|---|
| Gemifloxacin | ≤0.00375-0.12 | 0.12 | 0.123 |
| Ciprofloxacin | 0.5-8 | 4 | 4 |
| Trovafloxacin | 0.06-0.5 | 0.12 | 0.25 |
| Sparfloxacin | 0.12-1 | 0.5 | 0.5 |
| Clinafloxacin | 0.06-0.25 | 0.12 | 0.25 |
| Moxifloxacin | 0.12-0.5 | 0.25 | 0.5 |
| Levofloxacin | 1-2 | 1 | 2 |
| Cefuroxime | 0.06-0.25 | 0.25 | 0.25 |

| *Staphylococcus aureus* (β-lactamase positive and erythromycin resistant) (n = 10) | | | |
|---|---|---|---|
| Gemifloxacin | 0.06-0.12 | 0.06 | 0.06 |
| Ciprofloxacin | 0.5-2 | 1 | 2 |
| Trovafloxacin | ≤0.00375-0.25 | 0.12 | 0.25 |
| Sparfloxacin | 0.25-1 | 0.25 | 0.5 |
| Clinafloxacin | 0.06-0.25 | 0.12 | 0.25 |
| Moxifloxacin | 0.12-0.5 | 0.25 | 0.5 |
| Levofloxacin | 0.5-2 | 1 | 2 |
| Cefuroxime | 0.06-0.25 | 0.12 | 0.25 |

**Table 13.**

| MICs of Gemifloxacin and Comparative Drugs Against 50 Strains of *K. pneumoniae* | | | |
|---|---|---|---|
| Microrganism and antimicrobial | MIC (µg/ml) | | |
| | Range | MIC₅₀ | MIC₉₀ |
| *Klebsiella pneumoniae* (n = 50) | | | |
| Gemifloxacin | ≤0.0075-1 | 0.03 | 0.25 |
| Trovafloxacin | ≤0.0075-0.5 | 0.06 | 0.5 |
| Sparfloxacin | ≤0.0075-1 | 0.06 | 0.5 |
| Clinafloxacin | ≤0.0075-1 | 0.015 | 1 |
| Moxifloxacin | 0.015-1 | 0.06 | 0.5 |
| Ciprofloxacin | ≤0.0075-1 | 0.12 | 0.5 |
| Levofloxacin | 0.15-1 | 0.06 | 0.5 |
| Cefuroxime | 0.25->128 | 2 | 128 |

| *Klebsiella pneumoniae* (extended spectrum β-lactamase positive) (n = 20) | | | |
|---|---|---|---|
| Gemifloxacin | 0.03-1 | 0.25 | 0.5 |
| Trovafloxacin | 0.06-0.5 | 0.5 | 0.5 |
| Sparfloxacin | 0.03-0.5 | 0.5 | 0.5 |
| Clinafloxacin | ≤0.0075-1 | ≤0.0075 | 1 |
| Moxifloxacin | 0.06-1 | 0.5 | 1 |
| Ciprofloxacin | 0.015-0.5 | 0.25 | 0.5 |
| Levofloxacin | 0.06-1 | 0.5 | 0.5 |
| Cefuroxime | 1->128 | 128 | >128 |

| *Klebsiella pneumoniae* (extended spectrum β-lactamase negative) (n = 30) | | | |
|---|---|---|---|
| Gemifloxacin | ≤0.0075-0.12 | 0.015 | 0.06 |
| Trovafloxacin | ≤0.0075-0.015 | 0.06 | 0.12 |
| Sparfloxacin | ≤0.0075-0.25 | 0.03 | 0.12 |
| | | | |
| Clinafloxacin | ≤0.0075-0.12 | ≤0.0075 | 0.015 |
| Moxifloxacin | 0.015-0.5 | 0.06 | 0.25 |
| Ciprofloxacin | ≤0.0075-0.12 | 0.015 | 0.03 |
| Levofloxacin | 0.015-0.5 | 0.06 | 0.12 |

Against β-lactamase-positive strains there were no significant differences in the MIC ranges of all fluoroquinolones tested in comparison with β-lactamase-negative strains.

Towards *M. catarrhalis,* gemifloxacin showed activity (MIC₉₀ 0.015 µg/ml) superior to that of ciprofloxacin (0.03 µg/ml), sparfloxacin (0.03 µg/ml), levofloxacin (0.03 µg/ml), trovafloxacin (0.06 µg/ml), moxifloxacin (0.06 µg/ml) and cefuroxime (1 µg/ml) and comparable to that of clinafloxacin (0.015 µg/ml). No great differences in activity were noted after grouping *M. catarrhalis* according to β-lactamase production. The MICs of gemifloxacin and other antimicrobials against oxacillin-susceptible strains of *S. aureus* and *K. pneumoniae* are given in Tables 12 and 13. Gemifloxacin had the lowest MIC₉₀s against these microrganisms.

The activity of gemifloxacin against *S. pneumoniae* strains is 4-64 times greater than that of all comparator agents in this study. Gemifloxacin potency towards strains of *S. pyogenes* is 2-32-fold higher than that of the other fluoroquinolones tested. Gemifloxacin and clinafloxacin shows superior activity against *H. influenzae* and *M. catarrhalis* strains compared with comparator agents. Gemifloxacin has the lowest MIC₉₀s of the compounds tested against S. *aureus* and *K. pneumoniae.* These results indicate a role for gemifloxacin in the treatment of community-acquired respiratory tract infections.

The invention provides a method for modulating metabolism of respiratory tract pathogenic bacteria. Skilled artisans can readily choose respiratory tract pathogenic bacteria or patients infected with or suspected to be infected with these organisms to practice the methods of the invention. Alternatively, the bacteria useful in the methods of the invention can be those described herein.

Also provided by the invention is a method of treating or preventing a bacterial infection by respiratory tract pathogenic bacteria comprising the step of administering an antibacterially effective amount of a composition comprising a quinolone, particularly a gemifloxacin compound to' a mammal, preferably a human, suspected of having or being at risk of having an infection with respiratory tract pathogenic bacteria.

While a preferred object of the invention provides a method wherein said respiratory tract pathogenic bacteria is selected from the group consisting of: *Streptococcus pneumoniae, Streptococcus pyogenes, Haemophilus influenzae, Moraxella catarrhalis, Staphylococcus aureus* and *Klebsiella pneumoniae.* Other respiratory tract pathogenic bacteria may also be included in the methods. The skilled artisan may identify these organisms as provided herein as well as using other methods known in the art, *e.g.* MIC tests.

The present invention provides, among other things, methods for using a composition comprising a quinolone, particularly a gemifloxacin compound against respiratory tract pathenogenic bacteria.

This invention was based, in part, on analyses evaluating the comparative activity of gemifloxacin against various respiratory tract pathogens. An objective of these analyses was to determine the *in vitro* activity of gemifloxacin against recent clinical isolates of *Streptococcus pneumoniae* and *Haemophilus influenzae* and to compare its activity to that of other fluoroquinolones.

The *in vitro* activity of the new fluoroquinolone gemifloxacin (GEM) was compared with that of trovafloxacin (TRO), grepafloxacin (GRE), levofloxacin (LEV), ofloxacin (OFX) and ciprofloxacin (CIP) against 235 clinical isolates of *Streptococcus pneumoniae* (65 penicillin-susceptible (PSP), 111 penicillin-intermediate (PIP) and 59 penicillin-resistant (PRP)) and 145 isolates of *Haemophilus influenzae* (115 beta-lactamase negative (HiB1a-) and 30 beta-lactamase positive (HiB1a+)). Broth microdilution tests were performed in cation-adjusted Mueller-Hinton broth supplemented with blood for pneumococci and in HTM broth for *H. influenzae*, following NCCLS guidelines. All microorganisms were inhibited by gemifloxacin at 0.06 µg/ml. MIC₉₀s of GEM, TRO, GRE and LEV against all (380) isolates tested were 0.03, 0.12, 0.12 and 1 µg/ml respectively. Gemifloxacin is a potent agent against respiratory tract pathogens including strains with resistance to current therapies.

Increasing resistance to antimicrobial agents among respiratory tract pathogens is a cause of concern and has increased the need for new antimicrobials with activity against these pathogens. Gemifloxacin (SB-265805) is a potent new quinolone, which has an oxime-derivatized (aminomethyl) pyrrolidine substituent at C7 conferring excellent activity against both Gram positive and Gram negative pathogens. The favorable pharmacokinetics of gemifloxacin make it a promising agent for the therapy of infections for which the currently marketed quinolone antimicrobials have a limited role, for example, in the treatment of respiratory tract infections.

A total of 302 recent clinical isolates (1998-1999) of *S. pneumoniae* were studied. Among those, 98 were penicillin-susceptible (PSP), 124 were penicillin-intermediate (PIP) and 80 were penicillin-resistant (PRP). 28% of the isolates were also erythromycin-resistant. Eight hundred recent clinical isolates (1998-1999) of *H. influenzae* were also studied. Of these, 234 were β-lactamase negative and 66 were β-lactamase positive.

Susceptibility testing was performed by the broth microdilution method (NCCLS) using commercially dried microdilution panels (SB-265805 surveillance MIC1 and MIC2, Baxter, MicroScan RUO/IUO, Sacramento, CA) manufactured for this study. The panels included gemifloxacin at two-fold concentrations from 0.001-256 µg/ml; trovafloxacin, grepafloxacin, levofloxacin and ciprofloxacin at two-fold concentrations from 0.015-16 µg/ml; and ofloxacin at two-fold concentrations from 0.06-64 µg/ml. Panels were inoculated with the isolates suspended in the appropriate broth (cation-adjusted Mueller-Hinton broth with 3% lysed horse blood for S. *pneumoniae,* and *Haemophilus* Test Medium for *H. influenzae*) to achieve a final well concentration of 4-7 x 10⁵ CFU/ml. MIC readings were performed after incubation at 35°C for 20-24 h in a non-CO₂ incubator.

*Escherichia coli* ATCC 25922, *Staphylococcus aureus* ATCC 29213, *Enterococcus faecalis* ATCC 29212, *Haemophilus influenzae* ATCC 49247, and *Streptococcus pneumoniae* ATCC 49619 were used as control strains in each run.

The comparative in *vitro* antimicrobial activities of gemifloxacin and other quinolones are shown in Tables 14-17. Gemifloxacin demonstrated the most potent antimicrobial activity among the quinolones tested against *S. pneumoniae* and *H. influenzae.* All 602 isolates were inhibited by gemifloxacin at 0.12 µg/ml. MIC₉₀s of gemifloxacin, trovafloxacin, grepafloxacin and levofloxacin against all isolates tested were 0.06, 0.25, 0.12, and 1 µg/ml, respectively.

Gemifloxacin has the most potent activity of all the quinolones tested against *S. pneumoniae* and *H. influenzae*. Gemifloxacin is equally active against penicillin-susceptible and penicillin-resistant *S. pneumoniae,* erythromycin-resistant *S. pneumoniae* and β-lactamase positive and β-lactamase negative *H. influenzae*. Against *S. pneumoniae,* gemifloxacin is two-fold more active than grepafloxacin, four-fold more active than trovafloxacin and 16-fold more active than levofloxacin. Against *H. influenzae*, the activity of gemifloxacin is similar to that of grepafloxacin and trovafloxacin, and is four-fold superior to that of levofloxacin. Gemifloxacin is a potent new quinolone with excellent activity against respiratory tract pathogens, including strains with resistance to current antimicrobial agents.

**Table 14.**

| *Comparative in vitro* activity of gemifloxacin and other fluoroquinolones against *Streptococcus pneumoniae* (302 isolates) | | | | | | |
|---|---|---|---|---|---|---|
| Isolate (No.) | MIC₉₀ (µg/ml) | | | | | |
| | Gemi | Trova | Grepa | Levo | Oflox | Cipro |
| Penicillin-sensitive (98) | 0.06 | 0.25 | 0.12 | 1 | 2 | 2 |
| Penicillin-intermediate (124) | 0.03 | 0.25 | 0.12 | 1 | 4 | 2 |
| Penicillin-resistant (80) | 0.06 | 0.25 | 0.12 | 1 | 2 | 2 |
| Erythromycin-sensitive (216) | 0.06 | 0.25 | 0.25 | 2 | 4 | 2 |
| Erythromycin-resistant (86) | 0.03 | 0.25 | 0.12 | 1 | 2 | 2 |

**Table 15.**

| Comparative *in vitro* activity of gemifloxacin with other fluoroquinolones against *Streptococcus pneumoniae* (302 isolates). All isolates were inhibited by gemifloxacin 0.12 µg/ml | | | |
|---|---|---|---|
| Antimicrobial agent | MIC (µg/ml) | | |
| | Range | MIC₅₀ | MIC₉₀ |
| Gemifloxacin | ≤0.0015-0.12 | 0.03 | 0.06 |
| Trovafloxacin | ≤0.015-1 | 0.12 | 0.25 |
| Grepafloxacin | ≤0.015-0.25 | 0.03 | 0.12 |
| Levofloxacin | ≤0.015-2 | 1 | 1 |
| Ofloxacin | ≤0.06-4 | 2 | 4 |
| Ciprofloxacin | ≤0.015-4 | 1 | 2 |

**Table 16.**

| Comparative *in vitro* activity of gemifloxacin and other fluoroquinolones against *Haemophilus influenzae* (300 isolates) | | | | | | |
|---|---|---|---|---|---|---|
| Isolate (No.) | MIC₉₀ (µg/ml) | | | | | |
| | Gemi | Trova | Grepa | Levo | Oflox | Cipro |
| β-lactamase positive (66) | 0.03 | 0.03 | 0.03 | 0.03 | 0.12 | 0.03 |
| β-lactamase negative (234) | 0.008 | ≤0.015 | ≤0.015 | 0.03 | ≤0.06 | ≤0.015 |

**Table 17.**

| Comparative *in vitro* activity of gemifloxacin with other fluoroquinolones against *Haemophilus influenzae* (300 isolates). All isolates were inhibited by gemifloxacin 0.12 µg/ml | | | |
|---|---|---|---|
| Antimicrobial agent | MIC (µg/ml) | | |
| | Range | MIC₅₀ | MIC₉₀ |
| Gemifloxacin | ≤0.0015-0.12 | 0.004 | 0.008 |
| Trovafloxacin | ≤0.015-0.25 | ≤0.015 | ≤0.015 |
| Grepailoxacin | ≤0.015-0.12 | ≤0.015 | ≤0.015 |
| Levofloxacin | ≤0.015-0.5 | ≤0.015 | 0.03 |
| Ofloxacin | ≤0.06-0.5 | ≤0.06 | ≤0.06 |
| Ciprofloxacin | ≤0.015-0.5 | ≤0.015 | ≤0.015 |

The invention provides a method for modulating metabolism of respiratory tract pathogenic bacteria. Skilled artisans can readily choose respiratory tract pathogenic bacteria or patients infected with or suspected to be infected with these organisms to practice the methods of the invention. Alternatively, the bacteria useful in the methods of the invention may be those described herein.

Also provided by the invention is a method of treating or preventing a bacterial infection by respiratory tract pathogenic bacteria comprising the step of administering an antibacterially effective amount of a composition comprising a quinolone, particularly a gemifloxacin compound to a mammal, preferably a human, suspected of having or being at risk of having an infection with respiratory tract pathogenic bacteria.

While a preferred object of the invention provides a method wherein said respiratory tract pathogenic bacteria is selected from the group consisting of: *Streptococcus pneumoniae* and *Haemophilus influenzae*. Other respiratory tract pathogenic bacteria may also be included in the methods. The skilled artisan may identify these organisms as provided herein as well as using other methods known in the art, *e.g.* MIC tests.

The present invention provides, among other things, methods for using a composition comprising a quinolone, particularly a gemifloxacin compound against respiratory tract pathenogenic bacteria.

This invention was based, in part, on analyses evaluating the comparative activity of gemifloxacin against various respiratory tract pathogens. An objective of these analyses was to determine the *in vitro* activity of gemifloxacin against representative isolates of common respiratory tract pathogens, including quinolone-resistant strains. The *in vitro* activity of gemifloxacin was compared to those of sparfloxacin, ciprofloxacin, levofloxacin, tosufloxacin, trovafloxacin, cefditoren, cefaclor and amoxycillin. MIC values were determined by an agar dilution method.

Certain quinolones, such as sparfloxacin and tosufloxacin, which have improved activity against Gram positive bacteria, are available for clinical use in Japan. However, these agents are not potent enough against Gram positive bacteria to treat respiratory tract infections caused by staphylococci and streptococci, although they are effective against the respiratory pathogens *Haemophilus influenzae* and *Moraxella catarrhalis.*

The potential of gemifloxacin in the treatment of respiratory tract infections depends upon demonstrating its activity against *S. pneumoniae* as well as *H. influenzae* and *M. catarrhalis.* This is because these bacterial species are the most common pathogens associated with respiratory tract infections, including community-acquired pneumonia and acute exacerbation of chronic bronchitis. Moreover, activity against the increasingly common antimicrobial-resistant strains of *S. pnuemoniae* would further enhance the potential of gemifloxacin as an agent for empiric use in respiratory tract infections.

This study investigates the *in vitro* activity of gemifloxacin compared with those of other quinolones against pathogens involved in respiratory tract infections, including ciprofloxacin-resistant *S. pneumoniae.*

Clinical isolates and quinolone-resistant strains were collected in Kitasato University School of Medicine, Kanagawa, Japan. MICs were determined by the two-fold serial agar dilution method with Sensitivity Disk agar-N, which was supplemented with 5% defibrinated horse blood for streptococci, and with 5% Fildes enrichment (Difco) for *Haemophilus.* One loopful of an inoculum corresponding to approximately 10⁴ CFU was applied with an inoculating apparatus (Microplanter; Sakuma Seisakusho) to drug-containing agar plates, and the plates were incubated for 18 h at 37°C.

Comparative *in vitro* activities of gemifloxacin against respiratory tract pathogens are shown in Tables 18-20, and against quinolone-resistant strains in Table 21.

Gemifloxacin demonstrates high activity against the common respiratory tract pathogens, indicating potential for the treatment of respiratory tract infections. Gemifloxacin is the most active compound tested against ciprofloxacin-resistant *S. pneumoniae* as well as ciprofloxacin-susceptible *S. pneumoniae.* Bacterial strains highly resistant to gemifloxacin would not easily be developed due to a single mutation.

Against Gram negative clinical isolates of *H. influenzae* and *M. catarrhalis*, the activity of gemifloxacin was similar to other quinolones, including ciprofloxacin. Gemifloxacin shows activity comparable with trovafloxacin, but superior to the other quinolones tested against MSSA and MRSA. The activity of gemifloxacin against *S. pneumoniae* (MIC₉₀ 0.031 µg/ml) is 4-fold and 8-fold more potent than trovafloxacin and sparfloxacin, respectively, and is considerably more potent than the other quinolones tested. Gemifloxacin (MIC₉₀ 0.063 µg/ml) is the most potent agent against penicillin-resistant strains, erythromycin-resistant strains and minocycline-resistant strains of *S. pneumoniae*.

Against quinolone-resistant clinical isolates of *S. pneumoniae* (ciprofloxacin MIC ≥8 µg/ml), gemifloxacin exhibits the most potent activity of all the agents tested (MIC₉₀ 0.5 µg/ml). Gemifloxacin also shows the most potent activity against *E. coli* mutated in the DNA gyrase, with a MIC₅₀ of 0.032 µg/ml and MIC₉₀ of 0.25 µg/ml. The MIC of gemifloxacin (0.031 µg/ml) increased only 2-4-fold against *S. aureus* KU2240 with a single mutation in NorA, DNA gyrase or topoisomerase IV. Against the same strains with double mutation in both DNA gyrase and topoisomerase IV, MICs increased to 2 µg/ml and 32 µg/ml, respectively.

Gemifloxacin exhibits potent anti-bacterial activity against common respiratory tract pathogens, including quinolone-resistant strains, indicating potential for the treatment of respiratory tract infections. Moreover, these analyses indicate that bacterial strains highly resistant to gemifloxacin can not easily be developed by single mutation.

**Table 18.**

| Comparative *In Vitro* Activities of Gemifloxacin Against Clinical Isolates of *Haemophilus influenzae* and *Moraxella catarrhalis* | | | | |
|---|---|---|---|---|
| Microrganism (No. of strains tested) | Antimicrobial agent | MIC (µg/ml) | | |
| | | Range | MIC₅₀ | MIC₉₀ |
| *Haemophilus influenzae* (24) | Gemifloxacin | 0.008-0.031 | 0.016 | 0.016 |
| | Sparfloxacin | ≤0.004-0.063 | 0.016 | 0.031 |
| | Ciprofloxacin | 0.008-0.031 | 0.016 | 0.016 |
| | Levofloxacin | 0.016-0.031 | 0.031 | 0.031 |
| | Trovafloxacin | ≤0.004-0.031 | 0.016 | 0.031 |
| | Cefditoren | 0.016-0.5 | 0.031 | 0.031 |
| | Amoxycillin | 0.5->128 | 1 | 64 |
| *Moraxella catarrhalis* (22) | Gemifloxacin | 0.016-0.031 | 0.031 | 0.031 |
| | Sparfloxacin | 0.016-0.031 | 0.016 | 0.031 |
| | Ciprofloxacin | 0.031-0.063 | 0.031 | 0.031 |
| | Levofloxacin | 0.031-0.063 | 0.063 | 0.063 |
| | Trovafloxacin | 0.016-0.031 | 0.016 | 0.031 |
| | Cefditoren | 0.063-1 | 0.5 | 1 |
| | Amoxycillin | 1-16 | 8 | 16 |

**Table 19.**

| Comparative *In Vitro* Activities of Gemifloxacin Against Clinical Isolates of *Staphylococcus aureus*. | | | | |
|---|---|---|---|---|
| *S. aureus* strain (No. of strains tested) | Antimicrobial agent | MIC (µg/ml) | | |
| | | Range | MIC₅₀ | MIC₉₀ |
| Methicillin-susceptible (9) | Gemifloxacin | 0.031-0.25 | 0.063 | 0.25 |
| | Sparfloxacin | 0.063-0.5 | 0.125 | 0.5 |
| | Ciprofloxacin | 0.25-2 | 0.5 | 2 |
| | Levofloxacin | 0.125-1 | 0.5 | 1 |
| | Trovafloxacin | 0.031-0.5 | 0.031 | 0.5 |
| | Cefditoren | 0.5-1 | 1 | 1 |
| | Amoxycillin | 0.25-128 | 8 | 128 |
| Methicillin-resistant (38) | Gemifloxacin | 0.031-32 | 1 | 32 |
| | Sparfloxacin | 0.063-64 | 2 | 64 |
| | Ciprofloxacin | 0.5->128 | 16 | 64 |
| | Levofloxacin | 0.25-128 | 8 | 64 |
| | Trovafloxacin | 0.031-16 | 1 | 16 |
| | Cefditoren | 32-128 | 128 | 128 |
| | Amoxycillin | 8-64 | 32 | 64 |

**Table 20.**

| Comparative *In Vitro* Activities of Gemifloxacin Against Clinical Isolates of *Streptococcus pneumoniae.* | | | | |
|---|---|---|---|---|
| *S. pneumoniae* strain (No. of strains tested) | Antimicrobial agent | MIC (µg/ml) | | |
| | | Range | MIC₅₀ | MIC₉₀ |
| Ciprofloxacin-susceptible | Gemifloxacin | ≤0.008-0.063 | 0.031 | 0.031 |
| (94) | Sparfloxacin | 0.063-0.5 | 0.125 | 0.25 |
| | Tosufloxacin | 0.063-0.5 | 0.125 | 0.25 |
| | Ciprofloxacin | 0.25-4 | 1 | 2 |
| | Levofloxacin | 0.25-2 | 0.5 | 1 |
| | Trovafloxacin | 0.031-0.25 | 0.063 | 0.125 |
| | Cefditoren | ≤0.008-2 | 0.063 | 0.25 |
| | Amoxycillin | ≤0.008-8 | 0.063 | 0.5 |
| Ciprofloxacin-resistant | Gemifloxacin | 0.063-0.5 | 0.25 | 0.5 |
| (21) | Sparfloxacin | 0.25-8 | 4 | 8 |
| | Tosufloxacin | 0.25-16 | 4 | 8 |
| | Ciprofloxacin | 8-128 | 32 | 64 |
| | Levofloxacin | 2-32 | 16 | 16 |
| | Trovafloxacin | 0.125-4 | 2 | 4 |
| | Cefditoren | ≤0.008-1 | ≤0.008 | 0.25 |
| | Amoxycillin | 0.016-1 | 0.016 | 0.5 |
| Penicillin-resistant (19) | Gemifloxacin | 0.016-0.063 | 0.016 | 0.063 |
| | Sparfloxacin | 0.063-1 | 0.125 | 0.5 |
| | Tosufloxacin | 0.063-0.5 | 0.125 | 0.25 |
| | Ciprofloxacin | 0.5-8 | 0.5 | 4 |
| | Levofloxacin | 0.5-8 | 0.5 | 1 |
| | Trovafloxacin | 0.031-0.25 | 0.063 | 0.25 |
| | Cefditoren | 0.25-2 | 0.25 | 2 |
| | Amoxycillin | 0.25-8 | 0.5 | 8 |
| | | | | |
| Erythromycin-resistant | Gemifloxacin | 0.016-0.5 | 0.031 | 0.063 |
| (37) | Sparfloxacin | 0.125-8 | 0.25 | 0.5 |
| | Tosufloxacin | 0.063-4 | 0.125 | 0.5 |
| | Ciprofloxacin | 0.5-64 | 1 | 2 |
| | Levofloxacin | 0.5-16 | 1 | 2 |
| | Trovafloxacin | 0.063-2 | 0.063 | 0.25 |
| | Cefditoren | ≤0.008-2 | 0.063 | 0.25 |
| | Amoxycillin | 0.016-8 | 0.063 | 0.5 |
| Minocycline-resistant | Gemifloxacin | 0.016-0.5 | 0.031 | 0.063 |
| (37) | Sparfloxacin | 0.125-8 | 0.25 | 0.5 |
| | Tosufloxacin | 0.063-4 | 0.125 | 0.5 |
| | Ciprofloxacin | 0.25-64 | 1 | 2 |
| | Levofloxacin | 0.25-16 | 1 | 2 |
| | Trovafloxacin | 0.063-2 | 0.063 | 0.25 |
| | Cefditoren | ≤0.008-2 | 0.063 | 0.25 |
| | Amoxycillin | ≤0.008-8 | 0.063 | 0.5 |

**Table 21.**

| Comparative *In Vitro* Activities of Gemifloxacin Against Quinolone-resistant Strains of *S. aureus, E. coli* and *P. aeruginosa* | | | | | | | |
|---|---|---|---|---|---|---|---|
| Species | Mutant type* | MIC (µg/ml) | | | | | |
| | | Gemifloxacin | Trovafloxacin | Sparfloxacin | Tosufloxacin | Ciprofloxacin | Levofloxacin |
| *S. aureus* KU2240 | Wild | 0.031 | 0.063 | 0.25 | 0.063 | 0.5 | 0.25 |
| *S. aureus* KU2241 | NorA | 0.063 | 0.063 | 0.25 | 0.125 | 1 | 0.5 |
| *S. aureus* KU2242 | T | 0.063 | 0.25 | 0.25 | 0.25 | 2 | 0.5 |
| *S. aureus* KU2243 | G+T | 32 | 32 | 64 | >128 | 32 | >128 |
| *S. aureus* KU2244 | G | 0.125 | 0.063 | 0.25 | 0.125 | 0.5 | 0.5 |
| *S. aureus* KU2245 | G+T | 2 | 1 | 64 | 4 | 16 | 32 |
| *S. aureus* KU2246 | T | 0.125 | 0.25 | 0.25 | 0.5 | 2 | 1 |
| *E. coli* KU1034 | GA | 0.25 | 0.5 | 0.5 | 0.25 | 0.25 | 1 |
| *E. coli* KU1035 | GA | 0.25 | 0.5 | 0.5 | 0.25 | 0.25 | 1 |
| *E. coli* KU1036 | GB | 0.031 | 0.063 | 0.063 | 0.031 | 0.063 | 0.125 |
| *E. coli* KU1029 | SP | 0.031 | 0.063 | 0.031 | 0.063 | 0.031 | 0.063 |
| *E. coli* KU1030 | SP | 0.031 | 0.063 | 0.063 | 0.031 | 0.063 | 0.125 |
| *E. coli* KU1031 | SP | 0.031 | 0.063 | 0.031 | 0.031 | 0.031 | 0.063 |
| *E. coli* KU1032 | SP | 0.125 | 0.25 | 0.125 | 0.125 | 0.125 | 0.25 |
| *E. coli* KU1033 | SP | 0.016 | 0.031 | 0.031 | 0.031 | 0.031 | 0.063 |
| *E. coli* KU1038 | SP | 0.031 | 0.063 | 0.031 | 0.063 | 0.063 | 0.125 |
| *E. coli* KU1039 | SP | 0.125 | 0.25 | 0.25 | 0.063 | 0.125 | 0.25 |
| *E. coli* KU1040 | SP | ≤0.008 | ≤0.008 | ≤0.008 | ≤0.008 | ≤0.008 | 0.031 |
| *E. coli* KU1041 | SP | 0.25 | 0.5 | 0.25 | 0.25 | 0.125 | 0.5 |
| *E. coli* KU1042 | SP | 0.063 | 0.125 | 0.063 | 0.063 | 0.063 | 0.063 |
| *E. coli* (13 strains) | MIC₅₀ | 0.031 | 0.063 | 0.125 | 0.063 | 0.063 | 0.125 |
| | MIC₉₀ | 0.25 | 0.5 | 0.5 | 0.25 | 0.25 | 1 |
| *P. aeruginosa* | GA | 2 | 1 | 2 | 1 | 1 | 2 |
| KU 1043 | | | | | | | |

| | | | | | | | |
|---|---|---|---|---|---|---|---|
| *Wild = wild strain; NorA = efflux-mediated quinolonc resistant strain; SP = spontaneous mutant in the DNA gyrase; G = mutant in the DNA gyrase; GA = mutant in the DNA gyrase A; GB = mutant in the DNA gyrase B; T = mutant in the DNA topoisomerase IV; G+T = mutant in the DNA gyrase and topoisomerase IV. | | | | | | | |

The invention provides a method for modulating metabolism of respiratory tract pathogenic bacteria. Skilled artisans can readily choose respiratory tract pathogenic bacteria or patients infected with or suspected to be infected with these organisms to practice the methods of the invention. Alternatively, the bacteria useful in the methods of the invention may be those described herein.

Also provided by the invention is a method of treating or preventing a bacterial infection by respiratory tract pathogenic bacteria comprising the step of administering an antibacterially effective amount of a composition comprising a quinolone, particularly a gemifloxacin compound to a mammal, preferably a human, suspected of having or being at risk of having an infection with respiratory tract pathogenic bacteria.

While a preferred object of the invention provides a method wherein said respiratory tract pathogenic bacteria is selected from the group consisting of: *Haemophilus influenzae, Staphylococcus aureus, Streptococcus pneumoniae, E. coli, P. aeruginosa* and *Moraxella catarrhalis*. Other respiratory tract pathogenic bacteria may also be included in the methods. The skilled artisan may identify these organisms as provided herein as well as using other methods known in the art, *e*.*g*. MIC tests.

The present invention provides, among other things, methods for using a composition comprising a quinolone, particularly a gemifloxacin compound against respiratory and genital tract pathenogenic bacteria.

This invention was based, in part, on analyses evaluating the comparative activity of gemifloxacin against various respiratory and genital tract pathogens. An objective of these analyses was to determine the MIC of gemifloxacin, which was determined by a broth microdilution method and compared with trovafloxacin (TRO), sparfloxacin (SPA), ciprofloxacin (CIP), levofloxacin (LEV) and tosufloxacin (TOS) against common respiratory tract pathogens and urinary tract pathogens from clinical strains isolated in Japan between 1997 and 1998. Gemifloxacin is the most potent of the quinolones tested against the common respiratory tract pathogens of *S. pneumoniae, S. pyogenes, H. influenzae* and *M. catarrhalis.* Cross-resistance to macrolides and β-lactams was not detected for any quinolone against *S. pneumoniae*. Gemifloxacin had the most potent *in vitro* activity against urinary tract pathogens, such as *E. coli* and *Enterococcus* spp. including vancomycin-resistant enterococci. Results also indicated comparable activity to the other quinolones against CIP-R *E. coli*. These results suggest that gemifloxacin is an extremely potent agent against respiratory tract and urinary tract pathogens.

Gemifloxacin has been evaluated in the USA, Europe and Korea and shows potent *in vitro* activity against a wide range of Gram negative and Gram positive pathogens, particularly *Streptococcus pneumoniae* and other respiratory tract pathogens.

A purpose of this study was to determine and compare the MICs of gemifloxacin, sparfloxacin, ciprofloxacin, trovafloxacin, levofloxacin and tosufloxacin against a wide variety of Japanese clinical isolates including respiratory and urinary tract pathogens.

A total of 1100 clinical isolates were tested. Most strains were isolated from Omori Hospital at Toho University School of Medicine,Tokyo, Japan, between 1997 and 1998.

MICs were determined using the NCCLS (NCCLS, *4th ed.,* M7-A4, Wayne, PA (1997)) recommended procedure for broth microdilution, except for *Neisseria gonorrhoeae. Streptococcus* spp., *Haemophilus influenzae* and *Moraxella catarrhalis* were tested with cation-adjusted Mueller-Hinton broth supplemented with 5% lysed horse blood, NAD and yeast extract. *N. gonorrhoeae* isolates were tested by an agar dilution method using the GC II agar base and 1% defined growth supplement.

The *in vitro* activity of gemifloxacin and the other quinolones are summarized in Tables 22-25. The number of isolates of each bacterial species is also shown. Isolates were obtained from sputum or tracheal lavage or from swabs of the pharynx or nasal cavity in patients with RTI; from the urine, urinary catheter or urinary discharge in patients with UTI. Comparative activities against some drug-resistant strains of Gram positive cocci are listed separately in Table 26.

Gemifloxacin showed the most potent activity of all the antimicrobial agents tested against the common respiratory tract pathogens of *Streptococcus pyogenes, H. influenzae, M. catarrhalis* and *S. pneumoniae,* including amoxicillin-resistant and macrolide-resistant strains.

Against the urinary tract pathogens, ciprofloxacin-resistant and -susceptible *Enterococcus faecalis,* gemifloxacin exhibits the most potent activity of the antimicrobial agents tested. Gemifloxacin also has activity against ciprofloxacin-susceptible *Escherichia coli* and other Enterobacteriaceae. However, against *Enterococcus faecium* and ciprofloxacin-resistant *E. coli,* gemifloxacin and the other quinolones gemifloxacin has similarly limited activity. Against quinolone-resistant *S. pneumoniae,* the activity of gemifloxacin is 2-128 fold higher than that of the other quinolones. Against most quinolone-resistant *Staphylococcus aureus* and vancomycin-resistant *Enterococcus* spp., the activity of gemifloxacin is more potent than that of the other quinolones.
The antimicrobial activity of gemifloxacin against common Japanese respiratory tract and urinary tract pathogens examined in this study indicates that gemifloxacin can be a highly potent agent for the treatment of RTI and UTI in Japan.

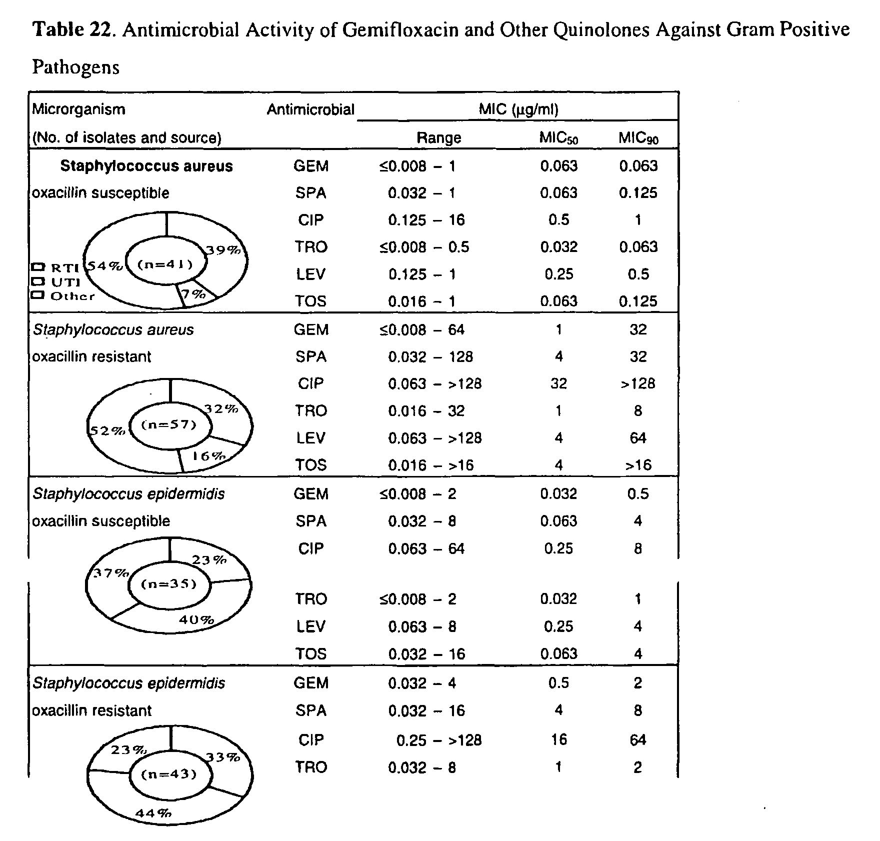

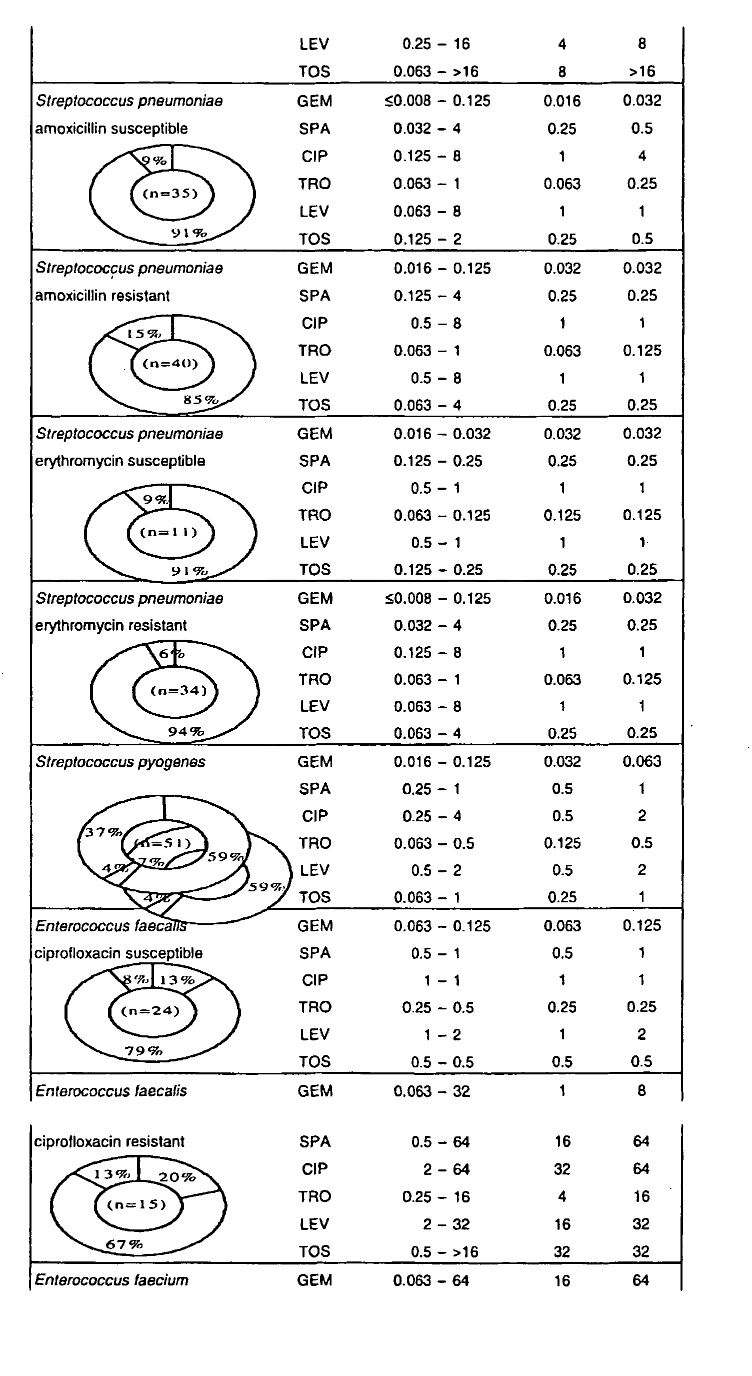

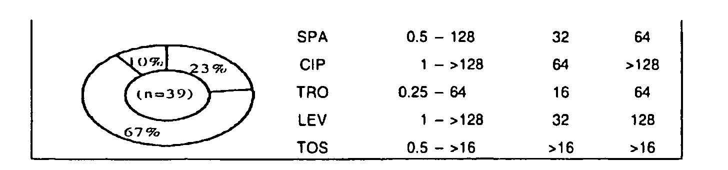

Oxacillin susceptible = MIC ≤2 µg/ml; oxacillin resistant = MIC ≥4 µg/ml; amoxicillin susceptible = MIC ≤0.5 µg/ml; amoxicillin resistant = MIC ≥1 µg/ml; erythromycin susceptible = MIC ≤0.25 µg/ml; erythromycin resistant = MIC ≥0.5 µg/ml; ciprofloxacin susceptible = MIC ≤1 µg/ml; ciprofloxacin resistant = MIC ≥2 µg/ml

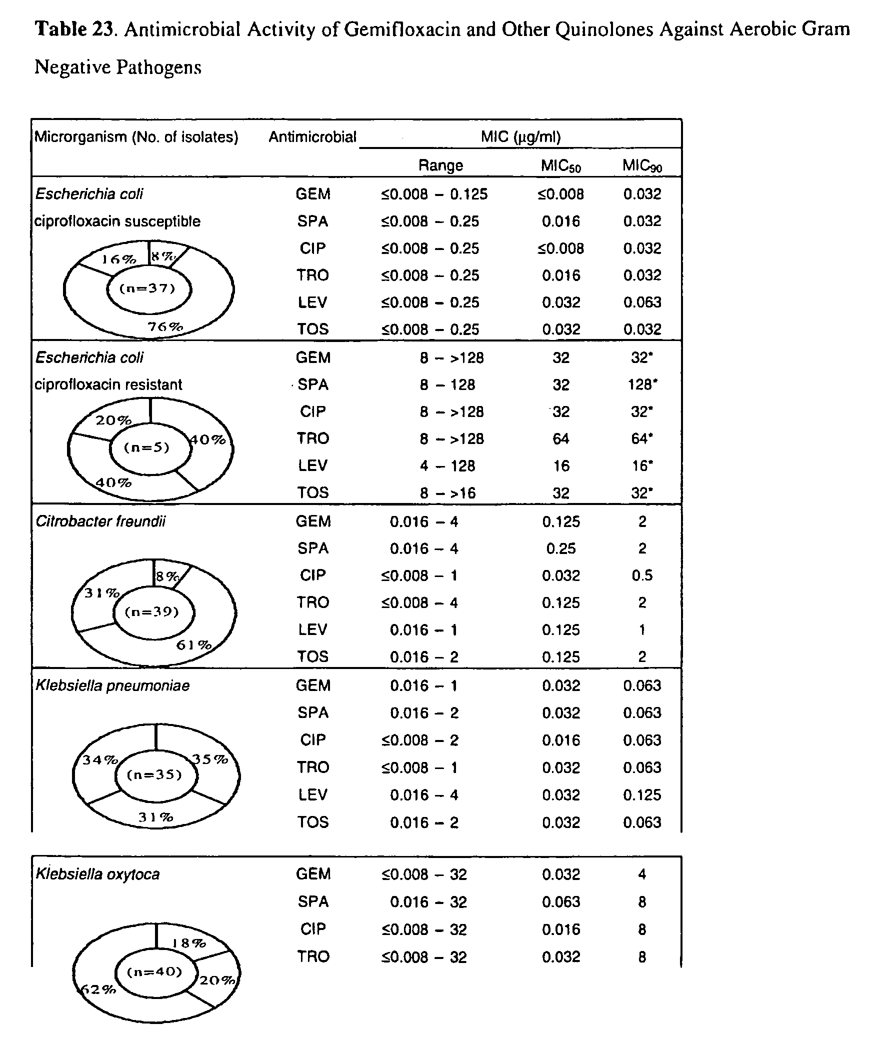

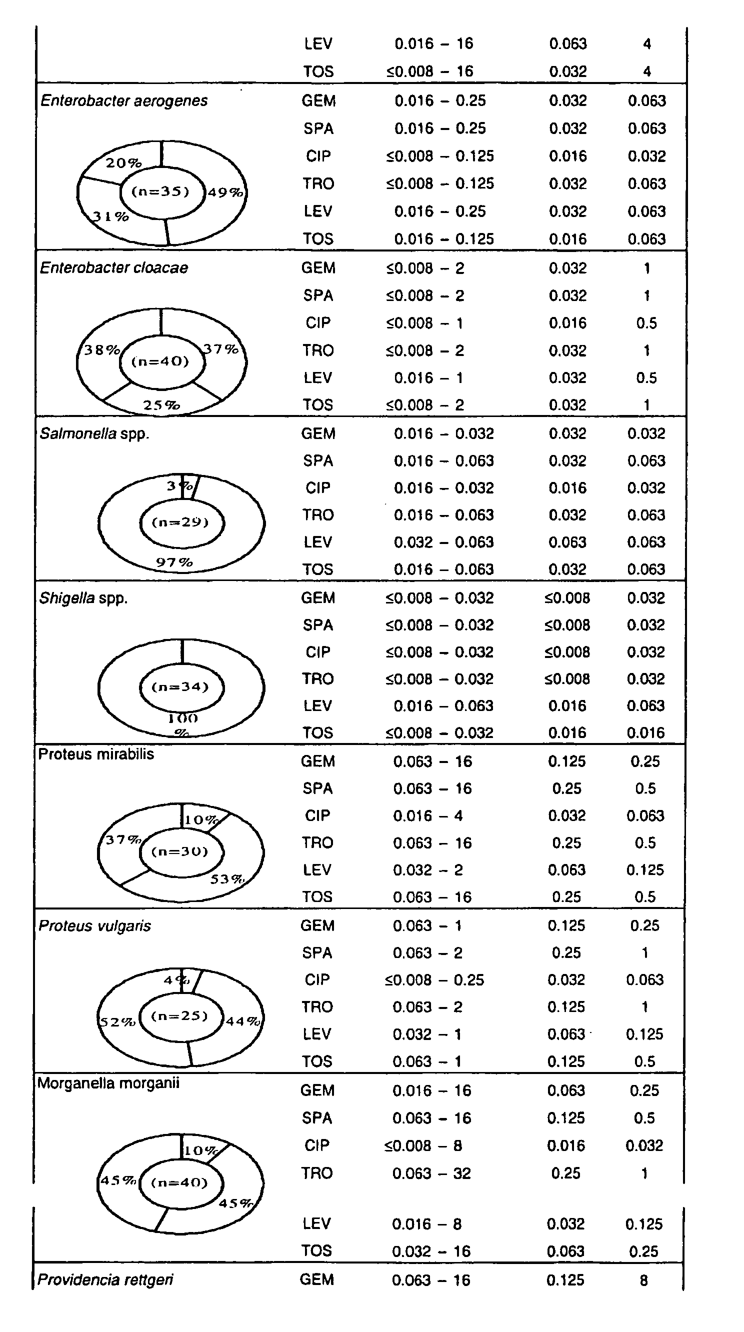

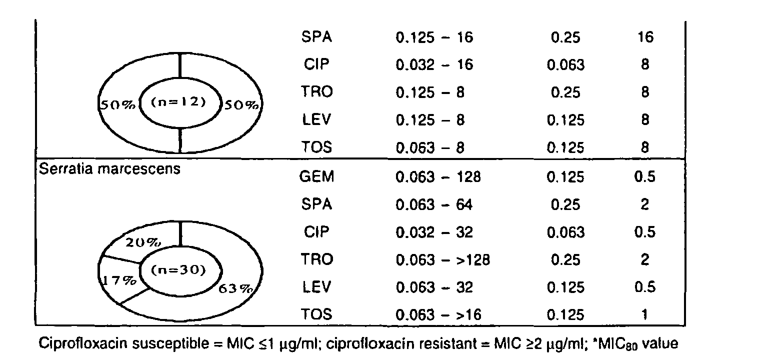

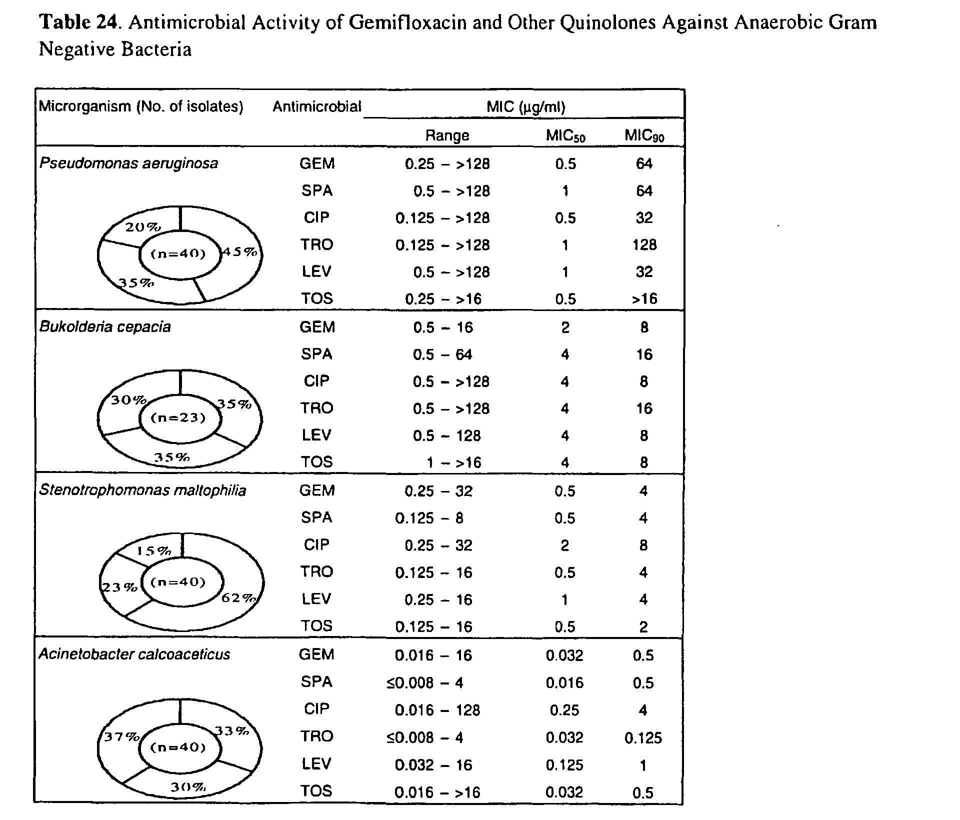

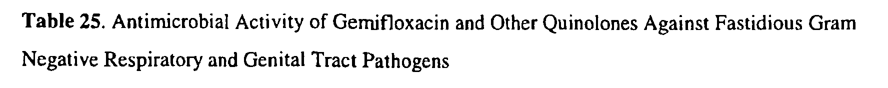

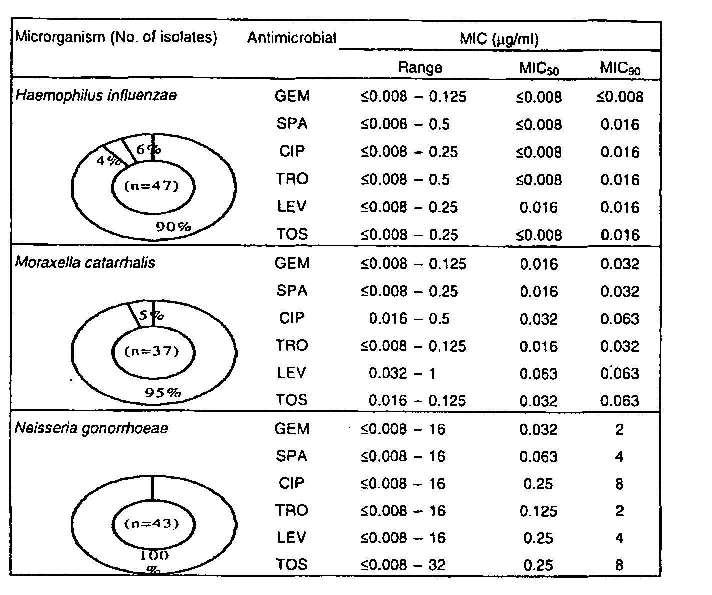

**Table 26.**

| Antimicrobial Activity of Gemifloxacin and Other Quinolones Against Drug-resistant Strains of Gram Positive Cocci | | | | | | | |
|---|---|---|---|---|---|---|---|
| Microrganism | Strain | MIC (µg/ml) | | | | | |
| | | GEM | SPA | CIP | TRO | LEV | TOS |
| *Staphylococcus aureus* | TMS1* | 0.032 | 0.125 | 1 | 0.125 | 0.5 | 0.25 |
| quinolone resistant | TMS2* | 1 | 2 | 4 | 0.5 | 4 | 2 |
| | TMS3 | 32 | 128 | >128 | 128 | >128 | >16 |
| | TMS4 | 4 | 8 | 16 | 0.5 | 16 | 4 |
| | TMS5 | 2 | 8 | 16 | 1 | 4 | 4 |
| | TMS6 | 0.25 | 2 | 8 | 0.5 | 2 | 2 |
| *Streptococcus pneumoniae* | TMS1 | 0.032 | 0.5 | 2 | 0.25 | 1 | 0.5 |
| quinolone resistant | TMS2 | 0.5 | 8 | 64 | 1 | 16 | 8 |
| *Enterococcus faecium* (vanA) | TMS1 | 16 | 32 | >128 | 8 | 64 | >16 |
| vancomycin resistant | TMS2 | 2 | 0.5 | 2 | 1 | 2 | 2 |
| | TMS3 | 4 | 64 | 32 | 8 | 32 | >16 |
| | TMS4 | 4 | 64 | 32 | 8 | 32 | >16 |
| | TMS5 | 2 | 2 | 4 | 2 | 4 | 4 |
| *Enterococcus laecium* (van B) | TMS6 | 32 | 64 | 64 | 32 | 32 | >16 |
| vancomycin resistant | TMS7 | 32 | 32 | 128 | 32 | 32 | >16 |
| *Enterococcus gallinarum* (vanC1) | TMS1 | 0.032 | 0.25 | 1 | 0.125 | 1 | 0.25 |
| | TMS2 | 0.125 | 0.5 | 2 | 0.25 | 2 | 0.5 |
| | TMS3 | 0.125 | 0.5 | 2 | 0.25 | 1 | 0.5 |
| | TMS4 | 0.032 | 0.25 | 0.5 | 0.063 | 0.5 | 0.125 |

| | | | | | | | |
|---|---|---|---|---|---|---|---|
| *Spontaneous mutant resistant to ciprofloxacin. Vancomycin-resistant; MIC ≥8 µg/ml | | | | | | | |

The invention provides a method for modulating metabolism of respiratory or urinary tract pathogenic bacteria. Skilled artisans can readily choose respiratory or urinary tract pathogenic bacteria or patients infected with or suspected to be infected with these organisms to practice the methods of the invention. Alternatively, the bacteria useful in the methods of the invention may be those described herein.

Also provided by the invention is a method of treating or preventing a bacterial infection by respiratory or urinary tract pathogenic bacteria comprising the step of administering an antibacterially effective amount of a composition comprising a quinolone, particularly a gemifloxacin compound to a mammal, preferably a human, suspected of having or being at risk of having an infection with respiratory or urinary tract pathogenic bacteria.

While a preferred object of the invention provides a method wherein said respiratory or urinary tract pathogenic bacteria is selected from the group consisting of: *Staphylococcus aureus* (including oxacillin-susceptible, oxacillin-resistant, and quinolone-resistant strains), *Staphylococcus epidermidis* (including oxacillin-susceptible, oxacillin-resistant strains), *Streptococcus pneumoniae* (including qauinolone-resistant, amoxicillin-susceptible, amoxicillin-resistant, erythromycin-susceptible and erythromycin-resistant strains), *Streptococcus pyrogenes, Enterococcus faecalis* (including ciprofloxacin-susceptible and ciprofloxacin-resistant strains), *Enterococcus faecium* (including vanA and vanB vancomycin-resistant strains), *Enterococcus gallinarum* (including vanC1), *Escherichia coli* (including ciprofloxacin-susceptible and ciprofloxacin-resistant strains), *Citrobacter freundii, Klebsiella pneumoniae, Klebsiella oxytoca, Enterobacter aerogenes, Enterobacter cloacae, Salmonella spp., Shigella spp., Proteus mirabilis, Proteus vulgaris, Morganella morganii, Providencia rettgeri, Serratia marcescens, Pseudomonas aeruginosa, Bukolderia cepacia, Stenotrophomonas maltophilia, Acintobacter calcoaceticus, Haemophilus influenzae, Moraxella catarrhalis,* and *Neisseria gonorrhoeae.* Other respiratory or urinary tract pathogenic bacteria may also be included in the methods. The skilled artisan may identify these organisms as provided herein as well as using other methods known in the art, *e.g.* MIC tests.

The present invention provides, among other things, methods for using a composition comprising a quinolone, particularly a gemifloxacin compound against anaerobic pathenogenic bacteria.

This invention was based, in part, on analyses evaluating the comparative activity of gemifloxacin against various anaerobic pathogens. An objective of these analyses was to determine the *in vitro* activity of gemifloxacin against a variety of anaerobic bacteria compared with that of trovafloxacin, sparfloxacin, ciprofloxacin and imipenem..

The *in vitro* activity of gemifloxacin was compared with that of trovafloxacin, sparfloxacin, ciprofloxacin and imipenem against 68 anaerobic reference strains and 491 recent clinical isolates. MICs were determined by the NCCLS-recommended agar dilution method, using Brucella HK agar, supplemented with 5% laked sheep blood. Against the reference strains, gemifloxacin shows more potent activity than sparfloxacin and ciprofloxacin. Gemifloxacin is more active than trovafloxacin against Gram positive pathogens, but less active than trovafloxacin against Gram negative pathogens. Against clinical isolates, the rank order of potency against *Bacteroides* spp., *Prevotella bivia* and other *Prevotella* spp. was trovafloxacin > gemifloxacin ≅ sparfloxacin > ciprofloxacin. The MIC₉₀ values of gemifloxacin, trovafloxacin, sparfloxacin and ciprofloxacin against these Gram negative pathogens were 8-32 µg/ml, 1-8 µg/ml, 4-32 µg/ml, 32-256 µg/ml, respectively. Gemifloxacin shows potent activity, however, against other Gram negative pathogens (*Prevotella intermedia, Porphyromonas* spp. and *Fusobacterium nucleatum),* with MIC₉₀s of 0.125-0.5 µg/ml. Against almost all anaerobic cocci and Gram positive pathogens, gemifloxacin is the most active quinolone tested. The MIC₉₀s of gemifloxacin against these isolates (except *Peptostreptococcus magnus* and *Clostridium difficile*) are 0.125-2 µg/ml.

Gemifloxacin is less active against *C. difficile*. Cross-resistance is observed among quinolones. From these results, it is indicated that gemifloxacin has good clinical potential for the treatment of Gram positive anaerobe infections but limited activity against Gram negative anaerobes.

Gemifloxacin has been reported that this agent is highly active against Gram positive and Gram negative aerobic pathogens (Cormican, *et al., Antimicrobial Agents Chemotherapy,* 41: 204-211 (1997); Hohl, *et al., Clinical Microbiology Infections,* 4: 280-284 (1998)). By contrast, the antimicrobial activity of gemifloxacin against anaerobic pathogens is not well known. This study investigated the *in vitro* activity of gemifloxacin against a variety of anaerobic bacteria compared with that of trovafloxacin, sparfloxacin, ciprofloxacin and imipenem.

A total of 68 Gram positive and Gram negative reference strains of anaerobic pathogens and some fastidious microaerophilic anaerobes were examined. In addition, 491 clinical strains isolated between 1994 and 1997 were also studied. *Propionibacterium acnes* strains, which were mainly isolated from purulent specimens were included in the study. Gemifloxacin, trovafloxacin, sparfloxacin, ciprofloxacin and imipenem of known potency were used.

MICs were determined by an agar dilution method. Brucella HK agar (Kyokuto, Tokyo, Japan) supplemented with 5% laked sheep blood was used as the test medium. A 10⁵ CFU/spot of test strains was inoculated and incubated at 37°C in an anaerobic chamber (82% N₂, 10% CO₂, 8% H₂) for 48 h. *Bacteroides fragilis* ATCC25285 and GAI 5562 were used as control strains.

The MIC data are shown in Tables 27-29.

Overall, gemifloxacin shows more potent activity than sparfloxacin and ciprofloxacin. Gemifloxacin is more active than trovafloxacin against most of the Gram positive and some Gram negative pathogens, but is less active than trovafloxacin against *Bacteroides* spp. and *Prevotella* spp. (except *Prevotella intermedia*). *Bacteroides* spp., *Prevotella bivia* and *Clostridium difficile* are less susceptible to gemifloxacin. Gemifloxacin shows potent activity against other Gram positive and Gram negative anaerobes. Results indicate that gemifloxacin has clinical potential for the treatment of Gram positive anaerobe infections.

**Table 27.**

| Antimicrobial activity of gemifloxacin and other agents against Gram positive anaerobic and facultative anaerobic pathogens | | | | | |
|---|---|---|---|---|---|
| Bacterial strain | Antimicrobial agent and MIC (µg/ml) | | | | |
| | Gemifloxacin | Trovafloxacin | Sparfloxacin | Ciprofloxacin | Imipenem |
| *Peptostreptococcus anaerobius* ATCC27337 | 0.06 | 0.06 | 0.5 | 0.5 | 0.06 |
| *Peptostreptococcus asaccharolyticus* WAL3218 | 0.125 | 0.5 | 0.25 | 2 | 0.125 |
| *Peptostreptococcus indolicus* GAI0915 | ≤0.03 | 0.125 | 0.06 | 1 | ≤0.03 |
| *Peptostreptococcus magnus* ATCC29328 | ≤0.03 | 0.125 | 0.125 | 0.25 | 0.25 |
| *Peptostreptococcus micros* VPI5464-1 | 0.125 | 0.06 | 0.5 | 0.5 | 0.125 |
| *Peptostreptococcus prevotil* ATCC9321 | 0.06 | 0.25 | 0.25 | 1 | 0.125 |
| *Staphylococcus saccharolyticus* ATCC14953 | ≤0.03 | 0.06 | 0.25 | 0.5 | ≤0.03 |
| *Atopobium parvulus* VPI0546 | 0.125 | 0.25 | 0.25 | 1 | 0.125 |
| *Streptococcus constellatus* ATCC27823 | ≤0.03 | 0.125 | 0.5 | 1 | 0.25 |
| *Streptococcus intermedius* ATCC27335 | 0.06 | 0.125 | 0.5 | 2 | 0.25 |
| *Gemella morbillorum* ATCC27824 | 0.25 | 0.25 | 0.25 | 0.5 | ≤0.03 |
| *Clostridium clostridioforme* NCTC11224 | 0.5 | 2 | 16 | 16 | 0.25 |
| *Clostridium difficile* GAI10029 | 2 | 1 | 8 | 8 | 4 |
| *Clostridium perfringens* ATCC13124 | 0.06 | 0.125 | 0.125 | 0.25 | 0.125 |
| *Clostridium septicum* ATCC12464 | 0.06 | 0.125 | 0.125 | 0.25 | ≤0.03 |
| *Clostridium sordellii* ATCC9714 | 0.5 | 0.25 | 2 | 2 | ≤0.03 |
| *Clostridium ramosum* ATCC25582 | 0.5 | 0.5 | 4 | 16 | 0.25 |
| *Propionibacterium acnes* ATCC11828 | 0.25 | 1 | 0.25 | 0.5 | 0.125 |
| *Propionibacterium granulosum* ATCC25564 | 0.125 | 0.25 | 0.125 | 0.5 | 0.125 |
| *Eubacterium lentum* ATCC25559 | 0.25 | 0.5 | 0.5 | 1 | 2 |
| *Actinomyces odontolyticus* GAI91002 | 4 | 4 | 4 | 16 | 0.5 |
| *Bifidobacterium adolescentis* ATCC15703 | 0.25 | 1 | 0.5 | 1 | 0.125 |
| *Bifidobacterium bifidum* JCM1255 | 2 | 4 | 2 | 8 | 0.125 |
| *Bifidobacterium breve* ATCC15700 | 1 | 4 | 2 | 8 | 1 |
| *Bifidobacterium longum* ATCC15707 | 1 | 4 | 2 | 8 | 0.25 |
| *Bifidobacterium pseudolongum* ATCC25526 | 1 | 4 | 2 | 8 | 0.25 |
| *Lactobacillus acidophilus* JCM1132 | 0.5 | 4 | 32 | 32 | 0.125 |
| *Lactobacillus brevis* subsp. *Brevis* JCM1059 | 0.5 | 1 | 2 | 32 | 0.06 |
| *Lactobacillus* casei subsp. *casei* JCM1134 | ≤0.03 | 0.06 | 0.125 | 1 | 0.5 |
| *Lactobacillus fermentum* JCM1173 | 0.25 | 1 | 2 | 16 | ≤0.03 |
| *Lactobacillus plantarum* JCM1149 | 2 | 4 | 8 | 64 | 0.06 |
| *Lactobacillus reuteri* JCM1112 | 0.25 | 1 | 2 | 32 | 0.06 |
| *Lactobacillus salivarius* subsp. *salivarius* JCM1231 | 0.06 | 0.125 | 0.5 | 2 | 0.5 |

**Table 28.**

| Antimicrobial activity of gemifloxacin and other agents against Gram negative anaerobic pathogens and facultative anaerobic pathogens | | | | | |
|---|---|---|---|---|---|
| Bacterial strain | Antimicrobial agent and MIC (µg/ml) | | | | |
| | Gemifloxacin | Trovafloxacin | Sparfloxacin | Ciprofloxacin | Imipenem |
| *Bacteroides fragilis* GA15562 | 0.25 | 0.125 | 1 | 4 | 0.25 |
| *Bacteroides fragilis* ATCC25285 | 0.5 | 0.125 | 1 | 4 | 0.5 |
| *Bacteroides fragilis* NCTC10581 | 0.5 | 0.25 | 1 | 4 | 0.125 |
| *Bacteroides fragilis* GA-I0558 | 0.5 | 0.125 | 1 | 2 | 1 |
| *Bacteroides fragilis* GA-I7955 | 2 | 0.25 | 1 | 8 | 4 |
| *Bacteroides fragilis* GAI10150 | 0.5 | 0.25 | 1 | 4 | 2 |
| *Bacteroides fragilis* GAI30079 | 8 | 0.5 | 2 | 32 | 256 |
| *Bacteroides fragilis* GAI30144 | 2 | 0.25 | 1 | 6 | 256 |
| *Bacteroides vulgatus* ATCC8482 | 0.25 | 0.5 | 2 | 2 | 1 |
| *Bacteroides distasonis* ATCC8503 | 0.25 | 0.5 | 2 | 2 | 1 |
| *Bacteroides ovatus* ATCC8483 | 1 | 0.5 | 2 | 8 | 0.25 |
| *Bacteroides thetaiotaomicron* ATCC29741 | 2 | 0.5 | 2 | 32 | 0.5 |
| *Bacteroides uniformis* ATCC8492 | 1 | 1 | 1 | 8 | 0.5 |
| *Bacteroides eggerthii* ATCC27754 | 4 | 0.5 | 4 | 16 | 0.125 |
| *Bacteroides ureolyticus* NCTC10941 | ≤0.03 | ≤0.03 | 0.06 | ≤0.03 | 0.25 |
| *Campylobacter gracilis* JCM8538 | ≤0.03 | ≤0.03 | ≤0.03 | ≤0.03 | 0.25 |
| *Sutterella wadsworthensis* ATCC51579 | 2 | 0.25 | 0.25 | 0.5 | 2 |
| *Prevotella bivia* ATCC29303 | 16 | 2 | 8 | 32 | 0.06 |
| *Prevotella buccaeATCC33574* | 2 | 0.5 | 2 | 1 | 0.25 |
| *Prevotella corporis* GAI91000 | 1 | 1 | 2 | 1 | ≤0.03 |
| *Prevotella heparinolytica* ATCC35895 | 0.125 | 0.125 | 0.5 | 2 | 0.125 |
| *Pravotella intermedia* ATCC25611 | 1 | 1 | 2 | 1 | 0.06 |
| *Prevotella melaninogenica* GAI5490 | 1 | 1 | 2 | 1 | ≤0.03 |
| *Prevotella oralis* ATCC33269 | 2 | 2 | 4 | 4 | ≤0.03 |
| *Prevotella oris* ATCC33573 | 0.5 | 1 | 2 | 1 | 0.06 |
| *Porphyromonas asaccharolytica* | 0.06 | 0.125 | 0.25 | 1 | ≤0.03 |
| ATCC25260 | | | | | |
| *Porphylomonas gingivalis* ATCC33277 | 0.06 | ≤0.03 | 0.125 | 0.25 | ≤0.03 |
| *Fusobacterium nucleatum* ATCC25586 | 0.125 | 0.5 | 1 | 2 | 1 |
| *Fusobacterium varium ATCC8501* | 0.5 | 4 | 8 | 8 | 4 |
| *Fusobacterium necrophorum* ATCC25286 | 0.25 | 0.25 | 1 | 1 | 0.125 |
| *Bilophilla wadsworthia* WAL7959 | 0.125 | 0.5 | 0.5 | 0.25 | 0.25 |
| *Desullomonas pigra* DSM749 | 0.06 | 0.125 | 0.06 | 0.25 | 0.06 |
| *Capnocytophaga ochracea* GAI5586 | ≤0.03 | ≤0.03 | ≤0.03 | 0.06 | 0.25 |
| *Veillonella parvula* ATCC10790 | 0.06 | 0.25 | 0.25 | 0.125 | 0.125 |
| *Veillonella dispar* ATCC17748 | ≤0.03 | 0.125 | 0.125 | 0.125 | 0.06 |

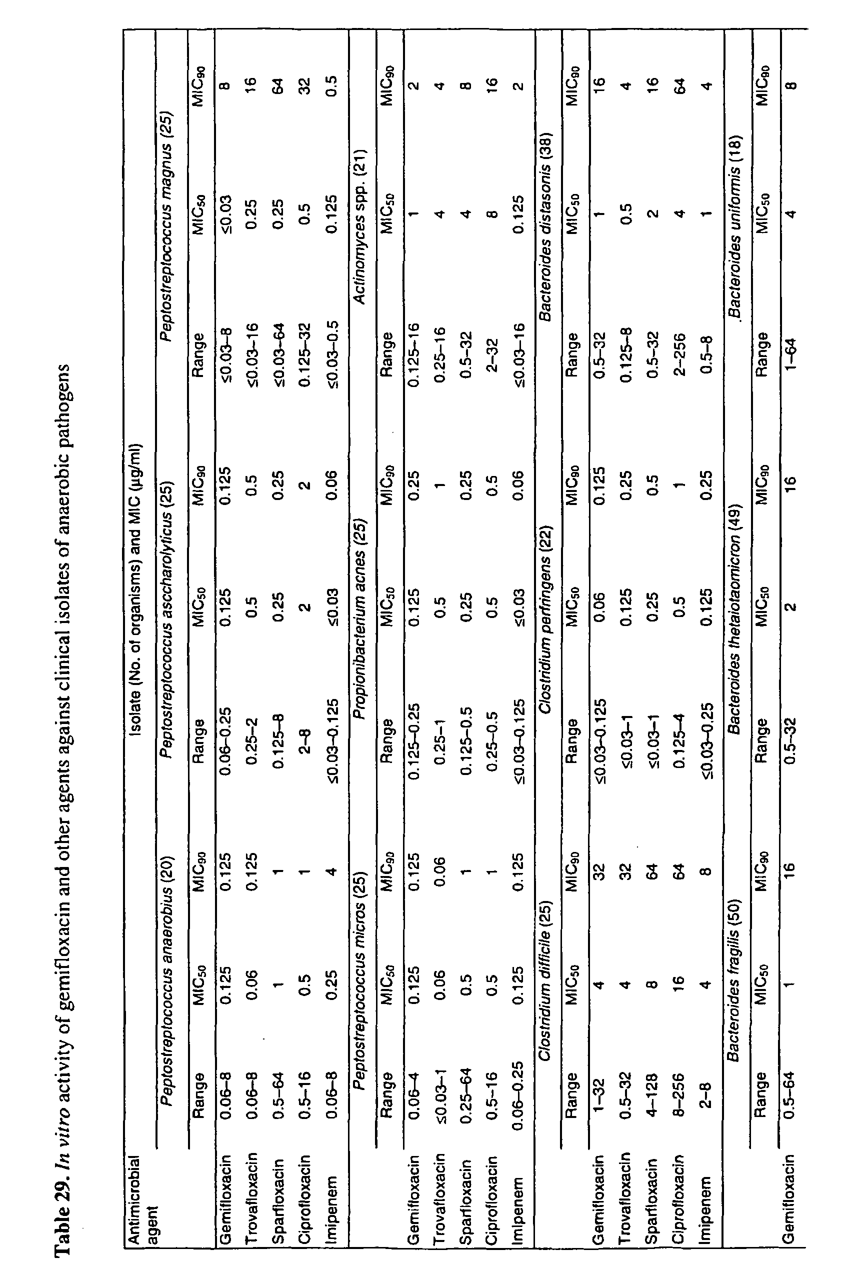

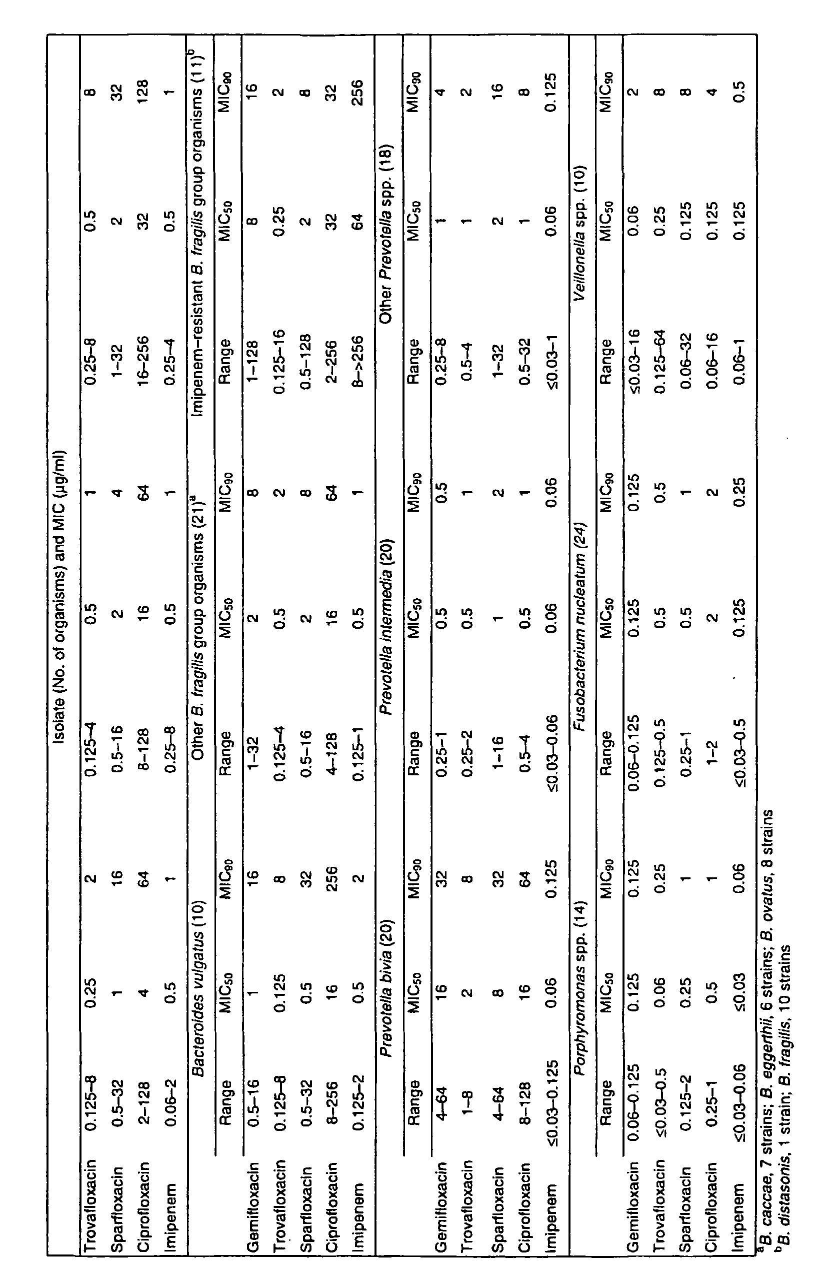

The invention provides a method for modulating metabolism of anaerobic pathogenic bacteria. Skilled artisans can readily choose anaerobic pathogenic bacteria or patients infected with or suspected to be infected with these organisms to practice the methods of the invention. Alternatively, the bacteria useful in the methods of the invention may be those described herein.

Also provided by the invention is a method of treating or preventing a bacterial infection by anaerobic pathogenic bacteria comprising the step of administering an antibacterially effective amount of a composition comprising a quinolone, particularly a gemifloxacin compound to a mammal, preferably a human, suspected of having or being at risk of having an infection with anaerobic pathogenic bacteria.

While a preferred object of the invention provides a method wherein said anaerobic pathogenic bacteria is selected from the group consisting of: *Peptostreptococcus anaerobius, Peptostreptococcus asaccharolyticus,Peptostreptococcus indolicus, Peptostreptococcus magnus, Peptostreptococcus micros, Peptostreptococcus prevotii, Staphylococcus saccharolyticus, Atopobium parvulus, Streptococcus constellatus, Streptococcus intermedius, Gemella morbillorum, Clostridium clostridioforme, Clostridium difficile, Clostridium perfringens, Clostridium septicum, Clostridium sordellii, Clostridium ramosum, Propionibacterium acnes, Propionibacterium granulosum, Eubacterium lentum, Actinomyces odontolyticus, Bifidobacterium adolescentis, Bifidobacterium bifidum, Bifidobacterium breve, Bifidobacterium longum, Bifidobacterium pseudolongum, Lactobacillus, Lactobacillus brevis subsp. Brevis, Lactobacillus casei subsp. casei, Lactobacillus fermentum, Lactobacillus plantarum, Lactobacillus reuteri, Lactobacillus salivarius subsp. salivarius, bacteroides fragilis, Bacteroides vulgatus, Bacteroides distasonis, Bacteroides ovatus, Bacteroides thetaiotaomicron, Bacteroides uniformis, Bacteroides eggerthii, Bacteroides ureolyticus, Campylobacter gracilis, Sutterella wadsworthensis, Prevotella bivia, Prevotella buccae, Prevotella corporis, Prevotella heparinolytica, Prevotella intermedia, Prevotella melaninogenica, Prevotella oralis, Prevotella oris, Porphyromonas asaccharolytica, Porphylomonas gingivalis, Fusobacterium nucleatum, Fusobacterium varium, Fusobacterium necrophorum, Bilophilla wadsworthia, Desulfomonas pigra, Capnocytophaga ochracea, Veillonella parvula, Veillonella dispar, Peptostreptococcus anaerobius, Peptostreptococcus asccharolyticus, Peptostreptococcus magnus, Peptostreptococcus micros, Propionibacterium acnes, Actinomyces spp., Clostridium difficile, Clostridium perfringens, Bacteroides distasonis, Bacteroides fragilis, Bacteroides thetaiotaomicron, Bacteroides uniformis, B. fragilis group organisms (B. caccae; B. eggerthii, B. ovatus), Imipenem-resistant B. fragilis group organisms (B. distasonis, B. fragilis), Prevotella bivia, Prevotella intermedia, Other Prevotella spp., Porphyromonas spp., Fusobacterium nucleatum,* and *Veillonella spp..* Other anaerobic pathogenic bacteria may also be included in the methods. The skilled artisan may identify these organisms as provided herein as well as using other methods known in the art, *e.g.* MIC tests.

The present invention provides, among other things, methods for using a composition comprising a quinolone, particularly a gemifloxacin compound against uropathogenic bacteria.

This invention was based, in part, on analyses evaluating the comparative activity of gemifloxacin against various uropathogens. An objective of these analyses was to determine the urinary excretion and bactericidal titers (UBT) among volunteers of 320 mg gemifloxacin *versus* 400 mg ofloxacin.

In a randomized cross-over study 16 volunteers (8 males, 8 females) received a single oral dose of 320 mg gemifloxacin *versus* 400 mg ofloxacin to assess the urinary excretion and bactericidal titers (UBT) in intervals up to 144 h. Ofloxacin showed higher urinary concentrations compared with gemifloxacin. The cumulative excretion (median, range) of gemifloxacin was 29.7% (8.4-48.7%) and that of ofloxacin 84.3% (46.5-95.2%) of the parent drug administered. The UBTs, i.e. the highest two-fold dilution (antibiotic free urine as diluent) of urine still bactericidal, were determined for a reference strain and nine uropathogens with the following MICs (µg/ml) for gemifloxacin/ofloxacin: *E. coli* ATCC 25922 (0.016/0.06), *K. pneumoniae* (0.03/0.06), *P. mirabilis* (0.125/0.125). *E. coli* (0.06/0.5), *P. aeruginosa* (1/4), *S. aureus* (0.008/0.25), *E. faecalis* (0.06/2), *S. aureus* (0.25/4), *E. faecalis* (0.5/32) and S. *aureus* (2/32). Generally the UBTs for Gram negative uropathogens were higher for ofloxacin than for gemifloxacin and for Gram positive uropathogens the UBTs were higher for gemifloxacin than for ofloxacin. For Enterobacteriaceae and susceptible Gram positive uropathogens, the initial UBTs were all at least 1:8 for either drug and thus the differences are not likely to be clinically relevant. However, if a peak UBT of at least 1:4 is considered desirable for treating complicated UTI, the gemifloxacin 320 mg dose can be too low for *P. aeruginosa* or a fluoroquinolone-resistant strain. Both gemifloxacin 320 mg and ofloxacin 400 mg can be inadequate for S. *aureus* (MIC 2/32) and for *E. faecalis* (MIC 0.5/32). The study indicates that a gemifloxacin 320 mg dose can be generally recommended for an UTI clinical trial.

Gemifloxacin, a new fluoroquinolone, has a broad antibacterial spectrum *in vitro* against Gram negative bacteria such as *Escherichia coli, Proteus mirabilis, Pseudomonas aeruginosa* and *Klebsiella pneumoniae,* but also against Gram positive bacteria such as streptococci and staphylococci. Gemifloxacin has a long plasma half life (*t*_{1/2}) and is approximately 20-30% excreted unchanged into urine, thereby reaching urinary concentrations which should provide sufficient antibacterial activity against most bacteria involved in the pathogenesis of UTI. A single oral dose of gemifloxacin (320 mg) was compared to ofloxacin (400 mg) in healthy volunteers in a combined pharmacokinetic/pharmacodynamic model determining their respective plasma and urinary concentrations and their urinary bactericidal activities against isolates of most common uropathogenic bacterial species.

One study was an open, randomized, crossover design trial, including 16 healthy volunteers (8 male, 8 female), median age (range): 31.5 years (18-40); median body weight: 66.5 kg (53.3-96.7); median height: 173.5 cm (160-179). Subjects received a single dose of 320 mg gemifloxacin or 400 mg ofloxacin with 14 days between doses.

Urine collections were made at -12-0 h pre-dose and 0-6, 6-12, 12-24, 24-48, 48-72, 72-96, 96-120 and 120-144 h post-dose. Gemifloxacin was assayed by HPLC/MS/MS (LLQ in plasma and urine was 0.0100 µg/ml). Ofloxacin was assayed by HPLC (LLQ in plasma was 0.00363 µg/ml and in urine 0.208 µg/ml).

MICs and MBCs were determined by a microdilution method with Mueller-Hinton broth. An inoculum of 1.3-9.4 x 10⁵ colony forming units (CFU) per ml was used. MIC was defined as the lowest concentration inhibiting visible growth after incubation at 37°C for 18 h and MBC by counting the CFUs on antibiotic-free Columbia agar supplemented with 5% blood, after additional incubation at 37°C for 18 h.

Bactericidal activity was defined as a reduction of CFUs of >99.9% (>3 logs). Serial dilutions, ranging from 1:2 to 1:1024 were prepared using drug free urine and UBTs determined by microdilution on a microplate with a final inoculum = 10⁵ CFU/ml. Bactericidal activity was determined according to recommended guidelines of the NCCLS (NCCLS, 12(19), M26-T, Villanova, PA (1992)). An UBT of 0 was taken as no bactericidal activity and an UBT of 1:1 was used only when undiluted urine displayed bactericidal activity.

Strains included a reference strain *E. coli* ATCC 25922, susceptible to nalidixic acid (Nal-S) and the following clinical isolates obtained from complicated UTI patients: *E. coli* (resistant to nalidixic acid), *K. pneumoniae, P. mirabilis , P. aeruginosa, Streptococcus aureus* (3 strains) and *Enterococcus faecalis* (2 strains).

UBTs transformed into ordinal data; scale: 1 for UBT = 0 to 12 for UBT ≥1024. The area under the UBT vs. time curve (AUBTC) was calculated by trapezoidal-rule. No formal statistical analysis was conducted.

Laboratory parameters displayed no clinically relevant changes, and there were no clinically significant differences between study phases. Gemifloxacin and ofloxacin were well tolerated in healthy male and female volunteers.

Urinary pH and volumes similar in the two study phases. Median urinary drug concentrations was determined. Median (range) renal excretion up to 144 h was 84.3% (46.5-95.2%) of dose for ofloxacin and 29.7% (8.4-48.7%) of dose for gemifloxacin.

MICs and MBCs (range 0.008-128µg/ml) for gemifloxacin and ofloxacin for each test organism are shown in Table 30. The median UBTs of both study drugs against the test organisms was detemined. For Gram negative uropathogens, UBT's are generally higher for ofloxacin than for gemifloxacin. For Gram positive uropathogens - UBTs are higher for gemifloxacin than for ofloxacin. Median UBTs of gemifloxacin exceed 0 for 5 days for the reference strain and decrease from 1:≥1024 to 1:1. Median UBTs of ofloxacin exceed 0 for 4 days for the reference strain and decrease from 1:≥1024 to 1:2. There is a wide range of UBTs although the variant coefficient of the laboratory method was proven to be low (Well, *et al*., Int. J. Antimicrob. Agents, 10: 31-38 (1998)). As it is the aim of antibacterial treatment to reach efficacy in all treated individuals, the lower range of the UBT results can be regarded relevant for clinical dosage recommendations. Initial UBTs are at least 1:8 for Enterobacteriaceae and susceptible Gram positive uropathogens for both study drugs. The observed differences are not likely to be clinically relevant.

Considering a peak UBT of at least 1:4 desirable for treating complicated UTI, gemifloxacin 320 mg can be too low for *P. aeruginosa* or a fluoroquinolone-resistant strain. Both gemifloxacin 320 mg and ofloxacin 400 mg can be inadequate for quinolone-resistant *S. aureus* (MIC 2/32), and *E. faecalis* (MIC 0.5/32).

AUBTCs of both study drugs are shown in Table 31. AUBTCs of ofloxacin and gemifloxacin are comparable in the reference strain. AUBTCs in the other test organisms are higher for ofloxacin in Gram negative organisms and higher for gemifloxacin in Gram positive organisms (corresponding with UBT data).

Urinary concentrations and renal excretion are higher for ofloxacin than for gemifloxacin. *In vitro* activity against the test strains is in general higher for gemifloxacin (except for *P. mirabilis*).
UBTs are higher for gemifloxacin in Gram positive strains, whereas in Gram negative strains UBTs are higher for ofloxacin. However, most UBTs are high enough to produce likely clinical efficacy. An oral dosage of 320 mg gemifloxacin once daily could be generally recommended for an UTI clinical trial.

**Table 30.**

| Minimal Inhibitory Concentrations (MIC)/Minimal Bactericidal Concentrations (MBC) Of Gemifloxacin *Versus* Ofloxacin Against *E. Coli* ATCC 25922 and Nine Clinical Isolates | | | | | | |
|---|---|---|---|---|---|---|
| Test strain | Laboratory No. | Inoculum CFU 10⁵/ml | Gemifloxacin | | Ofloxacin | |
| | | | MIC (µg/ml) | MBC (µg/ml) | MIC (µg/ml) | MBC (µg/ml) |
| 1 *E. coli* | ATCC 25922 | 1.3 | 0.016 | 0.016 | 0.06 | 0.06 |
| *2 K. pneumoniae* | 595 | 3.7 | 0.03 | 0.03 | 0.06 | 0.06 |
| 3 *P. mirabilis* | 414 | 2.5 | 0.125 | 0.25 | 0.125 | 0.125 |
| 4 *E. coli* | 523 | 3.4 | 0.06 | 0.06 | 0.5 | 0.5 |
| 5 *P. aeruginosa* | 568 | 5.6 | 1 | 2 | 4 | 4 |
| | | | | | | |
| 5 *S. aureus* | 83 | 7.1 | 0.008 | 0.008 | 0.25 | 0.25 |
| 7 *E. faecalis* | 60 | 7.0 | 0.06 | 0.06 | 2 | 2 |
| 9 *S. aureus* | 161 | 9.4 | 0.25 | 0.25 | 4 | 4 |
| 8 *S. aureus* | 636 | 5.6 | 2 | 2 | 32 | 32 |
| 10 *E. faecalis* | 55 | 6.0 | 0.5 | 2 | 32 | 128 |

**Table 31.**

| Area Under the Urinary Bactericidal Titer Curve (AUBTC) for Gemifloxacin and Ofloxacin | | | | |
|---|---|---|---|---|
| Test strain (No. of isolates) | | Laboratory No. | AUBTC (range) | |
| | | | Gemifloxacin | Ofloxacin |
| 1 | *E. coli* (16) | ATCC 25922 | 546 (174-816) | 537 (258-888) |
| 2 | *K. pneumoniae* (16) | 595 | 321 (72-564) | 414 (180-636) |
| 3 | *P. mirabilis* (16) | 414 | 261 (120-510) | 438 (276-654) |
| 4 | *E. coli* (16) | 523 | 237(48-384) | 276 (90-516) |
| 5 | *P. aeruginosa* (16) | 568 | 48 (12-300) | 99 (42-258) |
| 5 | *S. aureus* (16) | 83 | 540 (246-834) | 387 (162-570) |
| 7 | *E. faecalis* (16) | 60 | 246 (96-516) | 135 (90-204) |
| 9 | *S. aureus* (16) | 161 | 60 (0-144) | 39 (0-78) |
| 8 | *S. aureus* (13) | 636 | 108 (0-408) | 105 (0-279) |
| 10 | *E. faecalis* (13) | 55 | 39 (0-102) | 0 (0-54) |

The invention provides a method for modulating metabolism of uropathogenic bacteria. Skilled artisans can readily choose uropathogenic bacteria or patients infected with or suspected to be infected with these organisms to practice the methods of the invention. Alternatively, the bacteria useful in the methods of the invention may be those described herein.

Also provided by the invention is a method of treating or preventing a bacterial infection by uropathogenic bacteria comprising the step of administering an antibacterially effective amount of a composition comprising a quinolone, particularly a gemifloxacin compound to a mammal, preferably a human, suspected of having or being at risk of having an infection with uropathogenic bacteria.

While a preferred object of the invention provides a method wherein said uropathogenic bacteria is selected from the group consisting of: *K. pneumoniae, P. mirabilis, E. coli, P. aeruginosa, S. aureus,* and *E. faecalis.* Other uropathogenic bacteria may also be included in the methods. The skilled artisan may identify these organisms as provided herein as well as using other methods known in the art, *e.g.* MIC tests.

The present invention provides, among other things, methods for using a composition comprising a quinolone, particularly a gemifloxacin compound against nosocomial Gram negative bacteria.

This invention was based, in part, on analyses evaluating the comparative activity of gemifloxacin against various nosocomial Gram negative pathogens. An objective of these analyses was to determine the *in vitro* activity of gemifloxacin compared with trovafloxacin and grepafloxacin against 322 Gram negative clinical nosocomial isolates, collected in 1998 from six tertiary hospitals in Athens.

The newer quinolones are known to have an expanded activity spectrum against Gram positive bacteria. Isolates included Enterobacteriaceae and *Pseudomonas aeruginosa* from different patients and various sources (urine, pus, blood). The *in vitro* activity was tested by the agar dilution method and ciprofloxacin, ofloxacin, 2^{nd} and 3^{rd} generation cephalosporins, carbapenems and piperacillin were used as comparators. Results are shown in Tables 32-37. Gemifloxacin retains activity against nosocomial Gram negative strains comparable to that of ciprofloxacin, whilst exhibiting better activity against multi-resistant Acinetobacter spp., with a MIC₅₀ comparable with that of imipenem. Gemifloxacin (SB-265805) is a promising fluoroquinolone with extended spectrum against both Gram positive and Gram negative microrganisms. High resistance rates of nosocomial Gram negative isolates are a devastating reality in Greece, complicating the management and outcome of hospital acquired and ICU infections. An aim of this study was to compare the *in vitro* activity of gemifloxacin against Greek nosocomial Gram negative isolates with that of other antimicrobial agents.

Three hundred twenty-two (322) Gram negative isolates were collected in 1998 (May-October) from six tertiary hospitals in Athens (1 Children's Hospital, 1 Military Hospital, 1 Cancer Hospital and 3 General Hospitals). Isolates included *Enterobacteriaceae* and *Pseudomonas aeruginosa* from different infected patients and from various sources (urine, pus, blood).

The *in vitro* activity of gemifloxacin was tested by the agar dilution method according to NCCLS methodology and the following compounds were used as comparators: ciprofloxacin, trovafloxacin, grepafloxacin, ofloxacin, cefoxitin, piperacillin, ceftazidime, imipenem, meropenem, cefepime. Results were expressed as MIC₅₀ and MIC₉₀ values and distribution of MICs for each pathogen.

Tables 32-37 show the MIC distribution, range, MIC₅₀ and MIC₉₀ for each of the antimicrobials tested against the range of test organisms.

Gemifloxacin displays activity against nosocomial Gram negative strains comparable with that of ciprofloxacin. Gemifloxacin exhibits improved activity against multi-resistant *Acinetobacter* spp. (MIC₅₀s comparable with that of carbapenems) over ciprofloxacin. Gemifloxacin has the potential to play an important future role in the management of severe hospital acquired and ICU infections.

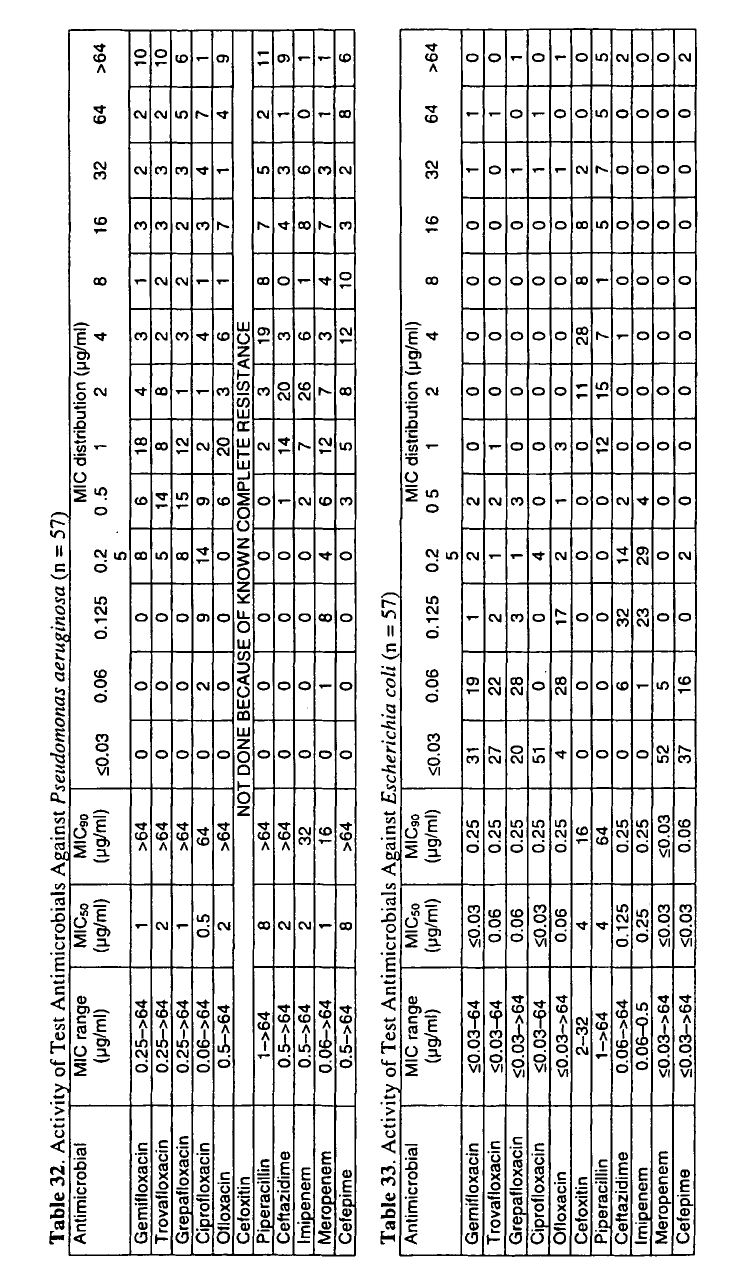

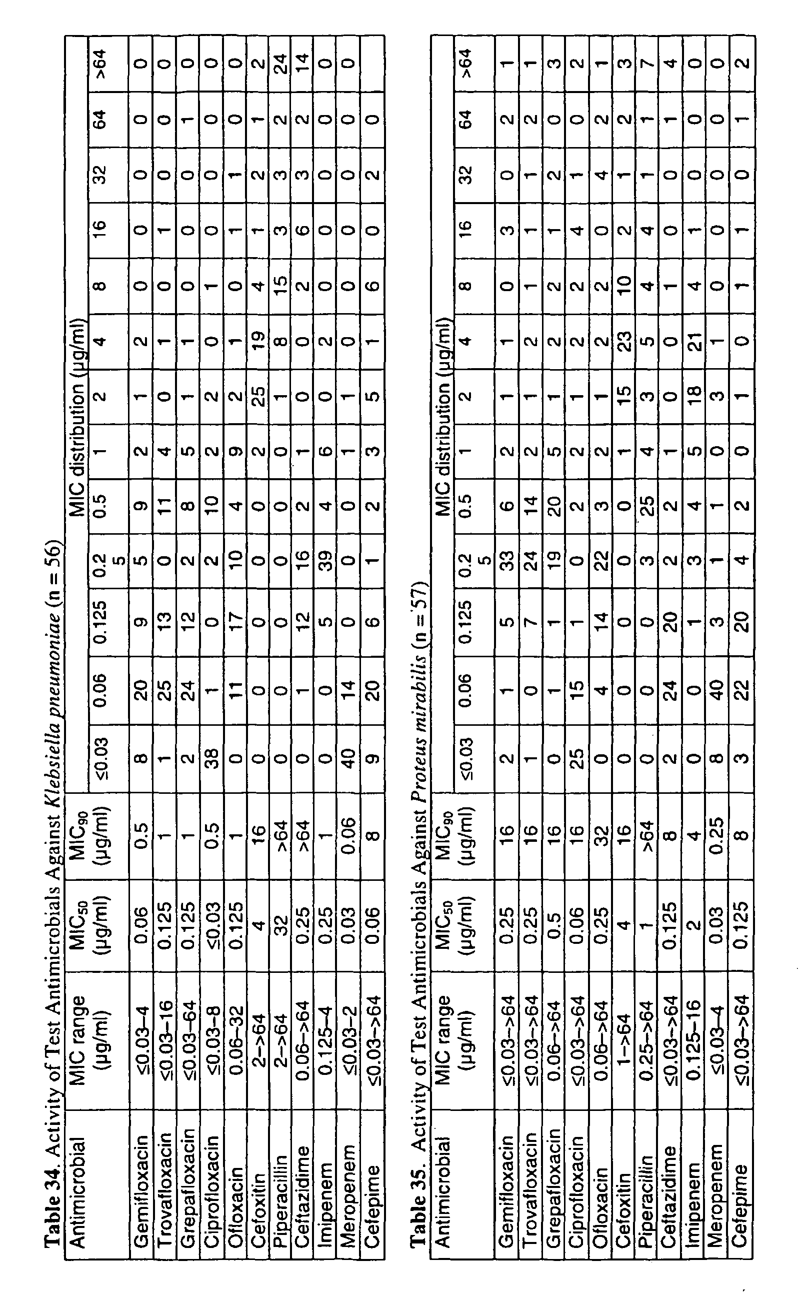

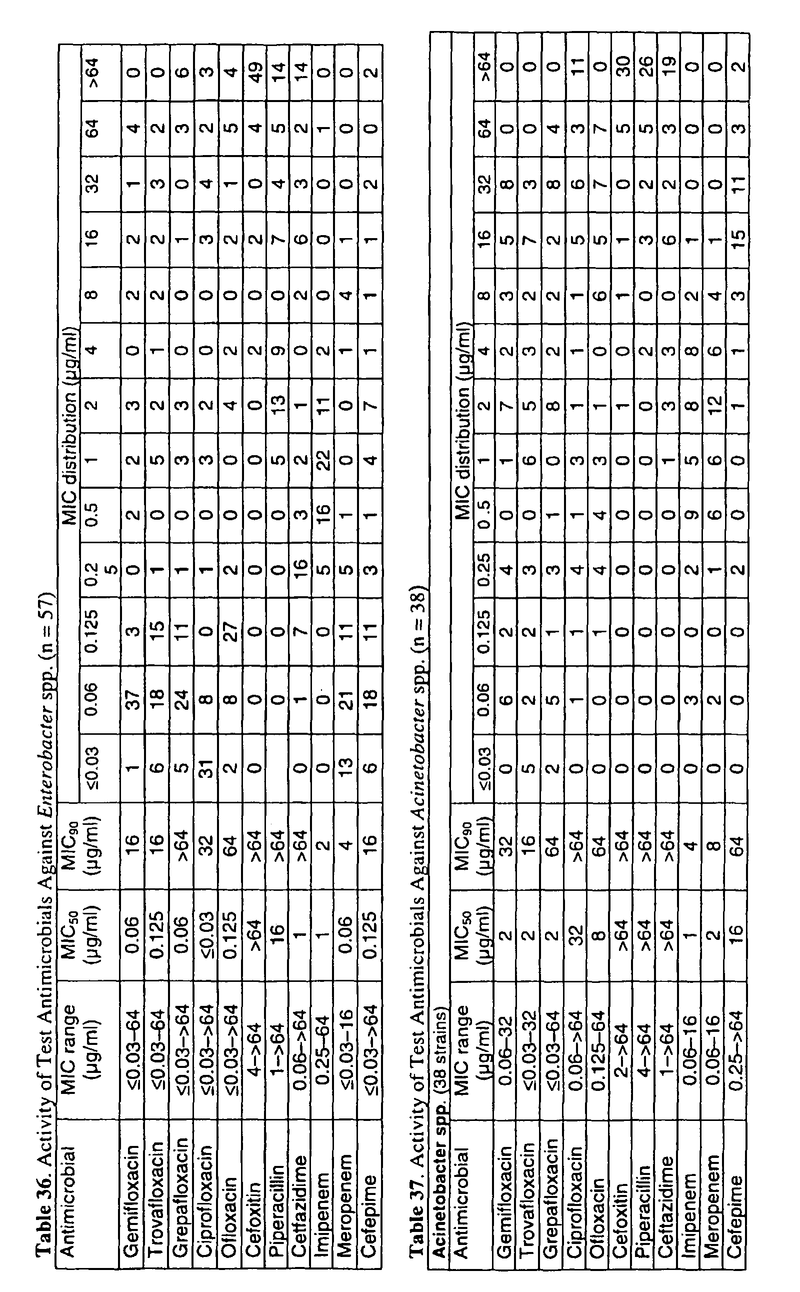

The invention provides a method for modulating metabolism of nosocomial Gram negative pathogenic bacteria. Skilled artisans can readily choose nosocomial Gram negative pathogenic bacteria or patients infected with or suspected to be infected with these organisms to practice the methods of the invention. Alternatively, the bacteria useful in the methods of the invention may be those described herein.

Also provided by the invention is a method of treating or preventing a bacterial infection by nosocomial Gram negative pathogenic bacteria comprising the step of administering an antibacterially effective amount of a composition comprising a quinolone, particularly a gemifloxacin compound to a mammal, preferably a human, suspected of having or being at risk of having an infection with nosocomial Gram negative pathogenic bacteria.

While a preferred object of the invention provides a method wherein said nosocomial Gram negative pathogenic bacteria is selected from the group consisting of: *Acinetobacter* spp., *Enterobacter* spp., *Proteus mirabilis, Klebsiella pneumoniae, Escherichia coli,* and *Pseudomonas aeruginosa.* Other nosocomial Gram negative pathogenic bacteria may also be included in the methods. The skilled artisan may identify these organisms as provided herein as well as using other methods known in the art, *e.g.* MIC tests.

The present invention provides, among other things, methods for using a composition comprising a quinolone, particularly a gemifloxacin compound against Gram positive bacteria.

This invention was based, in part, on analyses evaluating the comparative activity of gemifloxacin against various Gram positive pathogens. An objective of these analyses was to determine the comparative *in vitro* activity of gemifloxacin against a range of Gram positive cocci with that of other quinolones (ofloxacin, ciprofloxacin, grepafloxacin and trovafloxacin) and different classes of antimicrobials (glycopeptides, macrolides, azalides).

Gemifloxacin is a fluoroquinolone antimicrobial with a broad spectrum of activity including Gram positive bacteria. Gemifloxacin activity was compared with that of trovafloxacin, grepafloxacin, ciprofloxacin, ofloxacin and vancomycin against 373 Gram positive clinical isolates collected in 1998 in the Athens metropolitan area. Susceptibility testing was performed by agar dilution or epsilometer method (E-test), as recommended by the NCCLS. The broth microdilution technique was used to test gemifloxacin against pneumococci. Results are shown in Tables 38-44. These results indicate that gemifloxacin and trovafloxacin are more potent than comparator quinolones against Gram positive bacteria and especially against pneumococci.

There is an increasing amount of evidence showing that the problem of resistance in Gram positive organisms is becoming a global threat. There are reports of methicillin-resistant *Staphylococcus aureus* (MRSA) strains with intermediate susceptibility to vancomycin (VISA) having made their appearance along with macrolide, β-lactam and even quinolone-resistant pneumococci, (New England Journal of Medicine, 341: 233-239 (1999) and *Enterococcus faecium* strains with the VanA phenotype of resistance (high level vancomycin and teicoplanin resistance). In light of the continuing emergence of increasingly resistant strains, the development of new agents seems urgent. Gemifloxacin (SB-265805) is reported to combine the advantages of the quinolone class of antimicrobials - excellent oral bioavailability, few drug interactions and long half life - with an extremely broad antimicrobial spectrum and an activity even against strains resistant to many other different classes of antimicrobials.

A total of 373 Gram positive recent clinical isolates were tested. Of these, the 107 pneumococcal strains were nasopharyngeal isolates from healthy carriers, both children (3-7 years old) and adults (18-90 years old), all inhabitants of the metropolitan area of Athens. The specimens were collected during the 1998 winter period. The susceptibility profiles of the strains were assessed using the Epsilometer method (E-test). The isolates were subdivided into two groups according to their penicillin susceptibility patterns (penicillin-susceptible and penicillin-intermediate.

All other Gram positives tested were isolated from various sources (blood, urine, pus, sputum, prostatic fluid). These were obtained either from outpatients of the Infectious Diseases Outpatient Clinic of the 4^{th} Department of Internal Medicine of the University of Athens Medical School or from hospitalized patients in various wards of tertiary hospitals both in the capital area of Athens and the Greek province.

The susceptibility of staphylococcal strains to methicillin was tested by the inoculation of oxacillin-containing (at a concentration of 6 µg/ml) McConkey agar plates after a 48 h incubation at 30°C. The subtyping of the enterococcal strains employed the API ID32 Rapid Strep identification system (BioMerieux, Paris ,France). The susceptibility patterns of all those strains were assessed by the agar macrodilution method as recommended by the NCCLS.
Results are shown in Tables 38-44.

Gemifloxacin is the quinolone with the best anti-pneumococcal activity both against penicillin-susceptible and -intermediate strains (strains with MIC of penicillin ≥2 µg/ml were not included in the analysis). The activity of Gemifloxacin against enterococcal strains was moderate but with no particular discrimination against *E. faerium* and *E. faecalis* strains. Gemifloxacin and trovafloxacin seem equally potent against *S. aureus* strains irrespective of their methicillin susceptibility. Gemifloxacin also retains excellent *in vitro* activity against *S. epidermidis* strains, irrespective of methicillin susceptibility (the best among comparators).

**Table 38.**

| Activity Against Penicillin-susceptible and -intermediate *Streptococcus pneumoniae* Determined by E-test | | | | | | |
|---|---|---|---|---|---|---|
| Antimicrobial | Penicillin-susceptible *S. pneumoniae* | | | Penicillin-intermediate *S. pneumoniae* | | |
| | (n = 107) | | | (n = 24) | | |
| | MIC range | MIC₅₀ | MIC₉₀ | MIC range | MIC₅₀ | MIC₉₀ |
| Gemifloxacin | 0.002-0.19 | 0.032 | 0.064 | 0.004-0.125 | 0.064 | 0.23 |
| Trovafloxacin | 0.008-1 | 0.094 | 0.19 | 0.003-1 | 0.125 | 0.38 |
| Grepafloxacin | 0.047-1 | 0.19 | 0.38 | 0.047-0.5 | 0.19 | 0.38 |
| Ciprofloxacin | 0.094-2 | 0.75 | 2 | 0.125-≥32 | 0.75 | 1 |
| Ofloxacin | 0.75->32 | 2 | 4 | 0.75-6 | 2 | 3 |
| Teicoplanin | 0.023-3 | 0.064 | 0.38 | 0.023-0.125 | 0.064 | 0.125 |
| Vancomycin | 0.064-2 | 0.5 | 1 | 0.19-1.5 | 0.75 | 1 |
| Ceftrixone | 0.003-0.09 | 0.012 | 0.047 | 0.008-0.75 | 0.19 | 0.38 |
| | 4 | | | | | |
| Clarithromycin | 0.016-≥25 | 0.094 | 0.38 | 0.016-≥256 | 0.094 | ≥256 |
| | 6 | | | | | |
| Azithromycin | 0.012-≥25 | 0.75 | 3 | 0.38-≥256 | 1 | ≥256 |
| | 6 | | | | | |

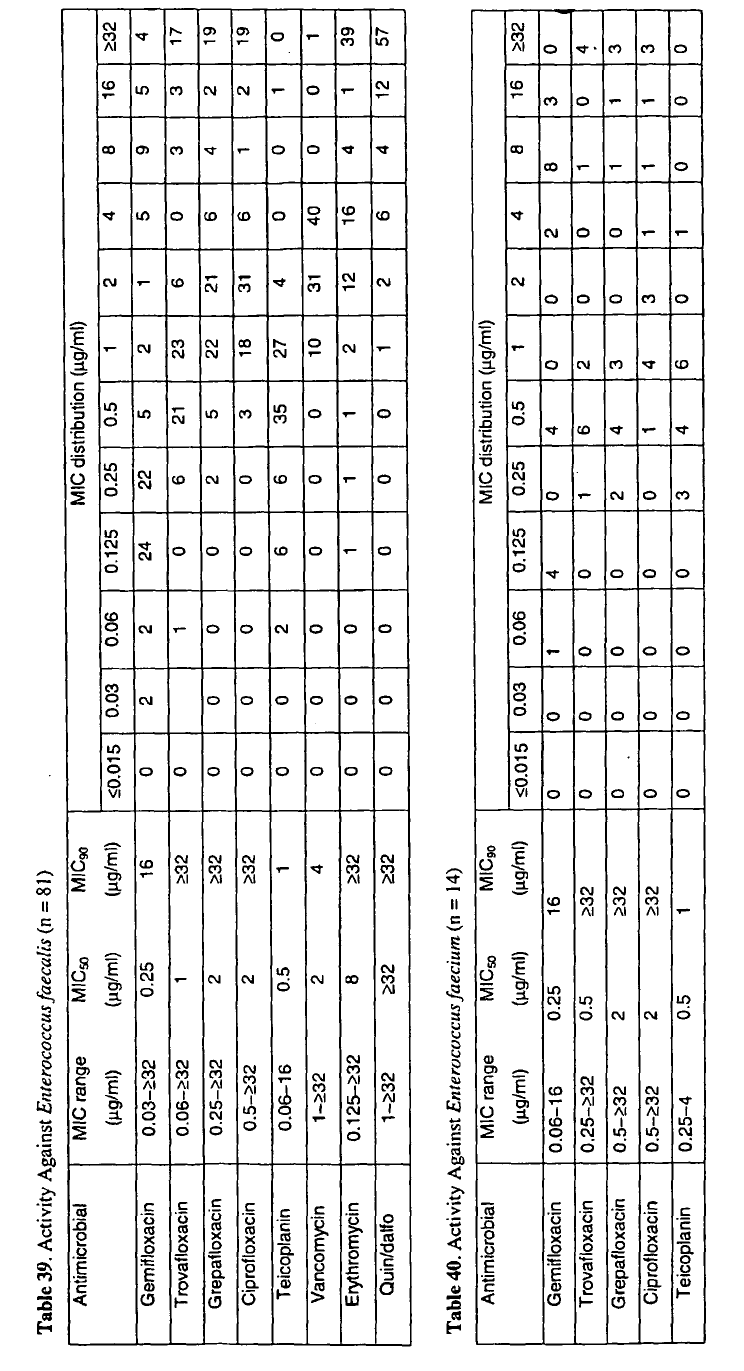

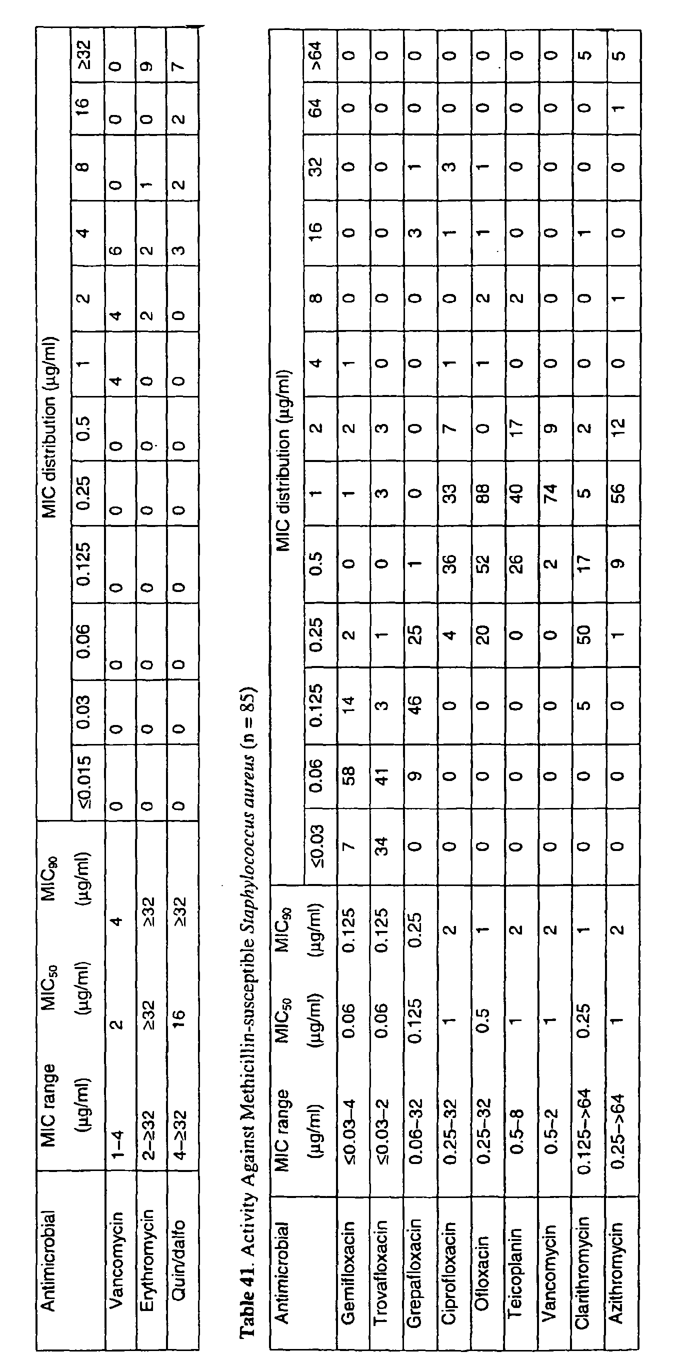

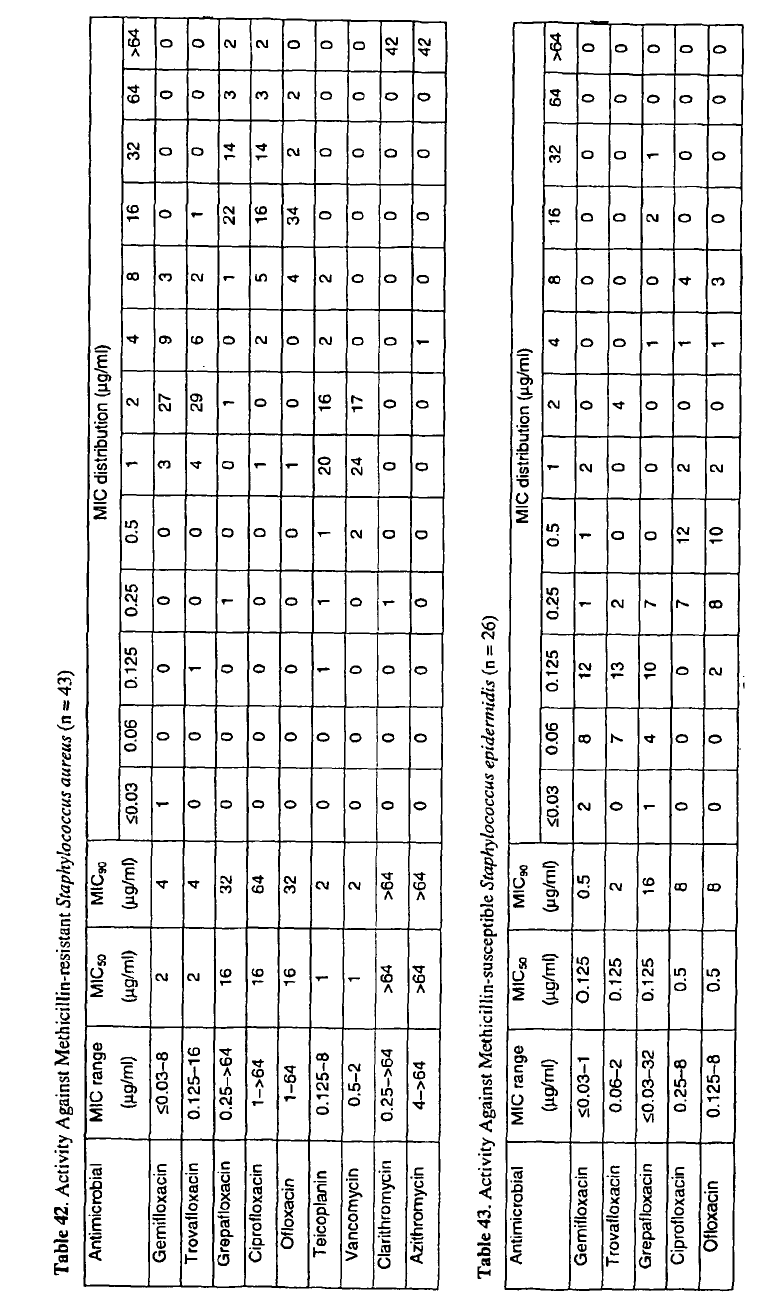

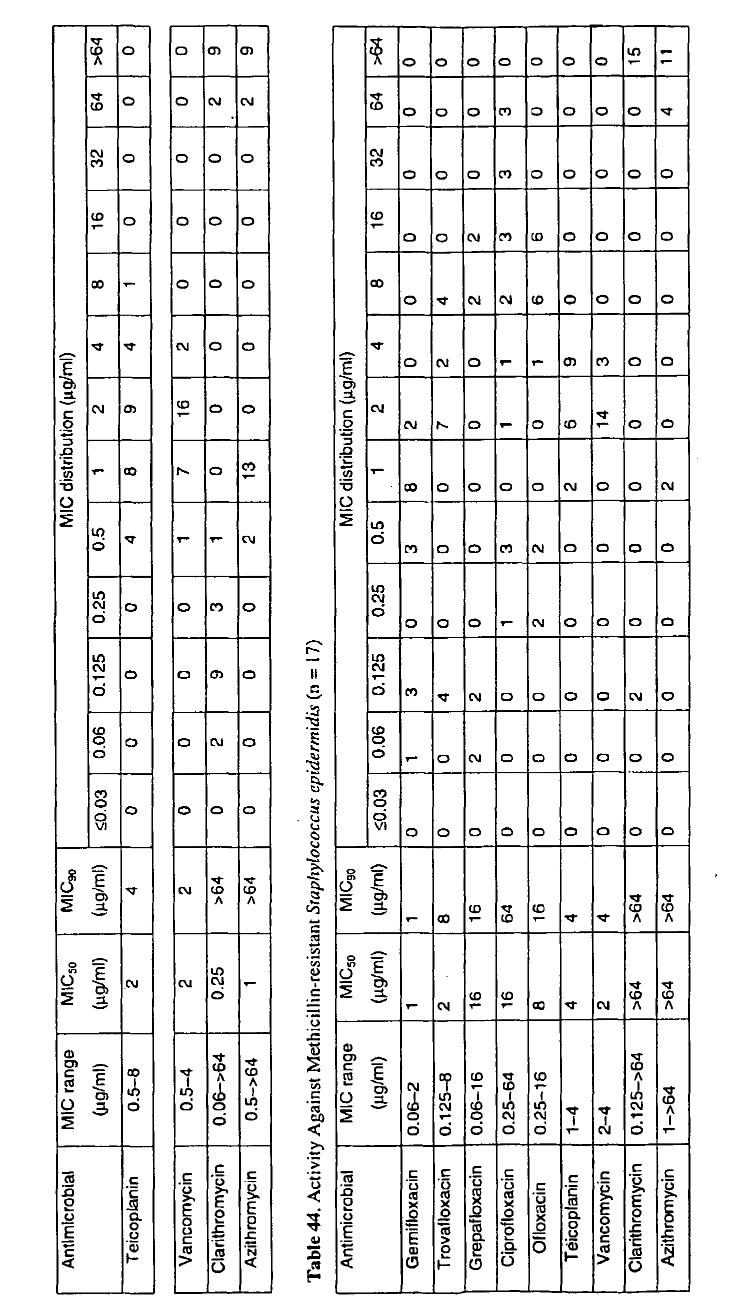

The invention provides a method for modulating metabolism of Gram positive pathogenic bacteria. Skilled artisans can readily choose Gram positive pathogenic bacteria or patients infected with or suspected to be infected with these organisms to practice the methods of the invention. Alternatively, the bacteria useful in the methods of the invention may be those described herein.

Also provided by the invention is a method of treating or preventing a bacterial infection by Gram positive pathogenic bacteria comprising the step of administering an antibacterially effective amount of a composition comprising a quinolone, particularly a gemifloxacin compound to a mammal, preferably a human, suspected of having or being at risk of having an infection with Gram positive pathogenic bacteria.

While a preferred object of the invention provides a method wherein said Gram positive pathogenic bacteria is selected from the group consisting of: *Streptococcus pneumoniae, Enterococcus faecalis, Enterococcus faecium, Staphylococcus aureus* (including Methicillin-susceptible and Methicillin-resistant strains), and *Staphylococcus epidermidis* (including Methicillin-susceptible and Methicillin-resistant strains). Other Gram positive pathogenic bacteria may also be included in the methods. The skilled artisan may identify these organisms as provided herein as well as using other methods known in the art, *e.g.* MIC tests.

The present invention provides, among other things, methods for using a composition comprising a quinolone, particularly a gemifloxacin compound against enteropathogenic bacteria.

This invention was based, in part, on analyses evaluating the comparative activity of gemifloxacin against various enteropathogenic bacteria. An objective of these analyses was to determine the in vitro activity of gemifloxacin and other antibiotics against enteropathogenic bacterial strains. Gemifloxacin was compared to trovafloxacin, grepafloxacin, ciprofloxacin, ofloxacin, norfloxacin, levofloxacin, nalidixic acid, ampicillin, amoxicillin, cefotaxime, gentamicin, doxycycline, colistin, co-trimoxazole, and for *C. jejuni* erythromycin

Certain classical oral antibiotics used against enteropathogenic bacterial isolates have recently demonstrated a poor in vitro activity (Aarestrup, *et al*., Antimicrob. Agents Chemother., 41: 2244-2250 (1997); Ramos, *et al.,* Eur. J. Clin. Microbiol. Infect. Dis., 15: 85-88 (1996); Sjogren, *et al.,* J. Antimicrob. Chemother., 40: 257-261 (1997); Soriano, *et al., J.* Antimicrob. Chemother., 34: 157-160 (1994); Stock, *et al.,* J. Antimicrob. Chemother., 43: 37-45 (1999); Stolk-Engelaar, *et al*., J. Antimicrob. Chemother., 36: 839-843 (1995)) and this fact prompted tests of antibiotics against these organisms. Here, the in vitro activity of gemifloxacin and 15 other antimicrobials was compared against recognized bacterial enteric pathogens and against *Hafnia alvei,* a bacteria with probable enteric pathogenic capacity (Ismaili, *et al*., J. Clin. Microbiol., 34: 2973-2979 (1997)).

A total of 288 enteropathogenic bacterial isolates from patients with acute gastroenteritis were studied, including 106 *Salmonella* spp., [*S*. *enteritidis* (75), *S*. *typhimurium* (19), *S. virchow* (5), *S.* t*shiongwe* (2), *S. newport* (1), *S. ohio* (1), *S. hadar (2),* and *S*. *georgia* (1)], 32 *Hafnia alvei, 22 Yersinia enterocolitica*, 21 *Shigella* spp., [*S. sonnei* (15), *S. f lexneri* (5), and *S. boydii* (1)], 16 *Aeromonas* spp. [*A. hydrophila* (12), and *A. sobria* (4)], and 91 *Campylobacter jejuni*. The microorganisms were isolated from 1996 to 1999 from stool samples. The strains were stored in skimmed-milk at -80 °C until studied.

The antibiotics tested were gemifloxacin, trovafloxacin, grepafloxacin, ciprofloxacin, ofloxacin, norfloxacin, levofloxacin, nalidixic acid, ampicillin, amoxicillin, cefotaxime, gentamicin, doxycycline, colistin, co-trimoxazole, and for *C. jejuni* erythromycin.

MICs were determined by an agar dilution method (NCCLS, 4th ed., M7-A4, Villanova, PA (1997)) on Mueller-Hinton agar, supplemented with 5% sheep blood for *Campylobacter jejuni* isolates. The plates were incubated aerobically at 35 °C for 24 h, except for *Campylobacter jejuni,* where a microaerophilic atmosphere was obtained by using Campy Pack (Becton Dickinson, Cockeysville, MD, USA), and incubation was for 48 h. All organisms were tested with an inoculum of ≅ 10⁴ cfu/spot. MICs were defined as the lower antibiotic concentration with no visible growth. *Escherichia coli* ATCC 25922, *Staphylococcus aureus* ATCC 29213 and *Pseudomonas aeruginosa* ATCC 27853 were used as controls. The antibiotic susceptibility breakpoints (mg/L) used to define the percentage of susceptible isolates were as follows: erythromycin: 0.5 mg/L, gemifloxacin, trovafloxacin, grepafloxacin and ciprofloxacin: 1 mg/L, colistin, co-trimoxazole, ofloxacin and levofloxacin: 2 mg/L, norfloxacin, gentamicin and doxycycline: 4 mg/L, ampicillin, amoxycillin and cefotaxime: 8 mg/L, and nalidixic acid: 16 mg/L.

Table 45 shows the activity of gemifloxacin and the other antibiotics tested against 288 enteropathogenic bacterial strains. Fluorquinolones were very active against all microorganisms except for *C. jejuni,* where only 32% of strains were susceptible. Rates of non-susceptible microorganisms for nalidixic acid were 27%, 5% and 6% for *Salmonella* spp., *Yersinia enterocolitica* and *Aeromonas* spp. respectively. Only 1% among 106 *Salmonella* spp. studied were non-susceptible to grepafloxacin (MIC = 2 mg/L). Table 46 shows MICs of quinolones studied against 6 *Salmonella* spp. with MICs for ciprofloxacin ≥0.25 mg/L.

Cefotaxime was active against 100% of organisms, except for *C. jejuni,* where only 57% were susceptible to this antibiotic. The activity of ampicillin and amoxicillin was variable, *Yersinia enterocolitica* and *Aeromonas* spp isolates were non susceptible to these betalactams, and 43% and 77% of *Shigella* spp and *Salmonella* spp. respectively were susceptible to both antibiotics. Ampicillin was more active than amoxicillin against *Hafnia alvei*. Their activity against *C. jejuni* was similar.

Gentamicin was the most active antibiotic tested, with only 4% of Salmonella spp. isolates being resistant to it.

Colistin was active against *Yersinia enterocolitica, Shigella* spp. and *Aeromonas* spp. Only 32% of *Salmonella* isolates were susceptible to colistin and its activity was also poor against *Hafnia alvei* and *C. jejuni.*

Except for *Shigella* spp. co-trimoxazole was very active, with only 4% of *Salmonella* spp. and *C. jejuni* being resistant to it.

Doxycycline was very active against *Aeromonas* spp. and its activity against the other organisms tested was variable with only 40% of *Campylobacter jejuni*, 43% of *Shigella* spp,, 22% of *Salmonella* spp., 22% of *Hafnia alvei* and 5% *Yersinia enterocolitica* being no susceptible to it.

77% of isolates of *C. jejuni* were inhibited by a concentration ≤0.5 mg/l of erythromycin, but only 1 strain was highly resistant to this antibiotic (MIC = 128 mg/L). The other isolates were all inhibited by a concentration ≤4 mg/L of erythromycin.

By weight, except for *C. jejuni,* gemifloxacin was the most active compound tested, 100% of isolates being inhibited by a concentration of 0.25 mg/L.

Antibiotics can be useful for treating bacterial diarrhea and also to prevent illness and the spread of infections (Reves, *et al.,* Arch. Int. Med., 148: 2421-2427 (1988)). The in vitro activity of many classical oral antibiotics against recently isolated bacterial enteropathogens is poor. Since 1988, an increase in the resistance of *Salmonella* spp. to classical antimicrobial such as ampicillin, chloramphenicol, tetracycline, cotrimoxazole has been described especially among *S. typhimurium* and *S. virchow* (Ramos, *et al*., Eur. J. Clin. Microbiol. Infect. Dis., 15: 85-88 (1996); Soriano, *et al.,* J. Antimicrob. Chemother., 34: 157-160 (1994); Threlfall, *et al.,* Clin. Microbiol. Infect., 5: 130-134 (1999)). Data confirms this fact with rates of non-susceptible *Salmonella* spp. to ampicillin, doxycycline, and co-trimoxazole of 23%, 22% and 4% respectively. MICs of ciprofloxacin ≥0.25 mg/L against *Salmonella* spp. isolates are considered for some authors as being clinically significant (Threlfall, *et al*., Clin. Microbiol. Infect., 5: 130-134 (1999)). Six isolates of *S. virchow, S. hadar, S. tshiongwe* and *S*. *newport* were studied, which MICs for ciprofloxacin were ≥0.25 mg/L, gemifloxacin being the most active quinolone against these isolates. Many *Shigella* spp. are resistant to ampicillin, cotrimoxazole and doxycycline (Soriano, *et al*., J. Antimicrob. Chemother., 34: 157-160 (1994)) as indicated by the data, with high rates of resistance in these species to these three antibiotics. *Hafnia alvei* in which virulence factors similar to some phenotypes of *E*. *coli* have been described (Ismaili, *et al.,* J. Clin. Microbiol., 34: 2973-2979, 1997)) is usually susceptible to quinolones, newer cephalosporins, carbapenems and piperacillin. Data indicates that a quinolone, cefotaxime, gentamicin and co-trimoxazole and in a less extent doxycycline can be used in the treatment of infections caused by this microorganism. Against *Aeromonas* spp., data confirms (Burgos, *et al.,* Eur. J. Clin. Microbiol. Infect. Dis., 9: 413-417 (1997)) the activity of quinolones, doxycycline, cotrimoxazole, gentamicin and cefotaxime and the ineficacy of aminopenicillins. *Yersinia enterocolitica* produces betalactamases that inactive some betalactams and resistance to other antibiotics has been described (Stock, *et al.,* J. Antimicrob. Chemother., 43: 37-45 (1999); Stolk-Engelaar, *et al*., J. Antimicrob. Chemother., 36: 839-843 (1995)). The antibiotics tested, except aminopenicillins, were very active against this microorganism.

High rates of resistance among *C*. *jejuni* strains are being described to quinolones (Aarestrup, *et al*., Antimicrob. Agents Chemother., 41: 2244-2250 (1997); Sjogren, *et al*., J. Antimicrob. Chemother., 40: 257-261 (1997); Soriano, *et al*., J. Antimicrob. Chemother., 34: 157-160 (1994)) but erythromycin and other macrolides are active against this microorganim. The data shows this increase in quinolone resistance in *C. jejuni* in comparison with isolates from 1996 (Soriano, *et al.,* J. Antimicrob. Chemother., 34: 157-160 (1994)). In contrast only 1 strain of *C*. *jejuni* showed a high resitance to erythromycin.

A quinolone can be used to treat a gastrointestinal infection when indicated. For *C*. *jejuni,* another antibiotic such as erythromycin must be considered. Gemifloxacin is a new quinolone with a good in vitro activity against important gastrointestinal pathogens and could be a good election in these infections. These results must be assessed in the context of in vivo trials before the clinical role of this new fluoroquinolone can be determined.

**Table 45.**

| **In vitro activity of gemifloxacin and 14* other antibiotics against enteropathogenic bacterial strains.** | | | | |
|---|---|---|---|---|
| | **MIC (mg/L)** | | | |
| | **Range** | **50%** | **90% %** | |
| **Susceptible** | | | | |
| ***Salmonella*** **spp. N= 106** | | | | |
| Gemifloxacin (SB 265805) | ≤0.015-0.25 | 0.03 | 0.12 | 100 |
| Trovafloxacin | ≤0.015-1 | 0.06 | 0.25 | 100 |
| Grepafloxacin | 0.03-2 | 0.06 | 0.25 | 99 |
| Ciprofloxacin | ≤0.015-1 | 0.03 | 0.12 | 100 |
| Ofloxacin | 0.06-2 | 0.12 | 0.5 | 100 |
| Norfloxacin | 0.06-4 | 0.06 | 0.5 | 100 |
| Levofloxacin | 0.06-1 | 0.06 | 0.25 | 100 |
| Nalidixic acid | 2->128 | 4 | >128 | 73 |
| Ampicillin | 1->128 | 4 | >128 | 77 |
| Amoxicillin | 1->128 | 1 | >128 | 77 |
| Cefotaxime | 0.06-0.5 | 0.12 | 0.12 | 100 |
| Gentamicin | 0.12-128 | 0.25 | 0.5 | 96 |
| Colistin | 0.5->128 | 8 | 8 | 32 |
| Doxycycline | 1-128 | 2 | 16 | 78 |
| Co-trimoxazole | ≤0.015->128 | 0.06 | 0.12 | 96 |

| ***Hafnia alvei*** **N= 32** | | | | |
|---|---|---|---|---|
| Gemifloxacin (SB 265805) | ≤0.015-0.06 | 0.03 | 0.03 | 100 |
| Trovafloxacin | 0.03-0.12 | 0.06 | 0.06 | 100 |
| Grepafloxacin | ≤0.015-0.06 | 0.03 | 0.06 | 100 |
| Ciprofloxacin | ≤0.015 | ≤0.015 | ≤0.015 | 100 |
| Ofloxacin | 0.03 | 0.03 | 0.03 | 100 |
| Norfloxacin | ≤0.015-0.03 | 0.03 | 0.03 | 100 |
| Levofloxacin | ≤0.015-0.03 | 0.03 | 0.03 | 100 |
| Nalidixic acid | 1-2 | 2 | 2 | 100 |
| Ampicillin | 2-64 | 16 | 64 | 14 |
| Amoxicillin | 16-128 | 64 | 64 | 0 |
| Cefotaxime | 0.12-0.5 | 0.25 | 0.5 | 100 |
| Gentamicin | 0.25-0.5 | 0.25 | 0.5 | 100 |
| Colistin | 0.5-16 | 8 | 16 | 6 |
| Doxycycline | 1-16 | 2 | 8 | 78 |
| Co-trimoxazole | 0.03-0.5 | 0.25 | 0.25 | 100 |

| ***Yersinia enterocolitica*** **N= 22** | | | | |
|---|---|---|---|---|
| Gemifloxacin (SB 265805) | ≤0.015-0.12 | 0.03 | 0.03 | 100 |
| Trovafloxacin | ≤0.015-0.25 | 0.06 | 0.06 | 100 |
| Grepafloxacin | ≤0.015-0.25 | 0.03 | 0.03 | 100 |
| Ciprofloxacin | ≤0.015-0.25 | 0.03 | 0.03 | 100 |
| Ofloxacin | 0.03-0.5 | 0.12 | 0.12 | 100 |
| Norfloxacin | 0.06-0.5 | 0.06 | 0.06 | 100 |
| Levofloxacin | 0.03-0.5 | 0.06 | 0.06 | 100 |
| Nalidixic acid | 0.5->128 | 2 | 2 | 95 |
| Ampicillin | 32-64 | 32 | 64 | 0 |
| Amoxicillin | 128 | 128 | 128 | 0 |
| Cefotaxime | 0.06-0.12 | 0.06 | 0.06 | 100 |
| Gentamicin | 0.5-1 | 1 | 1 | 100 |
| Colistin | 0.5-1 | 1 | 1 | 100 |
| Doxycycline | 0.25-8 | 1 | 2 | 95 |
| Co-trimoxazole | 0.06-1 | 0.12 | 1 | 100 |

| ***Shigella*** **spp. N= 21** | | | | |
|---|---|---|---|---|
| Gemifloxacin (SB 265805) | ≤0.015 | ≤0.015 | ≤0.015 | 100 |
| Trovafloxacin | ≤0.015 | ≤0.015 | ≤0.015 | 100 |
| Grepafloxacin | ≤0.015-0.03 | ≤0.015 | 0.03 | 100 |
| Ciprofloxacin | ≤0.015 | ≤0.015 | ≤0.015 | 100 |
| Ofloxacin | ≤0.015-0.06 | 0.03 | 0.06 | 100 |
| Norfloxacin | 0.03-0.06 | 0.06 | 0.06 | 100 |
| Levofloxacin | ≤0.015-0.03 | 0.03 | 0.03 | 100 |
| Nalidixic acid | 1-2 | 1 | 2 | 100 |
| Ampicillin | 2->128 | 64 | >128 | 43 |
| Amoxicillin | 4->128 | 128 | >128 | 43 |
| Cefotaxime | ≤0.015-0.03 | ≤0.015 | 0.03 | 100 |
| Gentamicin | 0.5-1 | 1 | 1 | 100 |
| Colistin | 0.25 | 0.25 | 0.25 | 100 |
| Doxycycline | 0.5-32 | 16 | 32 | 43 |
| Co-trimoxazole | 0.03->128 | 0.06 | >128 | 11 |

| ***Aeromonas*** **spp. N= 16** | | | | |
|---|---|---|---|---|
| Gemifloxacin (SB 265805) | ≤0.015-0.12 | ≤0.015 | 0.03 | 100 |
| Trovafloxacin | ≤0.015-0.25 | ≤0.015 | 0.03 | 100 |
| Grepafloxacin | ≤0.015-0.12 | 0.03 | 0.06 | 100 |
| Ciprofloxacin | ≤0.015-0.06 | ≤0.015 | ≤0.015 | 100 |
| Ofloxacin | ≤0.015-0.12 | ≤0.015 | 0.03 | 100 |
| Norfloxacin | ≤0.015-0.12 | ≤0.015 | 0.03 | 100 |
| Levofloxacin | ≤0.015-0.06 | ≤0.015 | ≤0.015 | 100 |
| Nalidixic acid | 0.06->128 | 0.12 | 0.25 | 94 |
| Ampicillin | 16->128 | >128 | >128 | 0 |
| Amoxicillin | 64->128 | >128 | >128 | 0 |
| Cefotaxime | ≤0.015-0.12 | 0.03 | 0.12 | 100 |
| Gentamicin | 0.25-1 | 0.5 | 1 | 100 |
| Colistin | 0.25-32 | 2 | 4 | 81 |
| Doxycycline | 0.25-4 | 0.5 | 2 | 100 |
| Co-trimoxazole | 0.06-2 | 0.25 | 0.25 | 100 |

| ***Campylobacter jejuni*** **N= 91** | | | | |
|---|---|---|---|---|
| Gemifloxacin (SB 265805) | 0.03-128 | 32 | 128 | 32 |
| Trovafloxacin | ≤0.015-32 | 8 | 8 | 32 |
| Grepafloxacin | 0.03-128 | 32 | 64 | 32 |
| Ciprofloxacin | 0.06-128 | 16 | 64 | 32 |
| Ofloxacin | 0.06->128 | 16 | 32 | 32 |
| Norfloxacin | 0.12->128 | 128 | >128 | 32 |
| Levofloxacin | 0.06-128 | 8 | 32 | 32 |
| Nalidixic acid | 2->128 | >128 | >128 | 31 |
| Ampicillin | 0.25->128 | 8 | 32 | 67 |
| Amoxicillin | 0.12->128 | 4 | 32 | 69 |
| Cefotaxime | 2-32 | 8 | 16 | 57 |
| Gentamicin | 0.12-1 | 0.25 | 0.5 | 100 |
| Colistin | 1-32 | 4 | 8 | 3 |
| Doxycycline | 0.06-64 | 16 | 32 | 40 |
| Co-trimoxazole | 0.25->128 | 1 | 2 | 96 |
| Erythromycin | 0.12-128 | 0.5 | 1 | 77 |

| | | | | |
|---|---|---|---|---|
| *15 antibiotics for *C.jejuni*. | | | | |

**Table 46.**

| **MICs of quinolones tested against 6** ***Salmonella*** **spp in which MICs for ciprofloxacin were ≥0.25 mg/L.** | | | | |
|---|---|---|---|---|
| | MIC (mg/L) | | | |
| | *S. virchow*(2) | *S. hadar*(2) | *S. tshiongwe*(1) | *S. newport*(1) |
| Gemifloxacin | 0.12 | 0.25 | 0.25 | 0.25 |
| Trovafloxacin | 0.25 | 0.5 | 1 | 0.5 |
| Grepafloxacin | 0.25 | 1 | 2 | 1 |
| Ciprofloxacin | 0.25 | 0.5 | 1 | 0.5 |
| Ofloxacin | 0.5 | 2 | 2 | 2 |
| Norfloxacin | 0.5 | 4 | 4 | 2 |
| Levofloxacin | 0.25 | 1 | 1 | 1 |
| Nalidixic acid | >128 | >128 | >128 | >128 |

The invention provides a method for modulating metabolism of enteropathogenic bacteria. Skilled artisans can readily choose enteropathogenic bacteria or patients infected with or suspected to be infected with these organisms to practice the methods of the invention. Alternatively, the bacteria useful in the methods of the invention may be those described herein.

While a preferred object of the invention provides a method wherein said enteropathogenic bacteria is selected from the group consisting of: *Salmonella* spp. (including *S. enteritidis, S. typhimurium, S. virchow, S. tshiongwe, S. newport, S. ohio, S. hadar*, and *S. georgia), Hafnia alvei, Yersinia enterocolitica, Shigella* spp. (including *S. sonnei, S. flexneri,* and *S. boydii*), *Aeromonas* spp. (including *A. hydrophila* and *A. sobria*), and *Campylobacter jejuni*.

The contacting step in any of the methods of the invention may be performed in many ways that will be readily apparent to the skilled artisan. However, it is preferred that the contacting step is a provision of a composition comprising a gemifloxacin compound to a human patient in need of such composition or directly to bacteria in culture medium or buffer.

For example, when contacting a human patient or contacting said bacteria in a human patient or *in vitro,* the compositions comprising a quinolone, particularly a gemifloxacin compound, preferably pharmaceutical compositions may be administered in any effective, convenient manner including, for instance, administration by topical, oral, anal, vaginal, intravenous, intraperitoneal, intramuscular, subcutaneous, intranasal or intradermal routes among others.

It is also preferred that these compositions be employed in combination with a non-sterile or sterile carrier or carriers for use with cells, tissues or organisms, such as a pharmaceutical carrier suitable for administration to a subject. Such compositions comprise, for instance, a media additive or a therapeutically effective amount of a compound of the invention, a quinolone, preferably a gemifloxacin compound, and a pharmaceutically acceptable carrier or excipient. Such carriers may include, but are not limited to, saline, buffered saline, dextrose, water, glycerol, ethanol and combinations thereof. The formulation should suit the mode of administration.

Quinolone compounds, particularly gemifloxacin compounds and compostions of the methods of the invention may be employed alone or in conjunction with other compounds, such as bacterial efflux pump inhibtor compounds or antibiotic compounds, particularly non-quinolone compounds, *e*.*g*., beta-lactam antibiotic compounds.

In therapy or as a prophylactic, the active agent of a method of the invention is preferably administered to an individual as an injectable composition, for example as a sterile aqueous dispersion, preferably an isotonic one.

Alternatively, the gemifloxacin compounds or compositions in the methods of the invention may be formulated for topical application for example in the form of ointments, creams, lotions, eye ointments, eye drops, ear drops, mouthwash, impregnated dressings and sutures and aerosols, and may contain appropriate conventional additives, including, for example, preservatives, solvents to assist drug penetration, and emollients in ointments and creams. Such topical formulations may also contain compatible conventional carriers, for example cream or ointment bases, and ethanol or oleyl alcohol for lotions. Such carriers may constitute from about 1% to about 98% by weight of the formulation; more usually they will constitute up to about 80% by weight of the formulation.

For administration to mammals, and particularly humans, it is expected that the antibacterially effective amount is a daily dosage level of the active agent from 0.001 mg/kg to 10 mg/kg, typically around 0.1 mg/kg to 1 mg/kg, preferably about 1 mg/kg. A physician, in any event, will determine an actual dosage that is most suitable for an individual and will vary with the age, weight and response of the particular individual. The above dosages are exemplary of the average case. There can, of course, be individual instances where higher or lower dosage ranges are merited, and such are within the scope of this invention. It is preferred that the dosage is selected to modulate metabolism of the bacteria in such a way as to inhibit or stop growth of said bacteria or by killing said bacteria. The skilled artisan may identify this amount as provided herein as well as using other methods known in the art, *e.g.* by the application MIC tests.

A further embodiment of the invention provides for the contacting step of the methods to further comprise contacting an in-dwelling device in a patient. In-dwelling devices include, but are not limited to, surgical implants, prosthetic devices and catheters, i.e., devices that are introduced to the body of an individual and remain in position for an extended time. Such devices include, for example, artificial joints, heart valves, pacemakers, vascular grafts, vascular catheters, cerebrospinal fluid shunts, urinary catheters, and continuous ambulatory peritoneal dialysis (CAPD) catheters.

A quinolone, particularly a gemifloxacin compound or composition of the invention may be administered by injection to achieve a systemic effect against bacteria of the invention, shortly before insertion of an in-dwelling device. Treatment may be continued after surgery during the in-body time of the device. In addition, the composition could also be used to broaden perioperative cover for any surgical technique to prevent bacterial wound infections caused by or related to bacteria.

In addition to the therapy described above, a gemifloxacin compound or composition used in the methods of this invention may be used generally as a wound treatment agent to prevent adhesion of bacteria to matrix proteins, particularly enteropathogenic bacteria, exposed in wound tissue and for prophylactic use in dental treatment as an alternative to, or in conjunction with, antibiotic prophylaxis.

Alternatively, a quinolone, particularly a gemifloxacin compound or composition of the invention may be used to bathe an indwelling device immediately before insertion. The active agent will preferably be present at a concentration of 1µg/ml to 10mg/ml for bathing of wounds or indwelling devices.

Other bacteria may also be included in the methods of th invention. The skilled artisan may identify these organisms as provided herein as well as using other methods known in the art, *e.g.* MIC tests.

Also provided by the invention is a method of treating or preventing a bacterial infection by enteropathogenic bacteria comprising the step of administering an antibacterially effective amount of a composition comprising a quinolone, particularly a gemifloxacin compound to a mammal, preferably a human, suspected of having or being at risk of having a bacterial infection.

Preferred embodiments of the invention include, among other things, methods wherein said composition comprises gemifloxacin, or a pharmaceutically acceptable derivative thereof.

All studies provided herein were carried out using standard techniques, which are well known and routine to those of skill in the art, except where otherwise described in detail. All parts or amounts set out in the following examples are by weight, unless otherwise specified.

Each reference cited herein is hereby incorporated by reference in its entirety. Moreover, each patent application to which this application claims priority is hereby incorporated by reference in its entirety.

## Claims

1. A method for modulating metabolism of respiratory tract pathogenic bacteria comprising contacting respiratory tract pathogenic bacteria with an antibacterially effective amount of a composition comprising a gemifloxacin compound, or antibacterially effective derivatives thereof.

2. The method of claim 1 wherein said respiratory tract pathogenic bacteria is selected from the group consisting of:
*Streptococcus pneumoniae, Streptococcus pyogenes, Haemophilus influenzae, Moraxella catarrhalis, Staphylococcus aureus* and *Klebsiella pneumoniae.*

3. A method of treating or preventing a bacterial infection by respiratory tract pathogenic bacteria comprising administering an antibacterially effective amount of a composition comprising a gemifloxacin compound to a mammal suspected of having or being at risk of having an infection with respiratory tract pathogenic bacteria.

4. The method of claim 3 wherein said respiratory tract pathogenic bacteria is selected from the group consisting of:
*Streptococcus pneumoniae, Streptococcus pyogenes, Haemophilus influenzae, Moraxella catarrhalis, Staphylococcus aureus* and *Klebsiella pneumoniae.*

5. The method of claim 3 wherein said mammal is a human.

6. The method of claim 1 wherein said modulating metabolism is inhibiting growth of said bacteria.

7. The method of claim 1 wherein said modulating metabolism is killing said bacteria.

8. The method of claim 1 wherein said contacting said bacteria comprises the further step of introducing said composition into a mammal.

9. The method of claim 8 wherein said mammal is a human.

10. The method of claim 1 wherein said bacteria is selected from the group consisting of: *Haemophilus influenzae, Streptococcus pyogenes, Streptococcus pneumoniae, Staphylococcus aureus,* and *Klebsiella pneumoniae*.

11. The method of claim 1 wherein said bacteria is selected from the group consisting of: *Haemophilus influenzae, Streptococcus pyogenes,* and *Streptococcus pneumoniae.*
